# EUROPEAN PATENT APPLICATION

(11) **EP 3 750 891 A1**
(43) Date of publication of application: **16.12.2020**
(21) Application number: 19752007.5
(22) Date of filing: 01.02.2019
(51) Int. Cl.: C07D 487/04, C07D 471/04, A61K 31/53, A61P 25/00, A61P 35/00, A61P 1/16

(54) **PYRAZOLO[1,5-A][1,3,5]TRIAZINE-2-AMINE DERIVATIVE, PREPARATION METHOD THEREFOR AND MEDICAL USE THEREOF**

(30) Priority: 06.02.2018 CN 201810118455
(71) Applicant: Jiangsu Hengrui Medicine Co., Ltd., Lianyungang, Jiangsu 222047 (CN); Shanghai Hengrui Pharmaceutical Co., Ltd, Shanghai 200245 (CN)
(72) Inventor: LU, Biao, Shanghai 200245 (CN); GUI, Bin, Shanghai 200245 (CN); ZHANG, Junzhen, Shanghai 200245 (CN); HE, Feng, Shanghai 200245 (CN); TAO, Weikang, Shanghai 200245 (CN)
(74) Representative: dompatent von Kreisler Selting Werner - Partnerschaft von Patent- und Rechtsanwälten mbB
(86) International application number: PCT/CN2019/074324
(87) International publication number: WO 2019/154294

(57) **Abstract**

Disclosed are a pyrazolo[1,5-*a*][1,3,5]triazine-2-amine derivative as shown in general formula (I), a preparation method therefor, a pharmaceutical composition containing the derivative and the use of same as a therapeutic agent, in particular the use thereof as an A₂ₐ receptor antagonist and the use thereof in the preparation of drugs for treating conditions or diseases improved by the inhibition of A₂ₐ receptors.

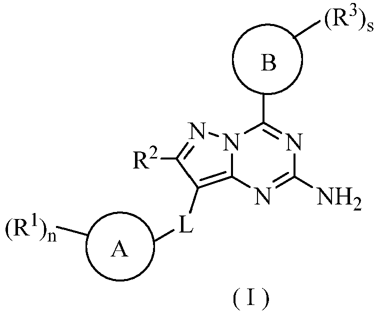

## Description

The present application claims the priority of Chinese Patent Application No. CN201810118455.1, filed on Feb. 6, 2018, the contents of which are incorporated herein by reference in their entireties.

### FIELD OF THE DISCLOSURE

The present disclosure belongs to pharmaceutical field, and relates to a pyrazolo[1,5-*a*][1,3,5]triazine-2-amine derivative of formula (I), a method for preparing the same, a pharmaceutical composition comprising the same, a use thereof as a therapeutic agent, in particular as an A₂ₐ receptor antagonist, and a use thereof in the preparation of a medicament for treating a disease or condition ameliorated by the inhibition of the A₂ₐ receptor.

### BACKGROUND OF THE DISCLOSURE

Adenosine is a naturally occurring purine nucleoside, which is an endogenous regulator of many physiological functions. It plays an important role in the regulation of the cardiovascular system, central nervous system, respiratory system, kidney, fat and platelets.

The action of adenosine is mediated by a family of G-protein coupled receptors. It is known currently that there are at least four subtypes of adenosine receptors, which are classified into A₁, A₂ₐ, A_{2b} and A₃. Among them, the A₁ and A₃ receptors inhibit the activity of the adenylate cyclase, whereas the A₂ₐ and A_{2b} receptors stimulate the activity of the same enzyme, thereby modulating the level of cyclic AMP in cells. Adenosine regulates a wide range of physiological functions through these receptors.

The A₂ₐ receptor (A₂ₐR) is widely distributed in the body. In the central nervous system, it is mainly expressed in the striatum, while it is also expressed in tissues such as the periphery, heart, liver, lung and kidney. Several preclinical studies show that adenosine A₂ₐ receptor antagonists have surprising efficacy in the treatment of neurodegenerative diseases, primarily Parkinson's disease, Huntington's disease or Alzheimer's disease (Trends in Neurosci. 2006, 29(11), 647-654; Expert Opinion on Therapeutic Patents, 2007, 17, 979-991 and the like). Moreover, adenosine A₂ₐ receptor antagonists can also be used to treat other central nervous system (CNS) related diseases such as depression, restless syndrome, sleep disorders and anxiety disorders (Clin. Neuropharmacol. 2010, 33, 55-60; J. Neurosci. 2010, 30 (48), 16284-16292; Parkinsonisn Relat. Disord. 2010, 16 (6), 423-426; and references therein: Mov. Disorders, 2010, 25(2), S305). In addition, adenosine A₂ₐ receptor antagonists also have therapeutic potential as neuroprotective agents (see Jenner P. J Neurol. 2000; 24 7Supp12: 1143-50).

Recent studies indicate that the activation of the adenosine A₂ₐ receptor can exert an important immunomodulatory effect in many pathological processes such as ischemia, hypoxia, inflammation, trauma, transplantation, which may be related to the higher expression level of the A₂ₐ receptor in various immune cells such as T cells, B cells, monocyte macrophages, neutrophils. Moreover, the activation of the A₂ₐ receptor can promote the body to generate immune tolerance, and closely participate in the formation of "immune escape" or "immunosuppression" of tumor cells, thereby creating a favorable condition for the occurrence and development of tumors. Lokshin and his colleagues (Cancer Res. 2006, Aug 1; 66 (15):7758-65) demonstrate that the activation of A₂ₐR in natural killer cells can inhibit the killing of tumor cells by natural killer cells through increasing cAMP and activating PKA. Studies also show that the activation of A₂ₐ receptor can promote the proliferation of tumor cells such as melanoma A375 cells, fibroblast NIH3T3 cells, pheochromocytoma PC12 cells, which may be related to the fact that the activation of the A₂ₐ receptor in T cells can inhibit T cell activation, proliferation, adhesion to tumor cells, and produce cytotoxic effect on tumor cells. However, in the A₂ₐ receptor knockout mice, the anti-tumor immunity of CD8⁺T cells is enhanced, and the tumor proliferation is significantly inhibited. Therefore, A₂ₐ receptor antagonists can be used in the treatment of tumors.

Although compounds having significant biological activity on a variety of subtypes of adenosine receptors can have therapeutic efficacy, they can cause undesired side effects. For example, during tissue ischemia/hypoxia, when cells of central system, circulatory system, digestive system, and skeletal muscle are in an anoxic and hypoxic stress environment, extracellular aggregated adenosine initiates a corresponding protective mechanism by activating the adenosine A₁ receptor on the cell membrane, thereby increasing the tolerance of the cells to anoxia and hypoxia. The A₁ receptor located on immune cells can promote cellular immune responses in a hypoxic environment. Moreover, the A₁ receptor can also reduce free fatty acids and triglycerides, and it is involved in regulating blood glucose. Therefore, the continued blocking of the A₁ receptor can cause various adverse effects in the body tissues (Chinese Pharmacological Bulletin, 2008, 24(5), 573-576). For example, it is reported that the blocking of the A₁ receptor will cause adverse effects such as anxiety, awakening in animal models (Basic & Clinical Pharmacology & Toxicology, 2011, 109 (3), 203-7). The adenosine released by the adenosine A₃ receptor during myocardial ischemia exerts a strong protective effect in heart (as described in Gessi S et al, Pharmacol. Ther. 117 (1), 2008, 123-140). The continued blocking of the A₃ receptor can increase the likelihood of complications caused by any pre-existing or developing ischemic heart disease such as angina or heart failure.

Currently, many compounds have been developed as A₂ₐ receptor antagonists for the treatment of various diseases, as described in WO2007116106, WO2009080197, WO2009156737, WO2011159302, WO2011095625, WO2014101373 and WO2015031221.

### SUMMARY OF THE DISCLOSURE

The object of the present disclosure is to provide a compound of formula (I) or a tautomer, mesomer, racemate, enantiomer or diastereomer thereof, or a mixture thereof, or a pharmaceutically acceptable salt thereof: 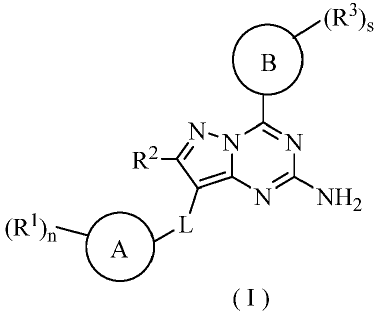 wherein:
L is selected from the group consisting of CR⁴R⁵, O, NH and S;
ring A and ring B are identical or different and are each independently selected from the group consisting of cycloalkyl, heterocyclyl, aryl and heteroaryl;
R¹ is identical or different and is each independently selected from the group consisting of hydrogen, halogen, alkyl, alkoxy, haloalkyl, haloalkoxy, hydroxyl, hydroxyalkyl, cyano, amino, nitro, cycloalkyl, heterocyclyl, heterocyclylalkyl, heterocyclyloxy, aryl, heteroaryl and -Y-R^{a};
Y is a covalent bond or alkylene;
R^{a} is selected from the group consisting of hydrogen, halogen, alkyl, alkoxy, haloalkyl, haloalkoxy, hydroxyl, hydroxyalkyl, cyano, amino, nitro, cycloalkyl, heterocyclyl, heterocyclylalkyl, heterocyclyloxy, -OR^{c}, -C(O)R⁹, -C(O)OR⁹, -OS(O)ₘR⁶, aryl and heteroaryl, wherein the alkyl, alkoxy, cycloalkyl, heterocyclyl, heterocyclylalkyl, heterocyclyloxy, aryl and heteroaryl are each optionally substituted by one or more substituents independently selected from the group consisting of halogen, alkyl, alkoxy, haloalkyl, hydroxyl, hydroxyalkyl, cyano, amino, nitro, cycloalkyl, heterocyclyl, heterocyclyloxy, aryl, heteroaryl and -OS(O)ₘR⁶;
R^{c} is selected from the group consisting of hydrogen, alkyl, haloalkyl, hydroxyalkyl, cycloalkyl and heterocyclyl, wherein the alkyl, cycloalkyl and heterocyclyl are each optionally substituted by one or more substituents independently selected from the group consisting of halogen, alkyl, alkoxy, haloalkyl, hydroxyl, hydroxyalkyl, cyano, amino, nitro, cycloalkyl and heterocyclyl;
R² is selected from the group consisting of hydrogen, halogen, alkyl, alkoxy, haloalkyl, hydroxyl, hydroxyalkyl, cyano, amino, nitro, cycloalkyl, heterocyclyl, aryl and heteroaryl;
R³ is selected from the group consisting of hydrogen, halogen, alkyl, alkoxy, haloalkyl, hydroxyl, hydroxyalkyl, cyano, amino, nitro, cycloalkyl, heterocyclyl, aryl and heteroaryl;
R⁴ and R⁵ are identical or different and are each independently selected from the group consisting of hydrogen, halogen, alkyl, alkoxy, haloalkyl, hydroxyl and hydroxyalkyl;
or, R⁴ and R⁵ together with each other form =NH or =O;
R⁶ is selected from the group consisting of hydrogen, halogen, alkoxy, haloalkoxy, amino, cycloalkyl, heterocyclyl, aryl, heteroaryl and -NR⁷R⁸;
R⁷ and R⁸ are identical or different and are each independently selected from the group consisting of hydrogen, alkyl, alkoxy, haloalkyl, haloalkoxy, hydroxyl, hydroxyalkyl, cyano, amino, nitro, cycloalkyl, heterocyclyl, aryl and heteroaryl;
or, R⁷and R⁸, together with the nitrogen atom to which they are attached, form a heterocyclyl, wherein the heterocyclyl optionally contains one to two identical or different heteroatoms selected from the group consisting of N, O and S besides the nitrogen atom to which R⁷ and R⁸ are attached, and the heterocyclyl is optionally substituted by one or more substituents selected from the group consisting of alkyl, alkoxy, oxo, halogen, amino, cyano, nitro, hydroxyl, hydroxyalkyl, cycloalkyl, heterocyclyl, aryl and heteroaryl;
R⁹ is selected from the group consisting of hydrogen, alkyl, haloalkyl, alkoxy, haloalkoxy, amino, cycloalkyl, heterocyclyl, aryl and heteroaryl;
n is 0, 1, 2, 3 or 4;
s is 0, 1, 2 or 3; and
m is 1 or 2.

In some embodiments of the present disclosure, the compound of formula (I) or a tautomer, mesomer, racemate, enantiomer or diastereomer thereof, or a mixture thereof, or a pharmaceutically acceptable salt thereof, wherein R^{a} is selected from the group consisting of hydrogen, halogen, alkyl, alkoxy, haloalkyl, haloalkoxy, hydroxyl, hydroxyalkyl, cyano, amino, nitro, cycloalkyl, heterocyclyl, heterocyclylalkyl, heterocyclyloxy, -OS(O)ₘR⁶, aryl and heteroaryl, wherein the alkyl, alkoxy, cycloalkyl, heterocyclyl, heterocyclylalkyl, heterocyclyloxy, aryl and heteroaryl are each optionally substituted by one or more substituents independently selected from the group consisting of halogen, alkyl, alkoxy, haloalkyl, hydroxyl, hydroxyalkyl, cyano, amino, nitro, cycloalkyl, heterocyclyl, heterocyclyloxy, aryl, heteroaryl and -OS(O)ₘR⁶; the definitions of other groups are as described in the present disclosure.

In some embodiments of the present disclosure, the compound of formula (I) or a tautomer, mesomer, racemate, enantiomer or diastereomer thereof, or a mixture thereof, or a pharmaceutically acceptable salt thereof, wherein R¹ is identical or different and is each independently selected from -Y-R^{a};
Y is a covalent bond or alkylene;
when Y is a covalent bond, R^{a} is selected from the group consisting of hydrogen, halogen, alkoxy, haloalkoxy, hydroxyl, cyano, amino, nitro, cycloalkyl, heterocyclyl, heterocyclyloxy, -OR^{c} , -COR⁹, -COOR⁹, -OS(O)ₘR⁶, aryl and heteroaryl; wherein the alkoxy, cycloalkyl, heterocyclyl, heterocyclyloxy, aryl and heteroaryl are each optionally substituted by one or more substituents independently selected from the group consisting of halogen, alkyl, alkoxy, haloalkyl, hydroxyl, hydroxyalkyl, cyano, amino, nitro, cycloalkyl, heterocyclyl, heterocyclyloxy, aryl, heteroaryl and -OS(O)ₘR⁶;
when Y is alkylene, R^{a} is selected from the group consisting of hydrogen, halogen, alkyl, alkoxy, haloalkyl, haloalkoxy, hydroxyl, hydroxyalkyl, cyano, amino, nitro, cycloalkyl, heterocyclyl, heterocyclylalkyl, heterocyclyloxy, -OR^{c}, -COR⁹, -COOR⁹, -OS(O)ₘR⁶, aryl and heteroaryl; wherein the alkyl, alkoxy, cycloalkyl, heterocyclyl, heterocyclylalkyl, heterocyclyloxy, aryl and heteroaryl are each optionally substituted by one or more substituents independently selected from the group consisting of halogen, alkyl, alkoxy, haloalkyl, hydroxyl, hydroxyalkyl, cyano, amino, nitro, cycloalkyl, heterocyclyl, heterocyclyloxy, aryl, heteroaryl and -OS(O)ₘR⁶;
other groups are as defined in the present disclosure.

In some embodiments of the present disclosure, the compound of formula (I) or a tautomer, mesomer, racemate, enantiomer or diastereomer thereof, or a mixture thereof, or a pharmaceutically acceptable salt thereof, wherein R¹ is identical or different and is each independently selected from -Y-R^{a};
Y is a covalent bond or alkylene;
when Y is a covalent bond, R^{a} is selected from the group consisting of hydrogen, halogen, -COR⁹ and -COOR⁹;
when Y is alkylene, R^{a} is selected from the group consisting of hydrogen, halogen, alkyl, alkoxy, haloalkyl, haloalkoxy, hydroxyl, hydroxyalkyl, cyano, amino, nitro, cycloalkyl, heterocyclyl, heterocyclylalkyl, heterocyclyloxy, -OR^{c}, -COR⁹, -COOR⁹, -OS(O)ₘR⁶, aryl and heteroaryl; wherein the alkyl, alkoxy, cycloalkyl, heterocyclyl, heterocyclylalkyl, heterocyclyloxy, aryl and heteroaryl are each optionally substituted by one or more substituents independently selected from the group consisting of halogen, alkyl, alkoxy, haloalkyl, hydroxyl, hydroxyalkyl, cyano, amino, nitro, cycloalkyl, heterocyclyl, heterocyclyloxy, aryl, heteroaryl and -OS(O)ₘR⁶;
other groups are as defined in the present disclosure.

In some embodiments of the present disclosure, the compound of formula (I) or a tautomer, mesomer, racemate, enantiomer or diastereomer thereof, or a mixture thereof, or a pharmaceutically acceptable salt thereof, wherein R¹ is identical or different and is each independently selected from -Y-R^{a};
Y is a covalent bond or alkylene, wherein the alkylene is 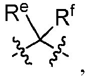 R^{e} and R^{f} are each independently hydrogen or alkyl;
when Y is a covalent bond, R^{a} is selected from the group consisting of hydrogen, halogen, -COR⁹ and -COOR⁹;
when Y is alkylene, R^{a} is selected from the group consisting of hydrogen, halogen, alkyl, alkoxy, haloalkyl, haloalkoxy, hydroxyl, hydroxyalkyl, cyano, amino, nitro, cycloalkyl, heterocyclyl, heterocyclylalkyl, heterocyclyloxy, -OR^{c}, -COR⁹, -COOR⁹, -OS(O)ₘR⁶, aryl and heteroaryl; wherein the alkyl, alkoxy, cycloalkyl, heterocyclyl, heterocyclylalkyl, heterocyclyloxy, aryl and heteroaryl are each optionally substituted by one or more substituents independently selected from the group consisting of halogen, alkyl, alkoxy, haloalkyl, hydroxyl, hydroxyalkyl, cyano, amino, nitro, cycloalkyl, heterocyclyl, heterocyclyloxy, aryl, heteroaryl and -OS(O)ₘR⁶;
other groups are as defined in the present disclosure.

In some embodiments of the present disclosure, the compound of formula (I) or a tautomer, mesomer, racemate, enantiomer or diastereomer thereof, or a mixture thereof, or a pharmaceutically acceptable salt thereof, wherein R¹ is identical or different and is each independently selected from -Y-R^{a};
Y is a covalent bond or alkylene, wherein the alkylene is 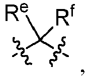 R^{e} and R^{f} are each independently hydrogen or alkyl;
when Y is a covalent bond, R^{a} is selected from the group consisting of hydrogen, -COR⁹ and -COOR⁹;
when Y is alkylene, R^{a} is selected from the group consisting of hydrogen, halogen, alkyl, alkoxy, haloalkyl, haloalkoxy, hydroxyl, hydroxyalkyl, cyano, amino, nitro, cycloalkyl, heterocyclyl, heterocyclylalkyl, heterocyclyloxy, -OR^{c}, -COR⁹, -COOR⁹, -OS(O)ₘR⁶, aryl and heteroaryl; wherein the alkyl, alkoxy, cycloalkyl, heterocyclyl, heterocyclylalkyl, heterocyclyloxy, aryl and heteroaryl are each optionally substituted by one or more substituents independently selected from the group consisting of halogen, alkyl, alkoxy, haloalkyl, hydroxyl, hydroxyalkyl, cyano, amino, nitro, cycloalkyl, heterocyclyl, heterocyclyloxy, aryl, heteroaryl and -OS(O)ₘR⁶;
other groups are as defined in the present disclosure.

In some embodiments of the present disclosure, the compound of formula (I) or a tautomer, mesomer, racemate, enantiomer or diastereomer thereof, or a mixture thereof, or a pharmaceutically acceptable salt thereof, wherein R¹ is identical or different and is each independently selected from -Y-R^{a};
Y is a covalent bond or alkylene, wherein the alkylene is 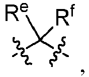 R^{e} and R^{f} are each independently hydrogen or alkyl;
when Y is a covalent bond, R^{a} is selected from the group consisting of hydrogen, -COR⁹ and -COOR⁹;
when Y is alkylene, R^{a} is selected from the group consisting of hydrogen, alkyl, alkoxy, hydroxyl, hydroxyalkyl, cycloalkyl, heterocyclyl, heterocyclylalkyl, heterocyclyloxy, -OR^{c}, -COR⁹, -COOR⁹ and -OS(O)ₘR⁶; wherein the alkyl, alkoxy, cycloalkyl, heterocyclyl, heterocyclylalkyl and heterocyclyloxy are each optionally substituted by one or more substituents independently selected from the group consisting of alkyl, alkoxy, hydroxyl, hydroxyalkyl and -OS(O)mR⁶;
other groups are as defined in the present disclosure.

In some embodiments of the present disclosure, the compound of formula (I) or a tautomer, mesomer, racemate, enantiomer, or diastereomer thereof, or a mixture thereof, or a pharmaceutically acceptable salt thereof, wherein Y is a covalent bond or alkylene, wherein the alkylene is 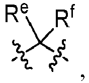 R^{e} and R^{f} are each independently hydrogen or alkyl; other groups are as defined in the present disclosure.

In some embodiments of the present disclosure, the compound of formula (I) or a tautomer, mesomer, racemate, enantiomer or diastereomer thereof, or a mixture thereof, or a pharmaceutically acceptable salt thereof, wherein the moiety 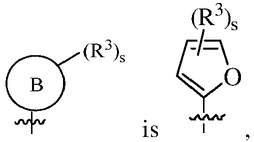 other groups are as defined in the present disclosure.

In some embodiments of the present disclosure, the compound of formula (IV) or a tautomer, mesomer, racemate, enantiomer or diastereomer thereof, or a mixture thereof, or a pharmaceutically acceptable salt thereof, wherein the moiety 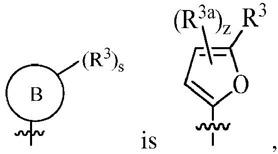 R^{3a} is defined the same as R³, z is 0, 1 or 2; other groups are as defined in the present disclosure.

In some embodiments of the present disclosure, the compound of formula (IV) or a tautomer, mesomer, racemate, enantiomer or diastereomer thereof, or a mixture thereof, or a pharmaceutically acceptable salt thereof, wherein the moiety 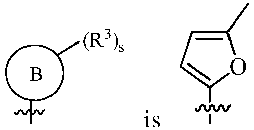 or 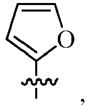 other groups are as defined in the present disclosure.

In some embodiments of the present disclosure, the compound of formula (I) or a tautomer, mesomer, racemate, enantiomer, or diastereomer thereof, or a mixture thereof, or a pharmaceutically acceptable salt thereof, wherein -Y-R^{a} is -F,

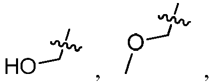

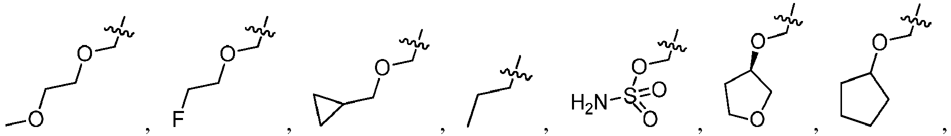

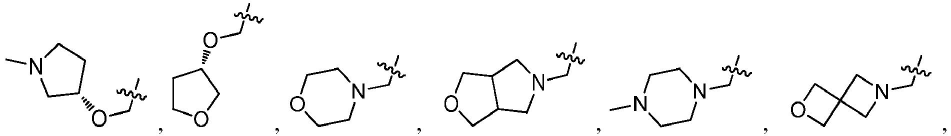

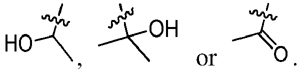

In some preferred embodiments of the present disclosure, the compound of formula (I) or a tautomer, mesomer, racemate, enantiomer or diastereomer thereof, or a mixture thereof, or a pharmaceutically acceptable salt thereof, wherein the compound of formula (I) is a compound of formula (II): 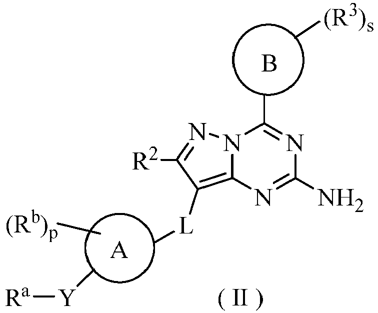 wherein:
R^{b} is identical or different and is each independently selected from the group consisting of hydrogen, halogen, alkyl, alkoxy, haloalkyl, haloalkoxy, hydroxyl, hydroxyalkyl, cyano, amino, nitro, cycloalkyl, heterocyclyl, heterocyclylalkyl, heterocyclyloxy, aryl and heteroaryl;
p is 0, 1, 2 or 3;
ring A, ring B, L, Y, R^{a}, R², R³ and s are as defined in formula (I).

In some preferred embodiments of the present disclosure, the compound of formula (I) or a tautomer, mesomer, racemate, enantiomer or diastereomer thereof, or a mixture thereof, or a pharmaceutically acceptable salt thereof, wherein ring A and ring B are identical or different and are each independently selected from the group consisting of aryl and heteroaryl, preferably selected from the group consisting of phenyl, pyridyl, furyl and thienyl.

In some embodiments of the present disclosure, the compound of formula (I) or a tautomer, mesomer, racemate, enantiomer or diastereomer thereof, or a mixture thereof, or a pharmaceutically acceptable salt thereof, wherein ring A is phenyl or pyridyl, and/or, ring B is furyl.

In some preferred embodiments of the present disclosure, the compound of formula (I) or a tautomer, mesomer, racemate, enantiomer or diastereomer thereof, or a mixture thereof, or a pharmaceutically acceptable salt thereof, is a compound of formula (III): 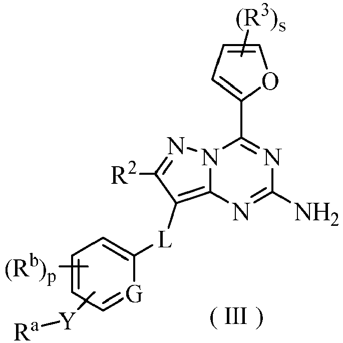 wherein:
G is selected from the group consisting of C, CH and N;
L, Y, R^{a}, R^{b}, R², R³, p and s are as defined in formula (II).

In some preferred embodiments of the present disclosure, the compound of formula (I) or a tautomer, mesomer, racemate, enantiomer or diastereomer thereof, or a mixture thereof, or a pharmaceutically acceptable salt thereof, is a compound of formula (III'): 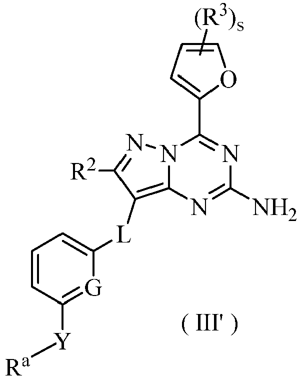 wherein:
G is selected from the group consisting of C, CH and N;
L, Y, R^{a}, R², R³ and s are as defined in formula (I).

In some preferred embodiments of the present disclosure, the compound of formula (I) or a tautomer, mesomer, racemate, enantiomer or diastereomer thereof, or a mixture thereof, or a pharmaceutically acceptable salt thereof, wherein -Y- is a covalent bond or -CH₂-.

In some preferred embodiments of the present disclosure, the compound of formula (I) or a tautomer, mesomer, racemate, enantiomer or diastereomer thereof, or a mixture thereof, or a pharmaceutically acceptable salt thereof, is a compound of formula (IV): 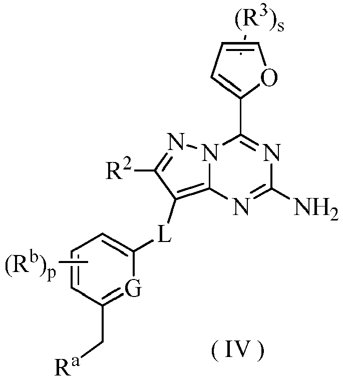 wherein:
G is selected from the group consisting of C, CH and N;
L, R^{a}, R^{b}, R², R³, p and s are as defined in formula (I).

In some preferred embodiments of the present disclosure, the compound of formula (I) or a tautomer, mesomer, racemate, enantiomer or diastereomer thereof, or a mixture thereof, or a pharmaceutically acceptable salt thereof, wherein L is selected from the group consisting of CR⁴R⁵, O, NH and S; R⁴ and R⁵ are hydrogen; or R⁴ and R⁵ together with each other form =NH.

In some preferred embodiments of the present disclosure, the compound of formula (I) or a tautomer, mesomer, racemate, enantiomer or diastereomer thereof, or a mixture thereof, or a pharmaceutically acceptable salt thereof, wherein R² is selected from the group consisting of hydrogen, halogen and alkyl, preferably hydrogen.

In some preferred embodiments of the present disclosure, the compound of formula (I) or a tautomer, mesomer, racemate, enantiomer or diastereomer thereof, or a mixture thereof, or a pharmaceutically acceptable salt thereof, wherein R³ is selected from the group consisting of hydrogen, halogen and alkyl, preferably hydrogen or C₁₋₆ alkyl.

In some embodiments of the present disclosure, the compound of formula (I) or a tautomer, mesomer, racemate, enantiomer or diastereomer thereof, or a mixture thereof, or a pharmaceutically acceptable salt thereof, wherein R^{a} is selected from the group consisting of hydrogen, halogen, alkyl, haloalkyl, alkoxy, haloalkoxy, hydroxyl, hydroxyalkyl, heterocyclyl, heterocyclylalkyl, heterocyclyloxy and -OS(O)ₘR⁶, wherein the alkyl, alkoxy, heterocyclyl, heterocyclylalkyl and heterocyclyloxy are each optionally substituted by one or more substituents independently selected from the group consisting of halogen, alkyl, alkoxy and cycloalkyl; R⁶ is alkyl or amino.

In some embodiments of the present disclosure, the compound of formula (I) or a tautomer, mesomer, racemate, enantiomer or diastereomer thereof, or a mixture thereof, or a pharmaceutically acceptable salt thereof, wherein R^{a} is selected from the group consisting of hydrogen, halogen, alkyl, haloalkyl, alkoxy, haloalkoxy, hydroxyl, hydroxyalkyl, heterocyclyl, heterocyclylalkyl, heterocyclyloxy, -OR^{c}, -COR⁹, -COOR⁹, and -OS(O)ₘR⁶, wherein the alkyl, alkoxy, heterocyclyl, heterocyclylalkyl and heterocyclyloxy are each optionally substituted by one or more substituents independently selected from the group consisting of halogen, alkyl, alkoxy and cycloalkyl; R⁶ is alkyl or amino; R^{c} is selected from the group consisting of hydrogen, alkyl, haloalkyl, hydroxyalkyl, cycloalkyl and heterocyclyl, wherein the alkyl, cycloalkyl and heterocyclyl are each optionally substituted by one or more substituents independently selected from the group consisting of alkoxy, hydroxyalkyl, cycloalkyl and heterocyclyl; R⁹ is alkyl.

In some preferred embodiments of the present disclosure, the compound of formula (I) or a tautomer, mesomer, racemate, enantiomer or diastereomer thereof, or a mixture thereof, or a pharmaceutically acceptable salt thereof, wherein R^{a} is selected from the group consisting of hydrogen, halogen, alkyl, hydroxyalkyl, heterocyclyl, -OR^{c}, -COR⁹, -COOR⁹ and -OS(O)ₘR⁶, wherein the alkyl and heterocyclyl are each optionally substituted by one or more substituents independently selected from the group consisting of alkyl, hydroxyl and oxo; R^{c} is selected from the group consisting of hydrogen, alkyl, haloalkyl, hydroxyalkyl, cycloalkyl and heterocyclyl, wherein the alkyl, cycloalkyl and heterocyclyl are each optionally substituted by one or more substituents independently selected from the group consisting of alkyl, alkoxy, hydroxyl and cycloalkyl; R⁶ is alkyl or amino; R⁹ is alkyl.

In some preferred embodiments of the present disclosure, the compound of formula (I) or a tautomer, mesomer, racemate, enantiomer or diastereomer thereof, or a mixture thereof, or a pharmaceutically acceptable salt thereof, wherein R^{b} is selected from the group consisting of hydrogen, halogen and alkyl; p is 0, 1 or 2.

Typical compounds of the present disclosure include, but are not limited to:

| Example No. | Structure and name of the compounds |
|---|---|
| 1 | 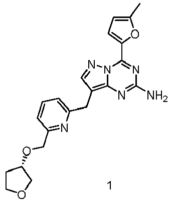 |
| | (*S*)-4-(5-methylfuran-2-yl)-8-((6-(((tetrahydrofuran-3-yl)oxy)methyl)pyridi n-2-yl)methyl)pyrazolo[1,5-*a*][1,3,5]triazin-2-amine **1** |
| 2 | 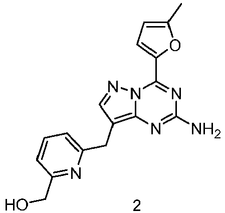 |
| | (6-((2-amino-4-(5-methylfuran-2-yl)pyrazolo[1,5-*a*][1,3,5]triazin-8-yl)met hyl)pyridin-2-yl)methanol **2** |
| 3 | 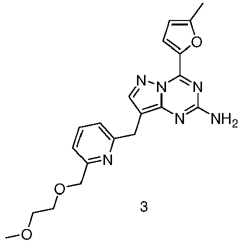 |
| | 8-((6-((2-methoxyethoxy)methyl)pyridin-2-yl)methyl)-4-(5-methylfuran-2-yl)pyrazolo[1,5-*a*][1,3,5]triazin-2-amine **3** |
| 3a | 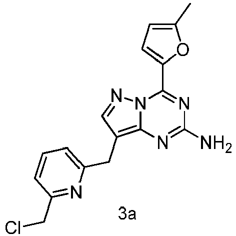 |
| | 8-((6-(chloromethyl)pyridin-2-yl)methyl)-4-(5-methylfuran-2-yl)pyrazolo[ 1,5-*a*][1,3,5]triazin-2-amine **3a** |
| 4 | 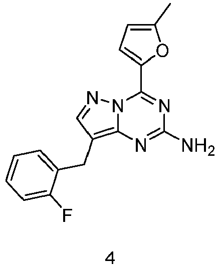 |
| | 8-(2-fluorobenzyl)-4-(5-methylfuran-2-yl)pyrazolo[1,5-*a*][1,3,5]triazin-2-a mine **4** |
| 5 | 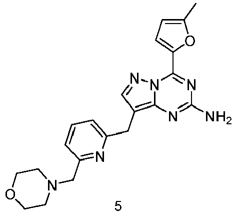 |
| | 4-(5-methylfuran-2-yl)-8-((6-(morpholinomethyl)pyridin-2-yl)methyl)pyra zolo[1,5-*a*][1,3,5]triazin-2-amine **5** |
| 6 | 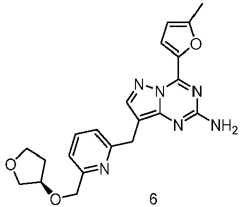 |
| | (*R*)-4-(5-methylfuran-2-yl)-8-((6-(((tetrahydrofuran-3-yl)oxy)methyl)pyridi n-2-yl)methyl)pyrazolo[1,5-*a*][1,3,5]triazin-2-amine **6** |
| 7 | 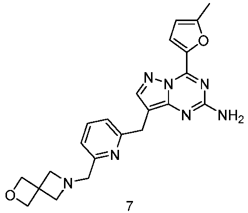 |
| | 8-((6-((2-oxa-6-azaspiro[3.3]heptan-6-yl)methyl)pyridin-2-yl)methyl)-4-(5-methylfuran-2-yl)pyrazolo[1,5-*a*] [1,3,5]triazin-2-amine **7** |
| 8 | 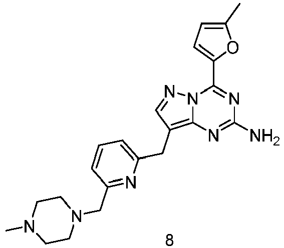 |
| | 4-(5-methylfuran-2-yl)-8-((6-((4-methylpiperazin-1-yl)methyl)pyridin-2-yl )methyl)pyrazolo[1,5-*a*] [1,3,5]triazin-2-amine **8** |
| 9 | 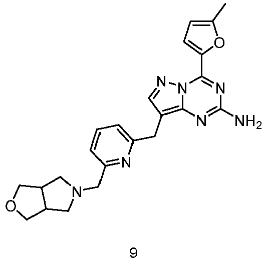 |
| | 4-(5-methylfuran-2-yl)-8-((6-((tetrahydro-1*H*-furo[3,4-*c*]pyrrol-5(3*H*)-yl)m ethyl)pyridin-2-yl)methyl)pyrazolo[1,5-*a*][1,3,5]triazin-2-amine **9** |
| 10 | 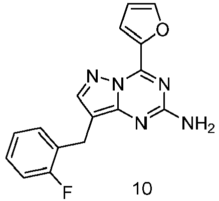 |
| | 8-(2-fluorobenzyl)-4-(furan-2-yl)pyrazolo[1,5-*a*][1,3,5]triazin-2-amine **10** |
| 11 | 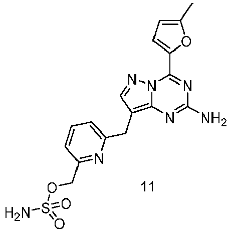 |
| | (6-((2-amino-4-(5-methylfuran-2-yl)pyrazolo[1,5-*a*][1,3,5]triazin-8-yl)met hyl)pyridin-2-yl)methyl sulfamate **11** |
| 12 | 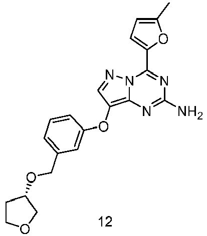 |
| | (*S*)-4-(5-methylfuran-2-yl)-8-(3-(((tetrahydrofuran-3-yl)oxy)methyl)pheno xy)pyrazolo[1,5-*a*] [1,3,5]triazin-2-amine **12** |
| 13 | 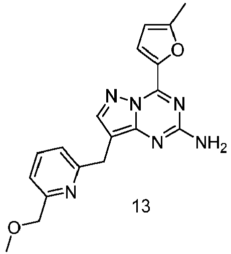 |
| | 8-((6-(methoxymethyl)pyridin-2-yl)methyl)-4-(5-methylfuran-2-yl)pyrazol o[1,5-*a*][1,3,5]triazin-2-amine **13** |
| 14 | 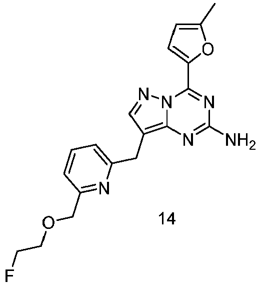 |
| | 8-((6-((2-fluoroethoxy)methyl)pyridin-2-yl)methyl)-4-(5-methylfuran-2-yl) pyrazolo[1,5-*a*][1,3,5]triazin-2-amine **14** |
| 15 | 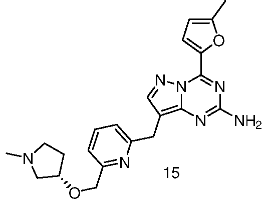 |
| | (*S*)-4-(5-methylfuran-2-yl)-8-((6-(((1-methylpyrrolidin-3-yl)oxy)methyl)py ridin-2-yl)methyl)pyrazolo[1,5-*a*][1,3,5]triazin-2-amine **15** |
| 16 | 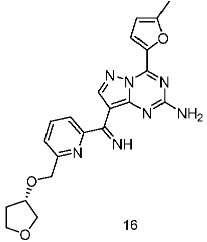 |
| | (*S*)-8-(imino(6-(((tetrahydrofuran-3-yl)oxy)methyl)pyridin-2-yl)methyl)-4-(5-methylfuran-2-yl)pyrazolo[1,5-*a*][1,3,5]triazin-2-amine **16** |
| 17 | 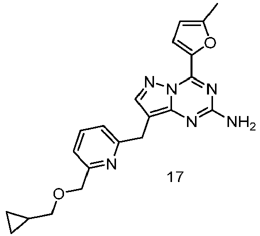 |
| | 8-((6-((cyclopropylmethoxy)methyl)pyridin-2-yl)methyl)-4-(5-methylfuran -2-yl)pyrazolo[1,5-*a*][1,3,5]triazin-2-amine **17** |
| 18 | 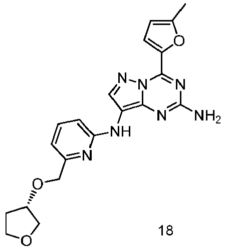 |
| | (*S*)-4-(5-methylfuran-2-yl)-*N*⁸-(6-(((tetrahydrofuran-3-yl)oxy)methyl)pyridi n-2-yl)pyrazolo[1,5-*a*][1,3,5]triazine-2,8-diamine **18** |
| 19 | 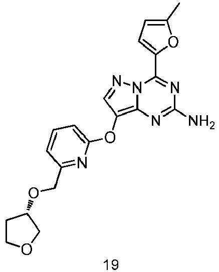 |
| | (*S*)-4-(5-methylfuran-2-yl)-8-((6-(((tetrahydrofuran-3-yl)oxy)methyl)pyridi n-2-yl)oxy)pyrazolo[1,5-*a*][1,3,5]triazin-2-amine **19** |
| 20 | 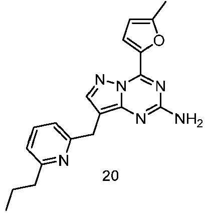 |
| | 4-(5-methylfuran-2-yl)-8-((6-propylpyridin-2-yl)methyl)pyrazolo[1,5-*a*][1, 3,5]triazin-2-amine **20** |
| 21 | 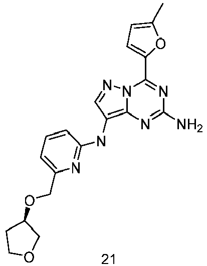 |
| | (*R*)-4-(5-methylfuran-2-yl)-*N*⁸-(6-(((tetrahydrofuran-3-yl)oxy)methyl)pyrid in-2-yl)pyrazolo[1,5-*a*][1,3,5]triazine-2,8-diamine **21** |
| 22 | 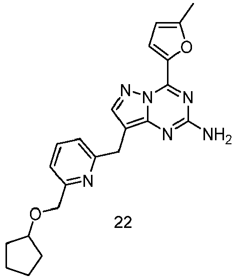 |
| | 8-((6-((cyclopentyloxy)methyl)pyridin-2-yl)methyl)-4-(5-methylfuran-2-yl )pyrazolo[1,5-*a*][1,3,5]triazin-2-amine **22** |
| 23 | 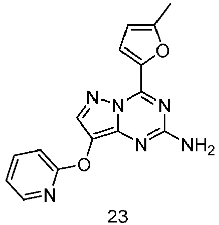 |
| | 4-(5-methylfuran-2-yl)-8-(pyridin-2-yloxy)pyrazolo[1,5-*a*][1,3,5]triazin-2-amine **23** |
| 24 | 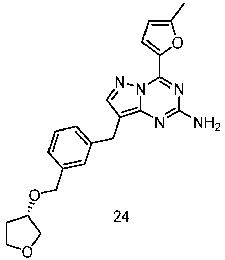 |
| | (*S*)-4-(5-methylfuran-2-yl)-8-(3-(((tetrahydrofuran-3-yl)oxy)methyl)benzyl )pyrazolo[1,5-*a*][1,3,5]triazin-2-amine **24** |
| 25 | 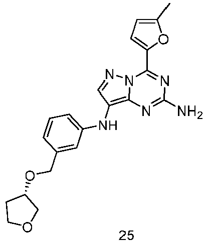 |
| | (*S*)-4-(5-methylfuran-2-yl)-*N*⁸-(3-(((tetrahydrofuran-3-yl)oxy)methyl)phen yl)pyrazolo[1,5-*a*][1,3,5]triazine-2,8-diamine **25** |
| 26 | 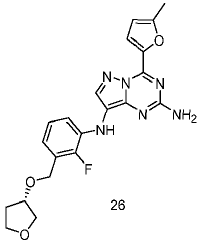 |
| | (*S*)-*N*⁸-(2-fluoro-3-(((tetrahydrofuran-3-yl)oxy)methyl)phenyl)-4-(5-methyl furan-2-yl)pyrazolo[1,5-*a*][1,3,5]triazine-2,8-diamine **26** |
| 27 | 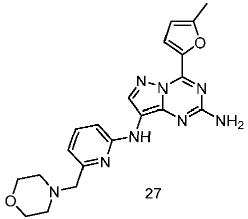 |
| | 4-(5-methylfuran-2-yl)-*N*⁸-(6-(morpholinomethyl)pyridin-2-yl)pyrazolo[1,5 -*a*][1,3,5]triazine-2,8-diamine **27** |
| 27c | 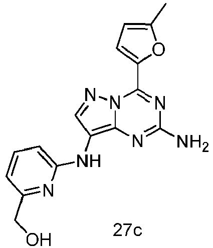 |
| | (6-((2-amino-4-(5-methylfuran-2-yl)pyrazolo[1,5-*a*][1,3,5]triazin-8-yl)ami no)pyridin-2-yl)methanol **27c** |
| 27d | 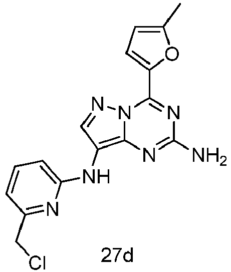 |
| | *N*⁸-(6-(chloromethyl)pyridin-2-yl)-4-(5-methylfuran-2-yl)pyrazolo[1,5-*a*][1 ,3,5]triazine-2,8-diamine **27d** |
| 28 | 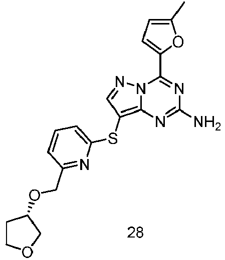 |
| | (*S*)-4-(5-methylfuran-2-yl)-8-((6-(((tetrahydrofuran-3-yl)oxy)methyl)pyridi n-2-yl)thio)pyrazolo[1,5-*a*] [1,3,5]triazin-2-amine **28** |
| 29 | 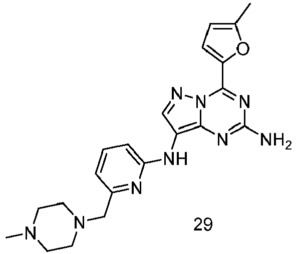 |
| | 4-(5-methylfuran-2-yl)-*N*⁸-(6-((4-methylpiperazin-1-yl)methyl)pyridin-2-yl )pyrazolo[1,5-*a*][1,3,5]triazine-2,8-diamine **29** |
| 30 | 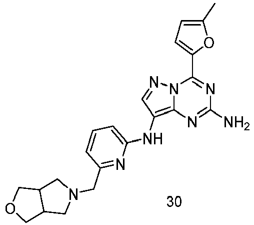 |
| | 4-(5-methylfuran-2-yl)-*N*⁸-(6-((tetrahydro-1*H*-furo[3,4-c]pyrrol-5(3*H*)-yl) methyl)pyridin-2-yl)pyrazolo[1,5-*a*][1,3,5]triazine-2,8-diamine **30** |
| 31 | 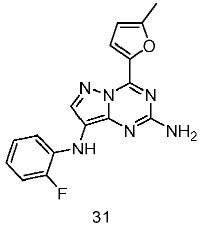 |
| | *N*⁸-(2-fluorophenyl)-4-(5-methylfuran-2-yl)pyrazolo[1,5-*a*][1,3,5]triazine-2 ,8-diamine **31** |
| 32 | 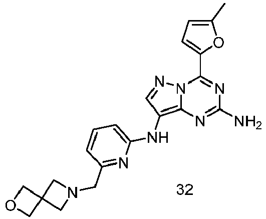 |
| | *N*⁸-(6-((2-oxa-6-azaspiro[3.3]heptan-6-yl)methyl)pyridin-2-yl)-4-(5-methyl furan-2-yl)pyrazolo[1,5-*a*][1,3,5]triazine-2,8-diamine **32** |
| 33 | 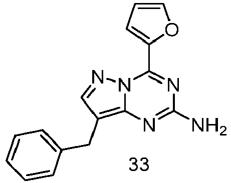 |
| | 8-benzyl-4-(furan-2-yl)pyrazolo[1,5-*a*][1,3,5]triazin-2-amine **33** |
| 34 | 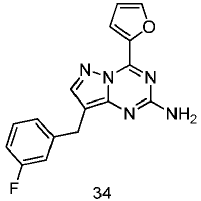 |
| | 8-(3-fluorobenzyl)-4-(furan-2-yl)pyrazolo[1,5-*a*][1,3,5]triazin-2-amine **34** |
| 35 | 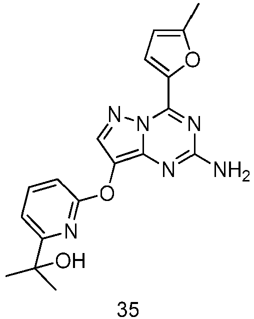 |
| | 2-(6-((2-amino-4-(5-methylfuran-2-yl)pyrazolo[1,5-*a*][1,3,5]triazin-8-yl)ox y)pyridin-2-yl)propan-2-ol **35** |
| 35a | 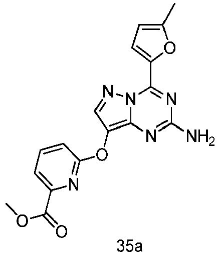 |
| | methyl 6-((2-amino-4-(5-methylfuran-2-yl)pyrazolo[1,5-*a*][1,3,5]triazin-8-yl)oxy) picolinate **35a** |
| 36 | 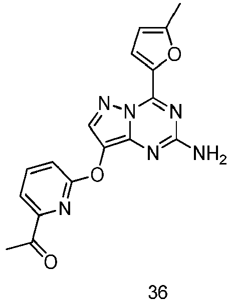 |
| | 1-(6-((2-amino-4-(5-methylfuran-2-yl)pyrazolo[1,5-*a*] [1,3,5]triazin-8-yl)ox y)pyridin-2-yl)ethan-l-one **36** |
| 37 | 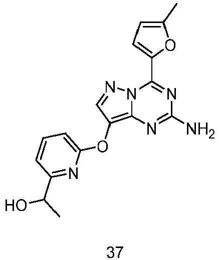 |
| | 1-(6-((2-amino-4-(5-methylfuran-2-yl)pyrazolo[1,5-*a*][1,3,5]triazin-8-yl)ox y)pyridin-2-yl)ethan-1-ol **37** |
| 38a | 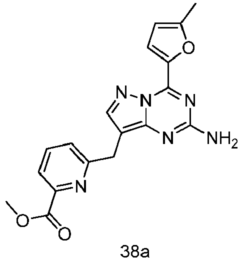 |
| | methyl 6-((2-amino-4-(5-methylfuran-2-yl)pyrazolo[1,5-*a*][1,3,5]triazin-8-yl)meth yl)picolinate **38a** |
| 38 | 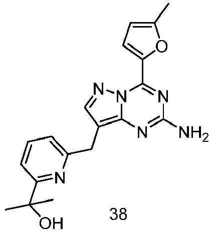 |
| | 2-(6-((2-amino-4-(5-methylfuran-2-yl)pyrazolo[1,5-*a*][1,3,5]triazin-8-yl)m ethyl)pyridin-2-yl)propan-2-ol **38** |

or a tautomer, mesomer, racemate, enantiomer or diastereomer thereof, or a mixture thereof, or a pharmaceutically acceptable salt thereof.

In another aspect, the present disclosure relates to a compound of formula (IA), 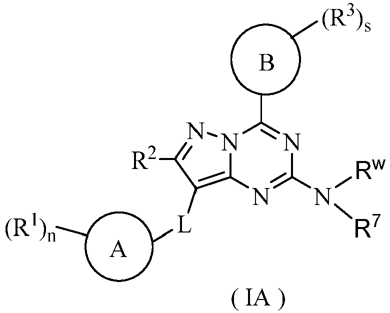 or a tautomer, mesomer, racemate, enantiomer or diastereomer thereof, or a mixture thereof, or a pharmaceutically acceptable salt thereof,
wherein:
R^{w} is an amino protecting group, preferably *tert-butyl* or *tert*-butoxycarbonyl;
R⁷ is hydrogen or R^{w};
L is selected from the group consisting of CR⁴R⁵, O, NH and S;
ring A and ring B are identical or different and are each independently selected from the group consisting of cycloalkyl, heterocyclyl, aryl and heteroaryl;
R¹ is identical or different and is each independently selected from the group consisting of hydrogen, halogen, alkyl, alkoxy, haloalkyl, haloalkoxy, hydroxyl, hydroxyalkyl, cyano, amino, nitro, cycloalkyl, heterocyclyl, heterocyclylalkyl, heterocyclyloxy, aryl, heteroaryl and -Y-R^{a};
Y is a covalent bond or alkylene;
R^{a} is selected from the group consisting of hydrogen, halogen, alkyl, alkoxy, haloalkyl, haloalkoxy, hydroxyl, hydroxyalkyl, cyano, amino, nitro, cycloalkyl, heterocyclyl, heterocyclylalkyl, heterocyclyloxy, -OR^{c}, -C(O)R⁹, -C(O)OR⁹, -OS(O)ₘR⁶, aryl and heteroaryl, wherein the alkyl, alkoxy, cycloalkyl, heterocyclyl, heterocyclylalkyl, heterocyclyloxy, aryl and heteroaryl are each optionally substituted by one or more substituents independently selected from the group consisting of halogen, alkyl, alkoxy, haloalkyl, hydroxyl, hydroxyalkyl, cyano, amino, nitro, cycloalkyl, heterocyclyl, heterocyclyloxy, aryl, heteroaryl and -OS(O)ₘR⁶;
R^{c} is selected from the group consisting of hydrogen, alkyl, haloalkyl, hydroxyalkyl, cycloalkyl and heterocyclyl, wherein the alkyl, cycloalkyl and heterocyclyl are each optionally substituted by one or more substituents independently selected from the group consisting of halogen, alkyl, alkoxy, haloalkyl, hydroxyl, hydroxyalkyl, cyano, amino, nitro, cycloalkyl and heterocyclyl;
R² is selected from the group consisting of hydrogen, halogen, alkyl, alkoxy, haloalkyl, hydroxyl, hydroxyalkyl, cyano, amino, nitro, cycloalkyl, heterocyclyl, aryl and heteroaryl;
R³ is selected from the group consisting of hydrogen, halogen, alkyl, alkoxy, haloalkyl, hydroxyl, hydroxyalkyl, cyano, amino, nitro, cycloalkyl, heterocyclyl, aryl and heteroaryl;
R⁴ and R⁵ are identical or different and are each independently selected from the group consisting of hydrogen, halogen, alkyl, alkoxy, haloalkyl, hydroxyl and hydroxyalkyl;
or, R⁴ and R⁵ together with each other form =NH or =O;
R⁶ is selected from the group consisting of hydrogen, halogen, alkoxy, haloalkoxy, amino, cycloalkyl, heterocyclyl, aryl and heteroaryl;
R⁹ is selected from the group consisting of hydrogen, alkyl, haloalkyl, alkoxy, haloalkoxy, amino, cycloalkyl, heterocyclyl, aryl and heteroaryl;
n is 0, 1, 2, 3 or 4;
s is 0, 1, 2 or 3; and
m is 1 or 2.

The compound of formula (IA) or a tautomer, a racemate, a racemate, an enantiomer or a diastereomer, or a mixture thereof, or a pharmaceutically acceptable salt thereof, wherein each group can be as defined in the formula (I).

Typical compounds of the present disclosure include, but are not limited to:

| Example No. | Structure and name of the compound |
|---|---|
| 1m | 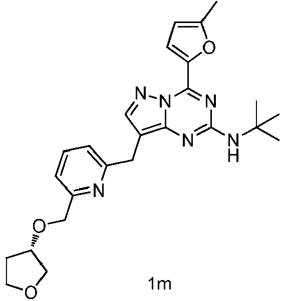 |
| | (*S*)-*N*-(*tert*-butyl)-4-(5-methylfuran-2-yl)-8-((6-(((tetrahydrofuran-3-yl)ox y)methyl)pyridin-2-yl)methyl)pyrazolo[1,5-*a*][1,3,5]triazin-2-amine **1m** |
| 11 | 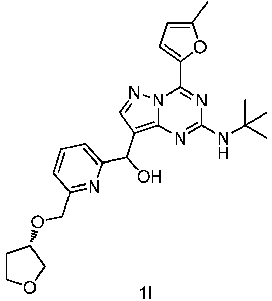 |
| | (2-(*tert*-butylamino)-4-(5-methylfuran-2-yl)pyrazolo[1,5-*a*][1,3,5]triazin-8 -yl)(6-((((*S*)-tetrahydrofuran-3-yl)oxy)methyl)pyridin-2-yl)methanol **11** |
| 2d | 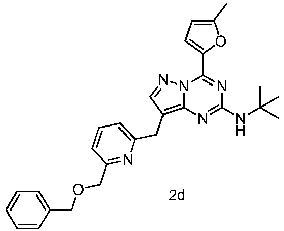 |
| | 8-((6-((benzyloxy)methyl)pyridin-2-yl)methyl)-*N*-(*tert*-butyl)-4-(5-methyl furan-2-yl)pyrazolo[1,5-*a*] [1,3,5]triazin-2-amine **2d** |
| 2c | 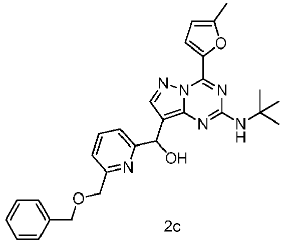 |
| | (6-((benzyloxy)methyl)pyridin-2-yl)(2-(*tert*-butylamino)-4-(5-methylfuran -2-yl)pyrazolo[1,5-*a*] [1,3,5]triazin-8-yl)methanol **2c** |
| 4c | 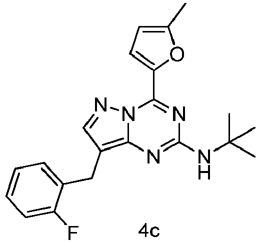 |
| | *N*-(*tert*-butyl)-8-(2-fluorobenzyl)-4-(5-methylfuran-2-yl)pyrazolo[1,5-*a*][1 ,3,5]triazin-2-amine **4c** |
| 4b | 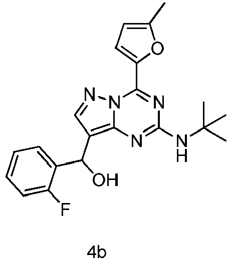 |
| | (2-(*tert*-butylamino)-4-(5-methylfuran-2-yl)pyrazolo[1,5-*a*][1,3,5]triazin-8 -yl)(2-fluorophenyl)methanol **4b** |
| 10g | 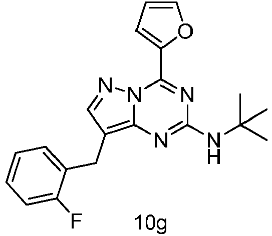 |
| | *N*-(*tert*-butyl)-8-(2-fluorobenzyl)-4-(furan-2-yl)pyrazolo[1,5-*a*][1,3,5]triaz in-2-amine **10g** |
| 10f | 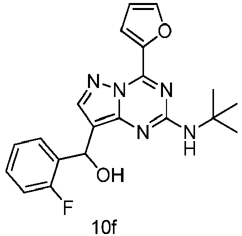 |
| | (2-(*tert*-butylamino)-4-(furan-2-yl)pyrazolo[1,5-*a*][1,3,5]triazin-8-yl)(2-fl uorophenyl)methanol **10f** |
| 12g | 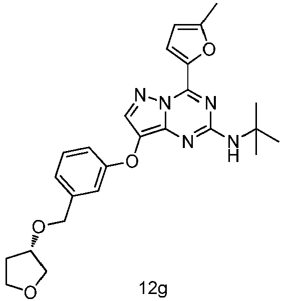 |
| | (*S*)-*N*-(*tert*-butyl)-4-(5-methylfuran-2-yl)-8-(3-(((tetrahydrofuran-3-yl)oxy )methyl)phenoxy)pyrazolo[1,5-*a*][1,3,5]triazin-2-amine **12g** |
| 16i | 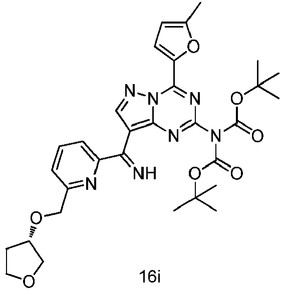 |
| | *tert-butyl N*-*tert*-butoxycarbonyl-*N*-[4-(5-methyl-2-furyl)-8-[6-[[(3*S*)-tetrahydrofura n-3-yl]oxymethyl]pyridine-2-carboximidoyl]pyrazolo[1,5-*a*][1,3,5]triazin-2-yl]carbamate **16i** |
| 18b | 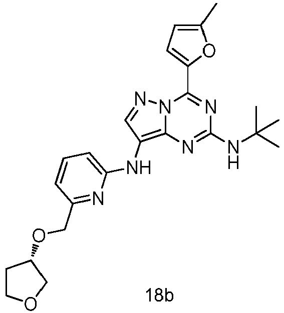 |
| | (*S*)-*N*²-(*tert*-butyl)-4-(5-methylfuran-2-yl)-*N*⁸-(6-(((tetrahydrofuran-3-yl)o xy)methyl)pyridin-2-yl)pyrazolo[1,5-*a*][1,3,5]triazine-2,8-diamine **18b** |
| 19f | 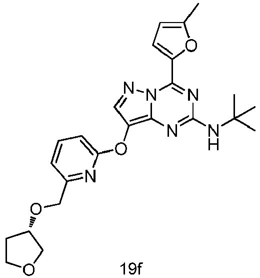 |
| | (*S*)-*N*-(*tert*-butyl)-4-(5-methylfuran-2-yl)-8-((6-(((tetrahydrofuran-3-yl)ox y)methyl)pyridin-2-yl)oxy)pyrazolo[1,5-*a*][1,3,5]triazin-2-amine **19f** |
| 20h | 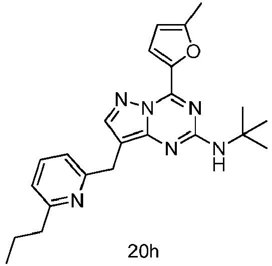 |
| | *N*-(*tert*-butyl)-4-(5-methylfuran-2-yl)-8-((6-propylpyridin-2-yl)methyl)pyr azolo[1,5-*a*][1,3,5]triazin-2-amine **20h** |
| 20g | 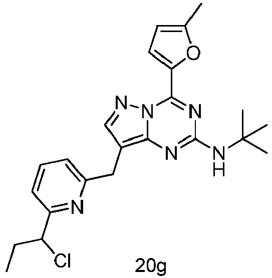 |
| | *N*-(*tert*-butyl)-8-((6-(1-chloropropyl)pyridin-2-yl)methyl)-4-(5-methylfura n-2-yl)pyrazolo[1,5-*a*] [1,3,5]triazin-2-amine **20g** |
| 20f | 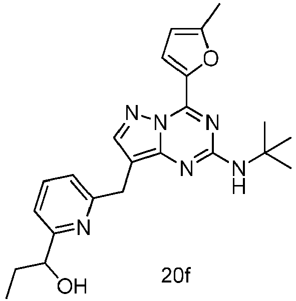 |
| | 1-(6-((2-(*tert*-butylamino)-4-(5-methylfuran-2-yl)pyrazolo[1,5-*a*][1,3,5]tri azin-8-yl)methyl)pyridin-2-yl)propan-1-ol**20f** |
| 26d | 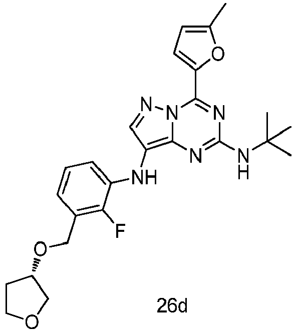 |
| | (*S*)-*N*²-(*tert*-butyl)-*N*⁸-(2-fluoro-3-(((tetrahydrofuran-3-yl)oxy)methyl)phe nyl)-4-(5-methylfuran-2-yl)pyrazolo[1,5-*a*][1,3,5]triazine-2,8-diamine **26d** |
| 28e | 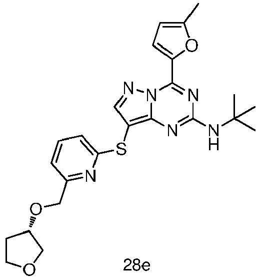 |
| | (*S*)-*N*-(*tert*-butyl)-4-(5-methylfuran-2-yl)-8-((6-(((tetrahydrofuran-3-yl)ox y)methyl)pyridin-2-yl)thio)pyrazolo[1,5-*a*][1,3,5]triazin-2-amine **28e** |
| 31b | 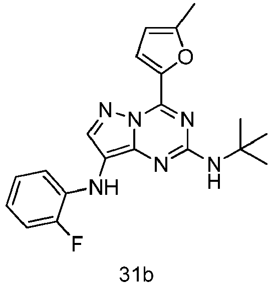 |
| | *N*²-(*tert*-butyl)-*N*⁸-(2-fluorophenyl)-4-(5-methylfuran-2-yl)pyrazolo[1,5-*a*] [1,3,5]triazine-2,8-diamine **31b** |
| 34b | 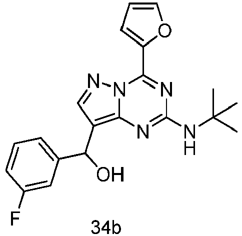 |
| | (2-(*tert*-butylamino)-4-(furan-2-yl)pyrazolo[1,5-*a*][1,3,5]triazin-8-yl)(3-fl uorophenyl)methanol **34b** |
| 34c | 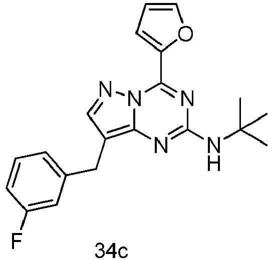 |
| | *N*-(*tert*-butyl)-8-(3-fluorobenzyl)-4-(furan-2-yl)pyrazolo[1,5-*a*][1,3,5]triaz in-2-amine **34c** |

In another aspect, the present disclosure relates to a method for preparing the compound of formula (I), comprising a step of: 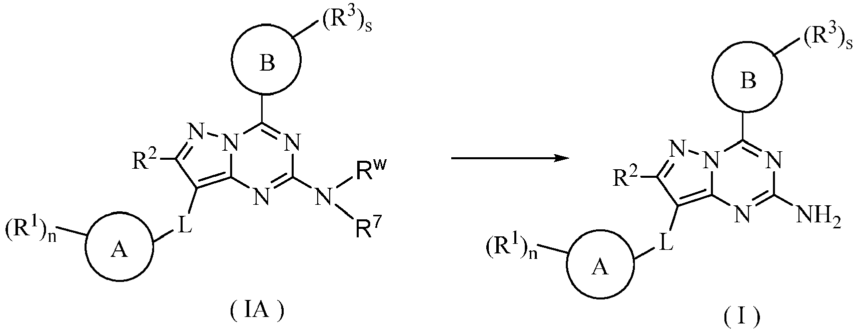 removing the amino protecting group from a compound of formula (IA) to obtain the compound of formula (I);
wherein:
R^{w} is an amino protecting group, preferably *tert-butyl* or *tert*-butoxycarbonyl;
R⁷ is hydrogen or R^{w};
ring A, ring B, L, R¹-R³, n and s are as defined in the formula (I).

In another aspect, the present disclosure relates to a method for preparing the compound of formula (II), comprising a step of: 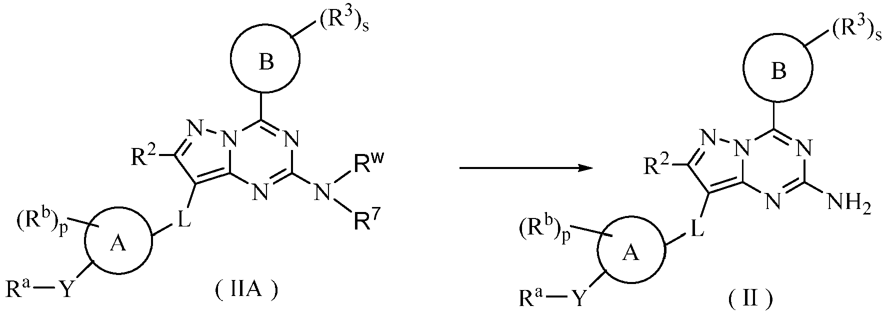 removing the amino protecting group from a compound of formula (IIA) to obtain the compound of formula (II); wherein:
R^{w} is an amino protecting group, preferably *tert-butyl* or *tert*-butoxycarbonyl;
R⁷ is hydrogen or R^{w};
ring A, ring B, L, Y, R^{a}, R^{b}, R², R³, p and s are as defined in the formula (II).

In another aspect, the present disclosure relates to a method for preparing the compound of formula (III), comprising a step of: 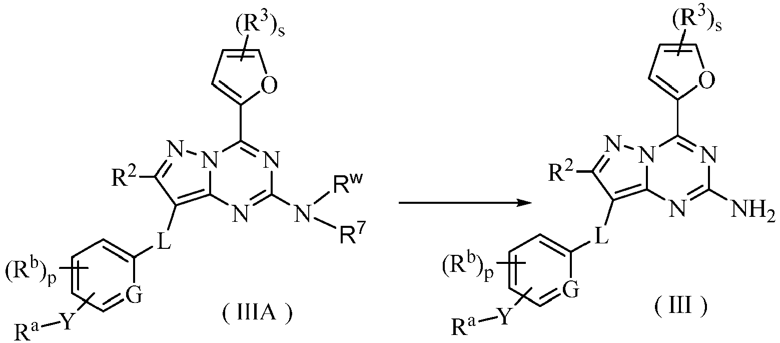 removing the amino protecting group from a compound of formula (IIIA) to obtain the compound of formula (III); wherein:
R^{w} is an amino protecting group, preferably *tert-butyl* or *tert*-butoxycarbonyl;
R⁷ is hydrogen or R^{w};
G is selected from the group consisting of C, CH and N;
L, Y, R^{a}, R^{b}, R², R³, p and s as defined in the formula (III).

In another aspect, the present disclosure relates to a method for preparing the compound of formula (III'), comprising a step of: 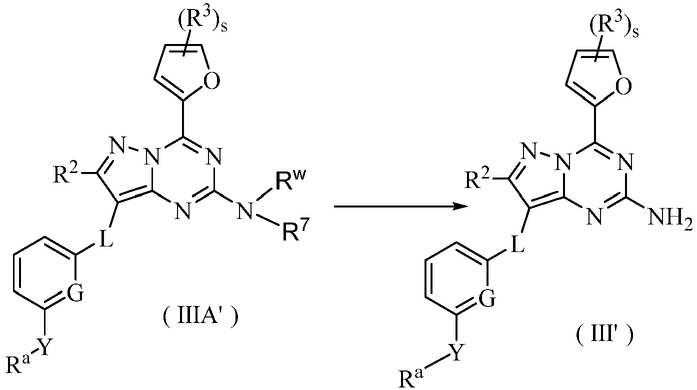 removing the amino protecting group from a compound of formula (IIIA') to obtain the compound of formula (III'); wherein:
R^{w} is an amino protecting group, preferably *tert-butyl* or *tert*-butoxycarbonyl;
R⁷ is hydrogen or R^{w};
G is selected from the group consisting of C, CH and N;
L, Y, R^{a}, R², R³ and s as defined in formula (III').

In another aspect, the present disclosure relates to a method for preparing the compound of formula (IV), comprising a step of: 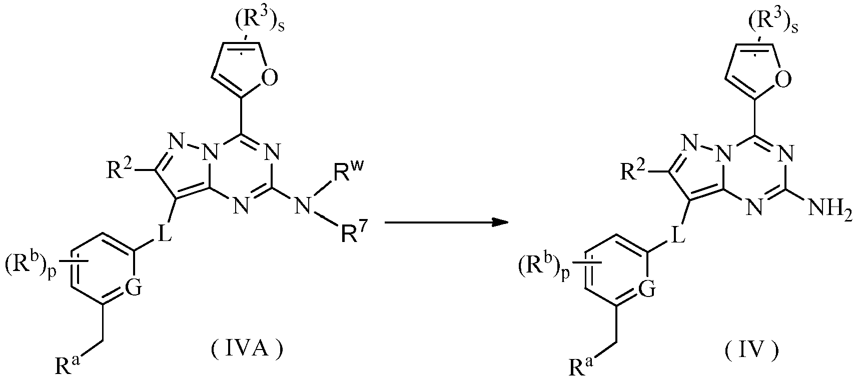 removing the amino protecting group from a compound of formula (IVA) to obtain the compound of formula (IV); wherein:
R^{w} is an amino protecting group, preferably *tert-butyl* or *tert*-butoxycarbonyl;
R⁷ is hydrogen or R^{w};
G is selected from the group consisting of C, CH and N;
L, R^{a}, R^{b}, R², R³, p and s as defined in the formula (IV).

In another aspect, the present disclosure relates to a pharmaceutical composition comprising a therapeutically effective amount of the compound of formula (I) or a tautomer, mesomer, racemate, enantiomer or diastereomer thereof, or a mixture thereof, or a pharmaceutically acceptable salt thereof, and one or more pharmaceutically acceptable carriers, diluents or excipients.

The present disclosure further relates to a use of the compound of formula (I) or a tautomer, mesomer, racemate, enantiomer or diastereomer thereof, or a mixture thereof, or a pharmaceutically acceptable salt thereof, or a pharmaceutical composition comprising the same, in the preparation of a medicament for inhibiting an A₂ₐ receptor.

The present disclosure further relates to a use of the compound of formula (I) or a tautomer, mesomer, racemate, enantiomer or diastereomer thereof, or a mixture thereof, or a pharmaceutically acceptable salt thereof, or a pharmaceutical composition comprising the same, in the preparation of a medicament for treating a disease or condition ameliorated by the inhibition of an A₂ₐ receptor.

In the context of the present disclosure, the disease or condition ameliorated by the inhibition of an A₂ₐ receptor is selected from the group consisting of tumor, depression, cognitive dysfunction, neurodegenerative disorder (Parkinson's disease, Huntington's disease, Alzheimer's disease or amyotrophic lateral sclerosis and the like), attention-related disorder, extrapyramidal syndrome, abnormal movement disorder, cirrhosis, liver fibrosis, fatty liver, dermal fibrosis, sleep disorder, stroke, brain injury, neuroinflammation and addictive behavior; preferably tumor.

The present disclosure further relates to a use of the compound of formula (I) or a tautomer, mesomer, racemate, enantiomer or diastereomer thereof, or a mixture thereof, or a pharmaceutically acceptable salt thereof, or a pharmaceutical composition comprising the same, in the preparation of a medicament for treating tumor, depression, cognitive dysfunction, neurodegenerative disorder (Parkinson's disease, Huntington's disease, Alzheimer's disease, or amyotrophic lateral sclerosis, *etc*.), aattention-related disorder, extrapyramidal syndrome, abnormal movement disorder, liver cirrhosis, liver fibrosis, fatty liver, skin fibrosis, sleep disorder, stroke, brain injury, neuroinflammation and addictive behaviors, preferably in the preparation of a medicament for treating tumor.

The present disclosure further relates to a use of the compound of formula (I) or a tautomer, mesomer, racemate, enantiomer or diastereomer thereof, or a mixture thereof, or a pharmaceutically acceptable salt thereof, or a pharmaceutical composition comprising the same, in the preparation of a medicament for treating tumor.

The present disclosure also relates to a method of inhibiting an A₂ₐ receptor, comprising administering to a patient in need thereof a therapeutically effective amount of the compound of formula (I) or a tautomer, mesomer, racemate, enantiomer or diastereomer thereof, or a mixture thereof, or a pharmaceutically acceptable salt thereof, or a pharmaceutical composition comprising the same.

The present disclosure also relates to a method of treating a condition or disorder ameliorated by the inhibition of an A₂ₐ receptor, comprising administering to a patient in need thereof a therapeutically effective amount of the compound of formula (I) or a tautomer, mesomer, racemate, enantiomer or diastereomer thereof, or a mixture thereof, or a pharmaceutically acceptable salt thereof, or a pharmaceutical composition comprising the same.

The present disclosure relates to a method for treating tumor, depression, cognitive dysfunction, neurodegenerative disorder (Parkinson's disease, Huntington's disease, Alzheimer's disease or amyotrophic lateral sclerosis and the like), attention-related disorder, extrapyramidal syndrome, abnormal movement disorder, cirrhosis, liver fibrosis, fatty liver, dermal fibrosis, sleep disorder, stroke, brain injury, neuroinflammation and addictive behavior, and preferably tumor, comprising administering to a patient in need thereof a therapeutically effective amount of the compound of formula (I), or a tautomer, mesomer, racemate, enantiomer or diastereomer thereof, or a mixture thereof, or a pharmaceutically acceptable salt thereof, or a pharmaceutical composition comprising the same.

The present disclosure further relates to the compound of formula (I), or a tautomer, mesomer, racemate, enantiomer or diastereomer thereof, or a mixture thereof, or a pharmaceutically acceptable salt thereof, or a pharmaceutical composition comprising the same, for use as a medicament.

The present disclosure also relates to the compound of formula (I), or a tautomer, mesomer, racemate, enantiomer or diastereomer thereof, or a mixture thereof, or a pharmaceutically acceptable salt thereof, or a pharmaceutical composition comprising the same, for use as an A₂ₐ receptor antagonist.

The present disclosure also relates to the compound of formula (I), or a tautomer, mesomer, racemate, enantiomer or diastereomer thereof, or a mixture thereof, or a pharmaceutically acceptable salt thereof, or a pharmaceutical composition comprising the same, for use in treating a disease or condition ameliorated by the inhibition of an A₂ₐ receptor.

The present disclosure also relates to the compound of formula (I), or a tautomer, mesomer, racemate, enantiomer or diastereomer thereof, or a mixture thereof, or a pharmaceutically acceptable salt thereof, or a pharmaceutical composition comprising the same, for use in treating tumor, depression, cognitive function disorder, neurodegenerative disorder (Parkinson's disease, Huntington's disease, Alzheimer's disease or amyotrophic lateral sclerosis and the like), attention-related disorder, extrapyramidal syndrome, abnormal movement disorder, cirrhosis, liver fibrosis, fatty liver, dermal fibrosis, sleep disorder, stroke, brain injury, neuroinflammation and addictive behavior, and preferably tumor.

The present disclosure also relates to the compound of formula (I), or a tautomer, mesomer, racemate, enantiomer or diastereomer thereof, or a mixture thereof, or a pharmaceutically acceptable salt thereof, or a pharmaceutical composition comprising the same, for use in treating tumor.

The tumor described in the present disclosure disclosure is selected from the group consisting of melanoma, brain tumor, esophageal cancer, stomach cancer, liver cancer, pancreatic cancer, colorectal cancer, lung cancer, kidney cancer, breast cancer, ovarian cancer, prostate cancer, skin cancer, neuroblastoma, sarcoma, osteochondroma, osteoma, osteosarcoma, seminoma, testicular tumor, uterine cancer, head and neck tumor, multiple myeloma, malignant lymphoma, polycythemia vera, leukemia, thyroid tumor, ureteral tumor, bladder tumor, gallbladder cancer, cholangiocarcinoma, chorionic epithelioma and pediatric tumor; preferably lung cancer.

Pharmaceutical compositions containing the active ingredient can be in a form suitable for oral administration, for example, a tablet, troche, lozenge, aqueous or oily suspension, dispersible powder or granule, emulsion, hard or soft capsule, syrup or elixir. Oral compositions can be prepared according to any known method in the art for the preparation of pharmaceutical compositions. Such composition can contain one or more ingredients selected from the group consisting of sweeteners, flavoring agents, colorants and preservatives, in order to provide a pleasing and palatable pharmaceutical preparation. Tablets contain the active ingredient in admixture with nontoxic pharmaceutically acceptable excipients suitable for the manufacture of tablets. These excipients can be inert excipients, granulating agents, disintegrating agents, binders and lubricants. These tablets may be uncoated or they may be coated by known techniques that provide sustained release for a longer period of time by masking the taste of the drug or delaying disintegration and absorption in the gastrointestinal tract.

Oral formulations can also be provided in soft gelatin capsules in which the active ingredient is mixed with an inert solid diluent or in which the active ingredient is mixed with a water-soluble carrier or oil vehicle.

An aqueous suspension contains the active ingredient in admixture with excipients suitable for the manufacture of an aqueous suspension. Such excipients are suspending agents, dispersing agents or wetting agents. The aqueous suspension can also contain one or more preservatives such as ethyl paraben or *n*-propyl paraben, one or more colorants, one or more flavoring agents, and one or more sweeteners.

An oil suspension can be formulated by suspending the active ingredient in a vegetable oil or mineral oil. The oil suspension can contain a thickener. The aforementioned sweeteners and flavoring agents can be added to provide a palatable formulation. These compositions can be preserved by adding antioxidants.

The active ingredient in admixture with the dispersants or wetting agents, suspending agent or one or more preservatives can be prepared as a dispersible powder or granule suitable for the preparation of an aqueous suspension by adding water. Suitable dispersants or wetting agents and suspending agents are exemplified by those already mentioned above. Additional excipients, such as sweeteners, flavoring agents and colorants, can also be added. These compositions can be preserved by adding an antioxidant, such as ascorbic acid.

The pharmaceutical composition of the present disclosure can also be in the form of an oil-in-water emulsion. The oil phase may be vegetable oil, or mineral oil or a mixture thereof. A suitable emulsifier may be a naturally-occurring phospholipid, and the emulsion may also contain sweeteners, flavoring agents, preservatives and antioxidants. Such preparations may also contain a demulcent, a preservative, a coloring agent and an antioxidant.

The pharmaceutical composition can be in the form of a sterile injectable aqueous solution. Acceptable vehicles or solvents that can be used are water, Ringer's solution or isotonic sodium chloride solution. The sterile injectable formulation can be a sterile injectable oil-in-water micro-emulsion in which the active ingredient is dissolved in the oil phase. The injectable solution or micro-emulsion can be introduced into a patient's bloodstream by local bolus injection. Alternatively, the solution and micro-emulsion are preferably administered in a manner that maintains a constant circulating concentration of the compound of the present disclosure. In order to maintain this constant concentration, a continuous intravenous delivery device can be used. An example of such a device is Deltec CADD-PLUS. TM. 5400 intravenous injection pump.

The pharmaceutical composition can be in the form of a sterile injectable aqueous or oily suspension for intramuscular and subcutaneous administration. Such a suspension can be formulated with suitable dispersants or wetting agents and suspending agents as described above according to known techniques. The sterile injectable formulation can also be a sterile injectable solution or suspension prepared in a nontoxic parenterally acceptable diluent or solvent. Moreover, sterile fixed oils can easily be used as a solvent or suspending medium. For this purpose, any blended fixing oil can be used. In addition, fatty acids can also be prepared for injection.

The compound of the present disclosure can be administered in the form of a suppository for rectal administration. These pharmaceutical compositions can be prepared by mixing the drug with a suitable non-irritating excipient that is solid at ordinary temperatures, but liquid in the rectum, thereby melting in the rectum to release the drug.

It is well known to those skilled in the art that the dosage of a drug depends on a variety of factors including but not limited to, the following factors: activity of a specific compound, age of the patient, weight of the patient, general health of the patient, behavior of the patient, diet of the patient, administration time, administration route, excretion rate, drug combination and the like. In addition, the optimal treatment, such as treatment mode, daily dose of the compound of formula (I) or the type of pharmaceutically acceptable salt thereof can be verified by traditional therapeutic regimens.

### DETAILED DESCRIPTION OF THE DISCLOSURE

Unless otherwise stated, the terms used in the specification and claims have the meanings described below.

The term "alkyl" refers to a saturated aliphatic hydrocarbon group, which is a straight or branched chain group comprising 1 to 20 carbon atoms, preferably an alkyl having 1 to 12 carbon atoms, and more preferably an alkyl having 1 to 6 carbon atoms. Non-limiting examples include methyl, ethyl, *n*-propyl, isopropyl, *n*-butyl, isobutyl, *tert*-butyl, *sec*-butyl, *n*-pentyl, 1,1-dimethylpropyl, 1,2-dimethylpropyl, 2,2-dimethylpropyl, 1-ethylpropyl, 2-methylbutyl, 3-methylbutyl, *n*-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, *n*-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, *n*-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, *n*-nonyl, 2-methyl-2-ethylhexyl, 2-methyl-3-ethylhexyl, 2,2-diethylpentyl, *n*-decyl, 3,3-diethylhexyl, 2,2-diethylhexyl, and various branched isomers thereof. More preferably, the alkyl group is a lower alkyl having 1 to 6 carbon atoms, and non-limiting examples include methyl, ethyl, *n*-propyl, isopropyl, *n*-butyl, isobutyl, *tert*-butyl, *sec*-butyl, *n*-pentyl, 1,1-dimethylpropyl, 1,2-dimethylpropyl, 2,2-dimethylpropyl, 1-ethylpropyl, 2-methylbutyl, 3-methylbutyl, *n*-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, and the like. The alkyl group can be substituted or unsubstituted. When substituted, the substituent group(s) can be substituted at any available connection point. The substituent group(s) is preferably one or more groups independently optionally selected from the group consisting of hydrogen, halogen, alkyl, alkoxy, haloalkyl, hydroxyl, hydroxyalkyl, cyano, amino, nitro, cycloalkyl, heterocyclyl, aryl, heteroaryl and -OS(O)ₘR⁶.

The term "alkylene" refers to a saturated straight-chain or branched-chain aliphatic hydrocarbon group derived from a parent alkane by removal of two hydrogen atoms from the same carbon atom or two different carbon atoms, which is a straight or branched chain group containing 1 to 20 carbon atoms. The alkylene preferably contains 1 to 12 carbon atoms, and more preferably contains 1 to 6 carbon atoms. Non-limiting examples of the alkylene include, but are not limited to, methylene (-CH₂-), 1,1-ethylene (-CH(CH₃)-), 1,2-ethylene (-CH₂CH₂-), 1,1-propylene (-CH(CH₂CH₃)-), 1,2-propylene (-CH₂CH(CH₃)-), 1,3-propylene (-CH₂CH₂CH₂-), 1,4- butylene (-CH₂CH₂CH₂CH₂-) and 1,5-butylene (-CH₂CH₂CH₂CH₂CH₂-) and so on. The alkylene group can be substituted or unsubstituted. When substituted, the substituent group(s) can be substituted at any available connection point, and the substituent group(s) is preferably one or more groups independently optionally selected from the group consisting of alkyl, alkenyl, alkynyl, alkoxy, alkylthio, alkylamino, halogen, mercapto, hydroxyl, nitro, cyano, cycloalkyl, heterocyclyl, aryl, heteroaryl, cycloalkoxy, heterocycloalkoxy, cycloalkylthio, heterocycloalkylthio, oxo and -OS(O)ₘR⁶.

The term "alkoxy" refers to an -O-(alkyl) or an -O-(unsubstituted cycloalkyl) group, wherein the alkyl is as defined above. Non-limiting examples of the alkoxy include methoxy, ethoxy, propoxy, butoxy, cyclopropyloxy, cyclobutyloxy, cyclopentyloxy, cyclohexyloxy. The alkoxy can be optionally substituted or unsubstituted. When substituted, the substituent group(s) is preferably one or more groups independently selected from the group consisting of hydrogen, halogen, alkyl, alkoxy, haloalkyl, hydroxyl, hydroxyalkyl, cyano, amino, nitro, cycloalkyl, heterocyclyl, aryl, heteroaryl and -OS(O)ₘR⁶.

The term "cycloalkyl" refers to a saturated or partially unsaturated monocyclic or polycyclic hydrocarbon substituent group having 3 to 20 carbon atoms, preferably 3 to 12 carbon atoms, more preferably 3 to 10 carbon atoms, and most preferably 3 to 6 (for example 3, 4, 5 or 6) carbon atoms. Non-limiting examples of monocyclic cycloalkyl include cyclopropyl, cyclobutyl, cyclopentyl, cyclopentenyl, cyclohexyl, cyclohexenyl, cyclohexadienyl, cycloheptyl, cycloheptatrienyl, cyclooctyl and the like. Polycyclic cycloalkyl includes spiro cycloalkyl, fused cycloalkyl and bridged cycloalkyl.

The term "spiro cycloalkyl" refers to a 5 to 20 membered polycyclic group with individual rings connected through one shared carbon atom (called a spiro atom), wherein the rings can contain one or more double bonds, but none of the rings has a completely conjugated π-electron system. The spiro cycloalkyl is preferably 6 to 14 membered spiro cycloalkyl, and more preferably 7 to 10 membered spiro cycloalkyl. According to the number of the spiro atoms shared between the rings, the spiro cycloalkyl can be divided into mono-spiro cycloalkyl, di-spiro cycloalkyl, or poly-spiro cycloalkyl, and the spiro cycloalkyl is preferably mono-spiro cycloalkyl or di-spiro cycloalkyl, and more preferably 4-membered/4-membered, 4-membered/5-membered, 4-membered/6-membered, 5-membered/5-membered, or 5-membered/6-membered mono-spiro cycloalkyl. Non-limiting examples of spiro cycloalkyl include:

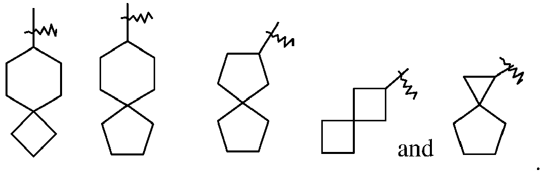

The term "fused cycloalkyl" refers to a 5 to 20 membered all-carbon polycyclic group, wherein each ring in the system shares an adjacent pair of carbon atoms with another ring, wherein one or more rings can contain one or more double bonds, but none of the rings has a completely conjugated π-electron system. The fused cycloalkyl is preferably 6 to 14 membered fused cycloalkyl, and more preferably 7 to 10 membered fused cycloalkyl. According to the number of the rings, the fused cycloalkyl can be divided into bicyclic, tricyclic, tetracyclic or polycyclic fused cycloalkyl, and the fused cycloalkyl is preferably bicyclic or tricyclic fused cycloalkyl, and more preferably 5-membered/5-membered, or 5-membered/6-membered bicyclic fused cycloalkyl. Non-limiting examples of the fused cycloalkyl include:

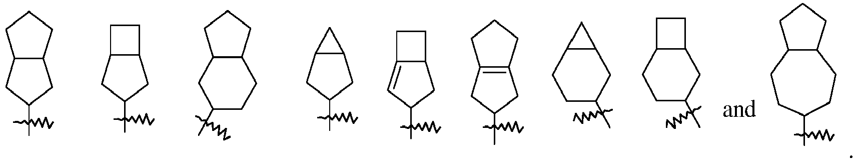

The term "bridged cycloalkyl" refers to a 5 to 20 membered all-carbon polycyclic group, wherein every two rings in the system share two disconnected carbon atoms, wherein the rings can have one or more double bonds, but none of the rings has a completely conjugated π-electron system. The bridged cycloalkyl is preferably 6 to 14 membered bridged cycloalkyl, and more preferably 7 to 10 membered bridged cycloalkyl. According to the number of the rings, the bridged cycloalkyl can be divided into bicyclic, tricyclic, tetracyclic or polycyclic bridged cycloalkyl, and the bridged cycloalkyl is preferably bicyclic, tricyclic or tetracyclic bridged cycloalkyl, and more preferably bicyclic or tricyclic bridged cycloalkyl. Non-limiting examples of the bridged cycloalkyl include:

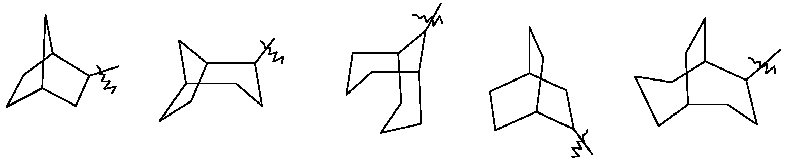

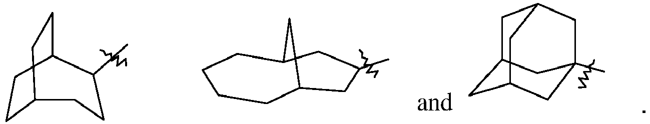

The cycloalkyl ring include the above said cycloalkyl groups (e.g., monocyclic, fused, spiro and bridged cycloalkyl) fused to the ring of aryl, heteroaryl or heterocyclyl, wherein the ring bound to the parent structure is cycloalkyl. Non-limiting examples include indanyl, tetrahydronaphthyl, benzocycloheptyl and the like, preferably benzocyclopentyl and tetrahydronaphthyl.

The cycloalkyl can be substituted or unsubstituted. When substituted, the substituent group(s) can be substituted at any available connection point, and the substituent group(s) is preferably one or more group(s) independently optionally selected from the group consisting of hydrogen, halogen, alkyl, alkoxy, haloalkyl, hydroxyl, hydroxyalkyl, cyano, nitro, amino, cycloalkyl, heterocyclyl, heterocyclyloxy, aryl, heteroaryl and -S(O)ₘR⁶.

The term "heterocyclyl" refers to a 3 to 20 membered saturated or partially unsaturated monocyclic or polycyclic hydrocarbon group, wherein one or more ring atoms are heteroatoms selected from the group consisting of N, O and S(O)ₘ (wherein m is an integer of 0 to 2), but excluding -O-O-, -O-S- or -S-S- in the ring, with the remaining ring atoms being carbon atoms. Preferably, the heterocyclyl has 3 to 12 ring atoms wherein 1 to 4 atoms are heteroatoms, more preferably, 3 to 10 ring atoms wherein 1 to 4 atoms are heteroatoms, and more preferably 5 to 6 ring atoms wherein 1 to 3 atoms are heteroatoms. Non-limiting examples of monocyclic heterocyclyl include pyrrolidinyl, tetrahydropyranyl, 1,2,3,6-tetrahydropyridyl, piperidinyl, piperazinyl, morpholinyl, thiomorpholinyl, homopiperazinyl and the like. Polycyclic heterocyclyl includes spiro heterocyclyl, fused heterocyclyl or bridged heterocyclyl.

The term "spiro heterocyclyl" refers to a 5 to 20 membered polycyclic heterocyclyl group with individual rings connected through one shared atom (called a spiro atom), wherein one or more ring atoms are heteroatoms selected from the group consisting of N, O and S(O)ₘ (wherein m is an integer of 0 to 2), with the remaining ring atoms being carbon atoms, where the rings can contain one or more double bonds, but none of the rings has a completely conjugated π-electron system. The spiro heterocyclyl is preferably 6 to 14 membered spiro heterocyclyl, and more preferably 7 to 10 membered (for example 7, 8, 9 or 10 membered) spiro heterocyclyl. According to the number of the spiro atoms shared between the rings, the spiro heterocyclyl can be divided into mono-spiro heterocyclyl, di-spiro heterocyclyl, or poly-spiro heterocyclyl, and the spiro heterocyclyl is preferably mono-spiro heterocyclyl or di-spiro heterocyclyl, and more preferably 4-membered/4-membered, 4-membered/5-membered, 4-membered/6-membered, 5-membered/5-membered, or 5-membered/6-membered mono-spiro heterocyclyl. Non-limiting examples of the spiro heterocyclyl include:

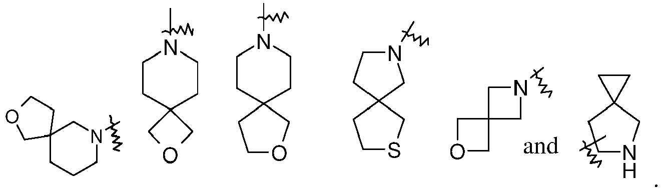

The term "fused heterocyclyl" refers to a 5 to 20 membered polycyclic heterocyclyl group, wherein each ring in the system shares an adjacent pair of atoms with another ring, wherein one or more rings can contain one or more double bonds, but none of the rings has a completely conjugated π-electron system, and wherein one or more ring atoms are heteroatoms selected from the group consisting of N, O and S(O)ₘ (wherein m is an integer of 0 to 2), with the remaining ring atoms being carbon atoms. The fused heterocyclyl is preferably 6 to 14 membered fused heterocyclyl, and more preferably 7 to 10 membered (for example 7, 8, 9 or 10 membered) fused heterocyclyl. According to the number of the rings, the fused heterocyclyl can be divided into bicyclic, tricyclic, tetracyclic or polycyclic fused heterocyclyl, and the fused heterocyclyl is preferably bicyclic or tricyclic fused heterocyclyl, and more preferably 5-membered/5-membered or 5-membered/6-membered bicyclic fused heterocyclyl. Non-limiting examples of the fused heterocyclyl include:

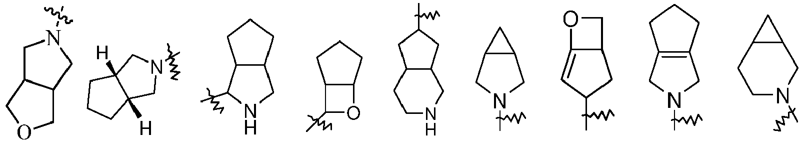

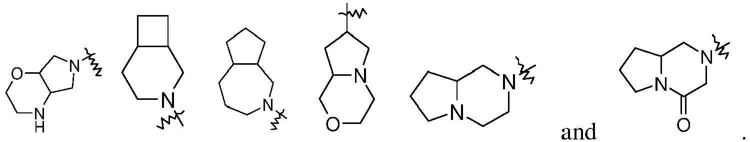

The term "bridged heterocyclyl" refers to a 5 to 14 membered polycyclic heterocyclyl group, wherein every two rings in the system share two disconnected atoms, wherein the rings can have one or more double bonds, but none of the rings has a completely conjugated π-electron system, and wherein one or more ring atoms are heteroatoms selected from the group consisting of N, O and S(O)ₘ (wherein m is an integer of 0 to 2), with the remaining ring atoms being carbon atoms. The bridged heterocyclyl is preferably 6 to 14 membered bridged heterocyclyl, and more preferably 7 to 10 membered (for example 7, 8, 9 or 10 membered) bridged heterocyclyl. According to the number of the rings, the bridged heterocyclyl can be divided into bicyclic, tricyclic, tetracyclic or polycyclic bridged heterocyclyl, and the bridged heterocyclyl is preferably bicyclic, tricyclic or tetracyclic bridged heterocyclyl, and more preferably bicyclic or tricyclic bridged heterocyclyl. Non-limiting examples of the bridged heterocyclyl include:

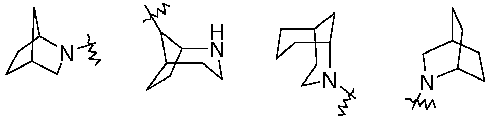

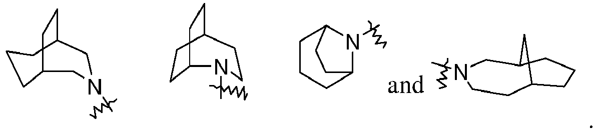

The ring of heterocyclyl include the above said heterocyclyl groups (*e.g*., monocyclic, fused, spiro and bridged heterocyclyl groups) fused to the ring of aryl, heteroaryl or cycloalkyl, wherein the ring bound to the parent structure is heterocyclyl. Non-limiting examples thereof include:

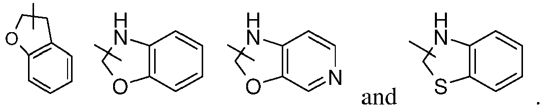

The heterocyclyl can be substituted or unsubstituted. When substituted, the substituent group(s) can be substituted at any available connection point. The substituent group(s) is preferably one or more group(s) independently optionally selected from the group consisting of hydrogen, halogen, alkyl, alkoxy, haloalkyl, hydroxyl, hydroxyalkyl, cyano, amino, nitro, cycloalkyl, heterocyclyl, aryl, heteroaryl and -OS(O)ₘR⁶.

The term "aryl" refers to a 6 to 14 membered all-carbon monocyclic ring or polycyclic fused ring (*i.e*., each ring in the system shares an adjacent pair of carbon atoms with another ring in the system) having a conjugated π-electron system, preferably 6 to 10 membered aryl, for example, phenyl and naphthyl. The ring of aryl include above said aryl fused to the ring of heteroaryl, heterocyclyl or cycloalkyl, wherein the ring bound to the parent structure is aryl ring. Non-limiting examples thereof include:

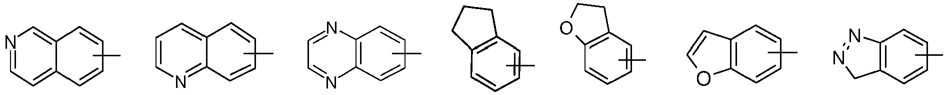

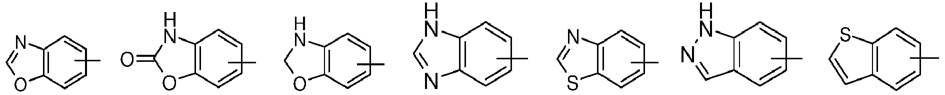

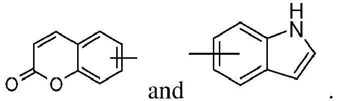

The aryl can be substituted or unsubstituted. When substituted, the substituent group(s) can be substituted at any available connection point. The substituent group(s) is preferably one or more group(s) independently optionally selected from the group consisting of hydrogen, halogen, alkyl, alkoxy, haloalkyl, hydroxyl, hydroxyalkyl, cyano, amino, nitro, cycloalkyl, heterocyclyl, heterocyclyloxy, aryl, heteroaryl and -OS(O)ₘR⁶.

The term "heteroaryl" refers to a 5 to 14 membered heteroaromatic system having 1 to 4 heteroatoms selected from the group consisting of O, S and N. The heteroaryl is preferably 5 to 10 membered heteroaryl, more preferably 5 or 6 membered heteroaryl, for example, furanyl, thienyl, pyridyl, pyrrolyl, *N*-alkylpyrrolyl, pyrimidinyl, pyrazinyl, pyridazinyl, imidazolyl, pyrazolyl, tetrazolyl and the like. The ring of heteroaryl include the above said heteroaryl fused to the ring of aryl, heterocyclyl or cycloalkyl, wherein the ring bound to the parent structure is heteroaryl ring. Non-limiting examples thereof include:

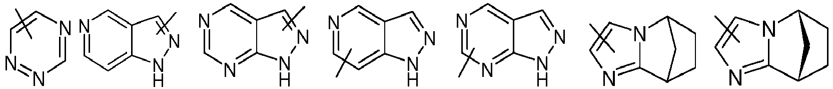

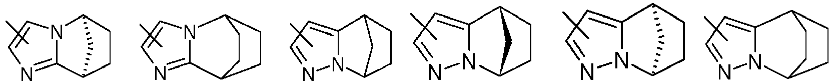

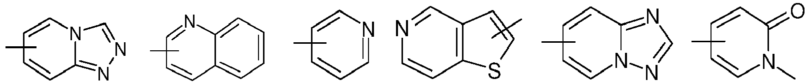

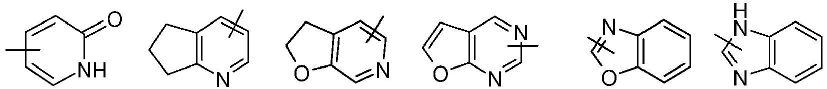

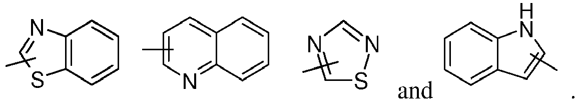

The heteroaryl can be substituted or unsubstituted. When substituted, the substituent group(s) can be substituted at any available connection point. The substituent group(s) is preferably one or more group(s) independently optionally selected from the group consisting of hydrogen, halogen, alkyl, alkoxy, haloalkyl, hydroxyl, hydroxyalkyl, cyano, amino, nitro, cycloalkyl, heterocyclyl, aryl, heteroaryl and -OS(O)ₘR⁶.

The term "amino protecting group" is to keep the amino group unchanged during the reaction of other parts of the molecule, and protect the amino group with a group that can be easily removed. Non-limiting examples include *tert*-butyl, *tert*-butoxycarbonyl, acetyl, benzyl, allyl, p-methoxybenzyl, and the like. These groups can be optionally substituted with 1-3 substituents selected from halogen, alkoxy or nitro. The amino protecting group is preferably *tert-butyl* or *tert*-butoxycarbonyl.

The term "oxo" refers to =O.

The term "heterocyclyloxy" refers to heterocyclyl-O-, wherein the heterocyclyl is as defined above.

The term "heterocyclylalkyl" refers to an alkyl group substituted with one or more heterocyclyl groups, wherein the alkyl and heterocyclyl groups are as defined above.

The term "haloalkyl" refers to an alkyl group substituted by one or more halogens, wherein the alkyl is as defined above.

The term "haloalkoxy" refers to an alkoxy group substituted by one or more halogens, wherein the alkoxy is as defined above.

The term "hydroxy" refers to a -OH group.

The term "hydroxyalkyl" refers to an alkyl group substituted by hydroxyl group(s), wherein the alkyl is as defined above.

The term "halogen" refers to fluorine, chlorine, bromine or iodine.

The term "amino" refers to a -NH₂ group.

The term "cyano" refers to a -CN group.

The term "nitro" refers to a -NO₂ group.

"Optional" or "optionally" means that the event or circumstance described subsequently can, but need not, occur, and such a description includes the situation in which the event or circumstance does or does not occur. For example, "the heterocyclyl optionally substituted by an alkyl" means that an alkyl group can be, but need not be, present, and such a description includes the situation of the heterocyclyl being substituted by an alkyl and the heterocyclyl being not substituted by an alkyl.

"Substituted" refers to one or more hydrogen atoms in a group, preferably up to 5, more preferably 1 to 3 hydrogen atoms, independently substituted by a corresponding number of substituents, each of these substituents has independent options (*i.e*., the substituents can be the same or different). It goes without saying that the substituents only exist in their possible chemical position. The person skilled in the art is able to determine whether the substitution is possible or impossible by experiments or theory without paying excessive efforts. For example, the combination of amino or hydroxy having free hydrogen and carbon atoms having unsaturated bonds (such as olefinic) may be unstable.

A "pharmaceutical composition" refers to a mixture of one or more of the compounds according to the present disclosure or physiologically/pharmaceutically acceptable salts or prodrugs thereof with other chemical components, and other components such as physiologically/pharmaceutically acceptable carriers and excipients. The purpose of the pharmaceutical composition is to facilitate administration of a compound to an organism, which is conducive to the absorption of the active ingredient so as to show biological activity.

A "pharmaceutically acceptable salt" refers to a salt of the compound of the present disclosure, which is safe and effective in mammals and has the desired biological activity.

The compound of the present disclosure can also include isotopic derivatives thereof. The term "isotopic derivative" refers to a compound that differs in structure only in the presence of one or more isotopically enriched atoms. For example, the compounds having the structures of the present disclosure except replacing hydrogen with "deuterium" or "tritium", or repalcing fluorine with an ¹⁸F-fluorine label (¹⁸F isotope), or replacing carbon with ^{l1}C-, ¹³C-, or ¹⁴C-enriched carbon (¹¹C- , ¹³C-, or ¹⁴C-carbon labeling; ¹¹C-, ¹³C-, or ¹⁴C- isotopes) are within the scope of the present disclosure. Such compounds can be used, for example, as analytical tools or probes in biological assays, or as *in vivo* diagnostic imaging tracers for diseases, or as tracers for pharmacodynamics, pharmacokinetics, or receptor studies. Deuterated compounds can generally retain activity comparable to undeuterated compounds, and when deuterated at certain specific sites, better metabolic stability can be achieved, resulting in certain therapeutic advantages (such as increased half-life *in vivo* or reduced required dose).

For drugs or pharmacologically active agents, the term "therapeutically effective amount" refers to a sufficient amount of a drug or medicament that is non-toxic but capable of achieving the desired effect. The determination of the effective amount varies from person to person, depending on the age and general condition of the recipient, and also on the specific active substance. The appropriate effective amount in a case can be determined by those skilled in the art based on routine experiments.

Wherein: R⁶ is as defined in the formula (I).

The present disclosure provides a novel adenosine A₂ₐ receptor antagonist containing a structure of pyrazolo[1,5-*a*][1,3,5]triazin-2-amine , and finds that compounds with such structure have strong inhibitory activity and high selectivity, and compounds with such structure have good pharmacological absorption.

### Synthesis Method for the Compound of the Present Disclosure

In order to achieve the object of the present disclosure, the present disclosure applies the following technical solutions:
A method for preparing the compound of formula (I) of the present disclosure or a tautomer, mesomer, racemate, enantiomer or diastereomer thereof, or a mixture thereof, or a pharmaceutically acceptable salt thereof, comprises a step of: 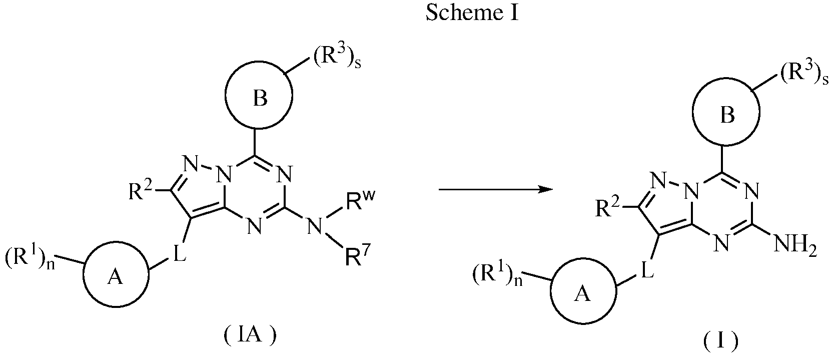 removing the amino protecting group from a compound of the formula (IA) under an acidic condition to obtain the compound of formula (I);
wherein:
R^{w} is an amino protecting group, preferably *tert-butyl* or *tert*-butoxycarbonyl;
R⁷ is hydrogen or R^{w};
ring A, ring B, L, R¹-R³, n and s are as defined in the formula (I).

The reagent that provides the acidic condition include, but is not limited to, hydrogen chloride, a solution of hydrogen chloride in 1,4-dioxane, ammonium chloride, trifluoroacetic acid, formic acid, acetic acid, hydrochloric acid, sulfuric acid, methanesulfonic acid, nitric acid, phosphoric acid, *p*-toluenesulfonic acid and TMSOTf, preferably trifluoroacetic acid;
the above reaction is preferably carried out in a solvent, wherein the solvent used includes, but is not limited to, acetic acid, methanol, ethanol, *n*-butanol, toluene, tetrahydrofuran, dichloromethane, petroleum ether, ethyl acetate, *n*-hexane, dimethyl sulfoxide, 1,4-dioxane, glycol dimethyl ether, water or *N*,*N*-dimethylformamide and a mixture thereof.

A method for preparing the compound of formula (II) of the present disclosure or a tautomer, mesomer, racemate, enantiomer or diastereomer thereof, or a mixture thereof, or a pharmaceutically acceptable salt thereof, comprises a step of: 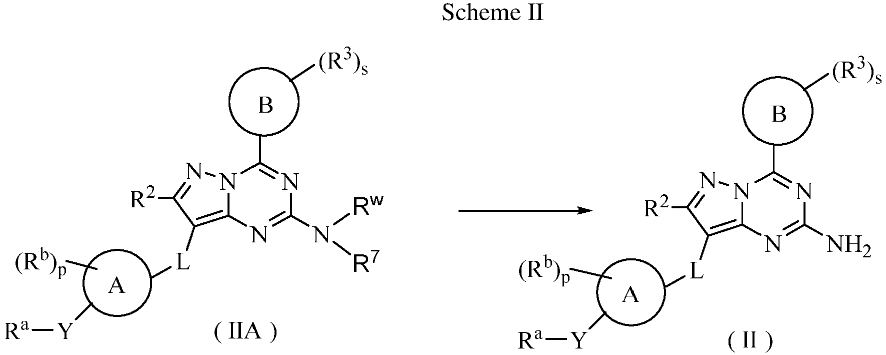 removing the amino protecting group from a compound of formula (IIA) under an acidic condition to obtain the compound of formula (II); wherein:
R^{w} is an amino protecting group, preferably *tert-butyl* or *tert*-butoxycarbonyl;
R⁷ is hydrogen or R^{w};
ring A, ring B, L, Y, R^{a}, R^{b}, R², R³, p and s are as defined in the formula (II).

The reagent that provides the acidic condition include, but is not limited to, hydrogen chloride, a solution of hydrogen chloride in 1,4-dioxane, ammonium chloride, trifluoroacetic acid, formic acid, acetic acid, hydrochloric acid, sulfuric acid, methanesulfonic acid, nitric acid, phosphoric acid, *p*-toluenesulfonic acid and TMSOTf, preferably trifluoroacetic acid;
the above reaction is preferably carried out in a solvent, wherein the solvent used includes, but is not limited to, acetic acid, methanol, ethanol, *n*-butanol, toluene, tetrahydrofuran, dichloromethane, petroleum ether, ethyl acetate, *n*-hexane, dimethyl sulfoxide, 1,4-dioxane, glycol dimethyl ether, water or *N*,*N*-dimethylformamide and a mixture thereof.

A method for preparing the compound of formula (III) of the present disclosure or a tautomer, mesomer, racemate, enantiomer or diastereomer thereof, or a mixture thereof, or a pharmaceutically acceptable salt thereof, comprises a step of: 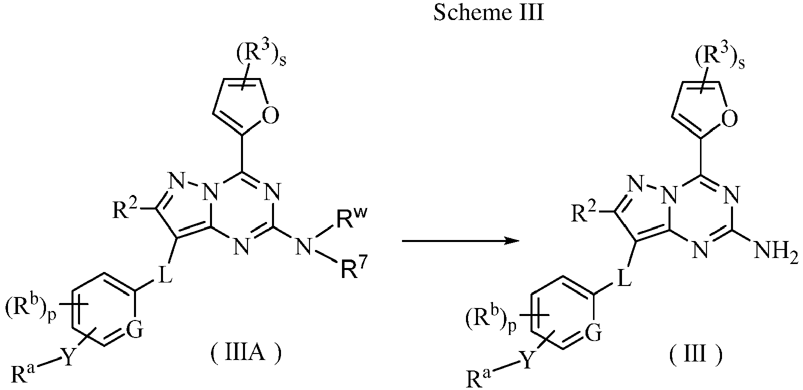 removing the amino protecting group from a compound of formula (IIIA) under an acidic condition to obtain the compound of formula (III), wherein:
R^{w} is an amino protecting group, preferably *tert-butyl* or *tert*-butoxycarbonyl;
R⁷ is hydrogen or R^{w};
G is selected from the group consisting of C, CH and N;
L, Y, R^{a}, R^{b}, R², R³, p and s are as defined in the formula (III).

The reagent that provides the acidic condition include, but is not limited to, hydrogen chloride, a solution of hydrogen chloride in1,4-dioxane, ammonium chloride, trifluoroacetic acid, formic acid, acetic acid, hydrochloric acid, sulfuric acid, methanesulfonic acid, nitric acid, phosphoric acid, p-toluenesulfonic acid and TMSOTf, preferably trifluoroacetic acid;
the above reaction is preferably carried out in a solvent, wherein the solvent used includes, but is not limited to, acetic acid, methanol, ethanol, *n*-butanol, toluene, tetrahydrofuran, dichloromethane, petroleum ether, ethyl acetate, *n*-hexane, dimethyl sulfoxide, 1,4-dioxane, glycol dimethyl ether, water or *N*,*N*-dimethylformamide and a mixture thereof.

A method for preparing the compound of formula (III') of the present disclosure or a tautomer, mesomer, racemate, enantiomer or diastereomer thereof, or a mixture thereof, or a pharmaceutically acceptable salt thereof, comprises a step of: 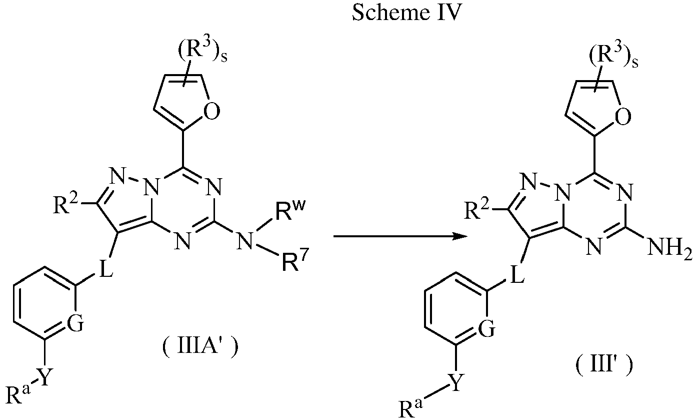 removing the amino protecting group from a compound of formula (IIIA') under an acidic condition to obtain the compound of formula (III'), wherein:
R^{w} is an amino protecting group, preferably *tert-butyl* or *tert*-butoxycarbonyl;
R⁷ is hydrogen or R^{w};
G is selected from the group consisting of C, CH and N;
L, Y, R^{a}, R², R³ and s are as defined in the general formula (III').

The reagent that provides the acidic condition include, but is not limited to, hydrogen chloride, a solution of hydrogen chloride in 1,4-dioxane, ammonium chloride, trifluoroacetic acid, formic acid, acetic acid, hydrochloric acid, sulfuric acid, methanesulfonic acid, nitric acid, phosphoric acid, *p*-toluenesulfonic acid and TMSOTf, preferably trifluoroacetic acid;
the above reaction is preferably carried out in a solvent, wherein the solvent used includes, but is not limited to, acetic acid, methanol, ethanol, *n*-butanol, toluene, tetrahydrofuran, dichloromethane, petroleum ether, ethyl acetate, *n*-hexane, dimethyl sulfoxide, 1,4-dioxane, glycol dimethyl ether, water or *N*,*N*-dimethylformamide and a mixture thereof.

A method for preparing the compound of formula (IV) of the present disclosure or a tautomer, mesomer, racemate, enantiomer or diastereomer thereof, or a mixture thereof, or a pharmaceutically acceptable salt thereof, comprises a step of: 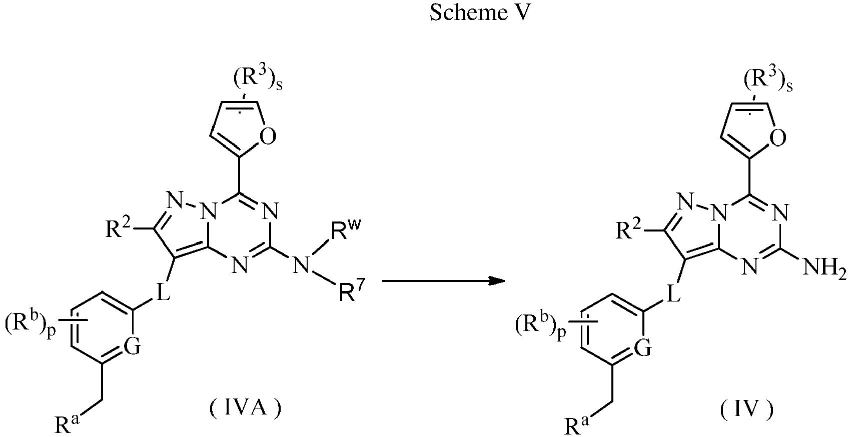 removing the amino protecting group from a compound of formula (IVA) under an acidic condition to obtain the compound of formula (IV), wherein:
R^{w} is an amino protecting group, preferably *tert*-butyl or *tert*-butoxycarbonyl;
R⁷ is hydrogen or R^{w};
G is selected from the group consisting of C, CH and N;
L, R^{a}, R^{b}, R², R³, p and s are as defined in the formula (IV).

The reagent that provides the acidic condition include, but is not limited to, hydrogen chloride, a solution of hydrogen chloride in 1,4-dioxane, ammonium chloride, trifluoroacetic acid, formic acid, acetic acid, hydrochloric acid, sulfuric acid, methanesulfonic acid, nitric acid, phosphoric acid, *p*-toluenesulfonic acid and TMSOTf, preferably trifluoroacetic acid;
the above reaction is preferably carried out in a solvent, wherein the solvent used includes, but is not limited to, acetic acid, methanol, ethanol, *n*-butanol, toluene, tetrahydrofuran, dichloromethane, petroleum ether, ethyl acetate, *n*-hexane, dimethyl sulfoxide, 1,4-dioxane, glycol dimethyl ether, water or *N*,*N*-dimethylformamide and a mixture thereof.

### DETAILED DESCRIPTION OF EXAMPLES

The present disclosure will be further described with reference to the following examples, but the examples should not be considered as limiting the scope of the present disclosure.

### EXAMPLES

The structures of the compounds were identified by nuclear magnetic resonance (NMR) and/or mass spectrometry (MS). NMR shifts (δ) are given in 10⁻⁶ (ppm). NMR spectra were determined by a Bruker AVANCE-400 nuclear magnetic resonance spectrometer. The solvents used in determination were deuterated-dimethyl sulfoxide (DMSO-*d₆*), deuterated-chloroform (CDCl₃) and deuterated-methanol (CD₃OD), and the internal standard was tetramethylsilane (TMS).

MS data were determined by an Agilent 1200/1290 DAD- 6110/6120 Quadrupole MS liquid-mass spectrometer (manufacturer: Agilent, MS Model: 6110/6120 Quadrupole MS), waters ACQuity UPLC-QD/SQD (manufacturer: waters, MS model: waters ACQui can Qda Detector/waters SQ Detector), THERMO Ultimate 3000-Q Exactive (manufacturer: THERMO, MS Model: THERMO Q Exactive).

High performance liquid chromatography (HPLC) was carried out on an Agilent HPLC 1200DAD, Agilent HPLC 1200VWD and Waters HPLC e2695-2489 high pressure liquid chromatographs.

Chiral HPLC was carried out on an Agilent 1260 DAD high performance liquid chromatograph.

High performance liquid preparation was carried out on Waters 2545-2767, Waters 2767-SQ Detecor2, Shimadzu LC-20AP and Gilson GX-281 preparative chromatographs.

Chiral preparation was carried out on a Shimadzu LC-20AP preparative chromatograph.

CombiFlash rapid preparation instrument used was Combiflash Rf200 (TELEDYNE ISCO).

Yantai Huanghai HSGF254 or Qingdao GF254 silica gel plates were used as the thin-layer silica gel chromatography (TLC) plate. The dimension of the silica gel plates used in TLC was 0.15 mm to 0.2 mm, and the dimension of the silica gel plates used in product purification by thin-layer chromatography was 0.4 mm to 0.5 mm.

Yantai Huanghai 200 to 300 mesh silica gel was generally used as a carrier for silica gel column chromatography.

The average kinase inhibition rates and IC₅₀ values were determined by a NovoStar microplate reader (BMG Co., Germany).

The known starting materials of the present disclosure can be prepared by known methods in the art, or can be purchased from ABCR GmbH & Co. KG, Acros Organnics, Aldrich Chemical Company, Accela ChemBio Inc., or Darui Chemical Company, *etc.*

Unless otherwise stated, the reactions were carried out under argon atmosphere or nitrogen atmosphere.

"Argon atmosphere" or "nitrogen atmosphere" means that a reaction flask is equipped with an argon or nitrogen balloon (about 1 L).

"Hydrogen atmosphere" means that a reaction flask is equipped with a hydrogen balloon (about 1 L).

Pressurized hydrogenation reactions were performed on a Parr 3916EKX hydrogenation instrument and a Qinglan QL-500 hydrogen generator or HC2-SS hydrogenation instrument.

In hydrogenation reactions, the reaction system was generally vacuumized and filled with hydrogen, and the above operations were repeated three times.

CEM Discover-S 908860 type microwave reactor was used in microwave reactions.

Unless otherwise stated, the solution refers to an aqueous solution.

Unless otherwise stated, the reaction temperature is room temperature from 20 °C to 30 °C.

The progress of the reaction in the examples was monitored by thin layer chromatography (TLC). The developing solvent used in the reactions, the eluent system in column chromatography and the developing solvent system in thin layer chromatography for purification of the compounds included: A: dichloromethane/methanol system; B: *n*-hexane/ethyl acetate system; C: petroleum ether/ethyl acetate system; D: acetone system; E: dichloromethane/acetone system; F: ethyl acetate/dichloromethane system; G: ethyl acetate/dichloromethane/*n*-hexane system; H: ethyl acetate/dichloromethane/acetone system. The ratio of the volume of the solvents was adjusted according to the polarity of the compounds, and a small quantity of alkaline reagent such as triethylamine or acidic reagent such as acetic acid can also be added for adjustment.

### Example 1

### (S)-4-(5-Methylfuran-2-yl)-8-((6-(((tetrahydrofuran-3-yl)oxy)methyl)pyridin-2-yl)methyl)pyr azolo[1,5-a][1,3,5]triazin-2-amine 1

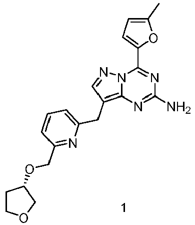

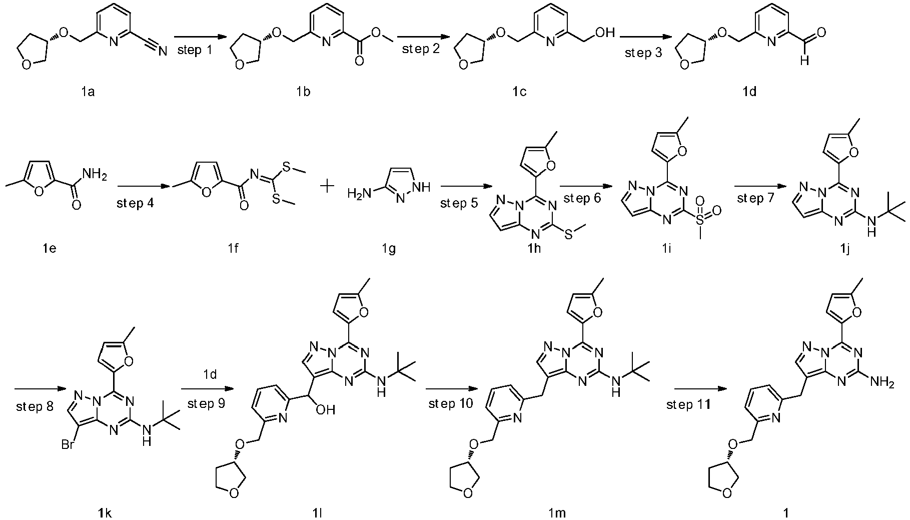

### Step 1

Methyl (*S*)-6-(((tetrahydrofuran-3-yl)oxy)methyl)picolinate **1b** (*S*)-6-(((Tetrahydrofuran-3-yl)oxy)methyl)-2-cyanopyridine **1a** (37.5 g, 183.6 mmol, prepared by the method disclosed in the patent application "WO2009156737") was dissolved in 700 mL of methanol, and cesium carbonate (119.65 g, 367.24 mmol) was added. The resulting mixture was stirred for 16 hours. The reaction solution was poured into 750 mL of 1*N* hydrochloric acid, and stirred for 2 hours. Saturated aqueous sodium bicarbonate solution was added to the reaction solution until the pH > 7. The reaction solution was concentrated under reduced pressure, the resulting aqueous phase was extracted with ethyl acetate (500 mL × 3). The organic phases were combined, dried over anhydrous sodium sulfate and filtered. The filtrate was concentrated under reduced pressure to obtain the crude title compound **1b** (40.2 g), which was directly used in the next reaction without purification.
MS *m*/*z* (ESI): 238.5 [M+1].

### Step 2

### (S)-(6-(((Tetrahydrofuran-3-yl)oxy)methyl)pyridin-2-yl)methanol 1c

The crude compound **1b** (27.5 g, 115.91 mmol) was dissolved in 1 L of absolute ethanol, sodium borohydride (15.56 g, 173.87 mmol) and lithium chloride (10.50 g, 173.87 mmol) were added, the resulting mixture was heated to 50 °C and reacted for 17 hours . After cooling to room temperature, 400 mL of saturated ammonium chloride solution was added to the reaction solution. The reaction solution was concentrated under reduced pressure, 200 mL of water was added to the residue, and extracted with ethyl acetate (300 mL × 4). The organic phases were combined, dried over anhydrous sodium sulfate and filtered. The filtrate was concentrated under reduced pressure. The residue was purified by CombiFlash rapid preparation instrument with eluent system A to obtain the title compound **1c** (22.5 g, yield: 92.77%).
MS *m*/*z* (ESI): 210.4 [M+1].

### Step 3

### (S)-6-(((Tetrahydrofuran-3-yl)oxy)methyl)picolinaldehyde 1d

Compound **1c** (22.5 g, 107.53 mmol) was dissolved in 500 mL of toluene, activated manganese dioxide (46.74 g, 537.66 mmol) was added, the reaction mixture was heated to 95 °C and stirred for 41 hours. The reaction solution was cooled to room temperature and filtered. The filter cake was washed with 1 L of ethyl acetate. The filtrate was concentrated under reduced pressure, and the residue was purified by CombiFlash rapid preparation instrument with eluent system B to obtain the title compound **1d** (15.2 g, yield: 68.21%).
MS *m*/*z* (ESI): 208.4 [M+1].

### Step 4

### Dimethyl (5-methylfuran-2-carbonyl)carbonimidodithioate 1f

5-Methylfuran-2-carboxamide **1e** (19.4 g, 155.04 mmol, prepared by the well-known method *"*Journal of Chemical Research, 2016, 40 (10), 594-596"), carbon disulfide (47.2 g, 620.18 mmol, 37.5 mL) and methyl iodide (70.4 g, 496.14 mmol, 33.5 mL) were dissolved in 600 mL of tetrahydrofuran, and then sodium hydride (12.4 g, 310.09 mmol, 60% purity) was added in portions. The reaction solution was stirred for 30 minutes and refluxed for 4 hours. After cooling to room temperature, the reaction solution was poured into water, extracted with ethyl acetate (300 mL × 3), the organic phases were combined, dried over anhydrous sodium sulfate and filtered. The filtrate was concentrated under reduced pressure, and the residue was purified by column chromatography with eluent system B to obtain the title compound **1f** (17.79 g, yield: 50.04%).
MS *m*/*z* (ESI): 230.1 [M+1].

### Step 5

### 4-(5-Methylfuran-2-yl)-2-(methylthio)pyrazolo[1,5-a][1,3,5]triazine 1h

Under an argon atmosphere, compound **1f** (20.5 g, 89.40 mmol) and 3-aminopyrazole **1g** (7.8 g, 93.87 mmol, Shaoyuan Technology (Shanghai) Co., Ltd.) were dissolved in 180 mL of N-methylpyrrolidone. The reaction solution was heated to 100 °C and reacted for 0.5 hour, and then heated to 185 °C and reacted for 3 hours. After cooling to room temperature, the reaction solution was poured into 2 L of water and filtered. The filter cake was washed with water and dried. The crude product was purified by silica gel column chromatography with eluent system E to obtain the title compound **1h** (14.01 g, yield: 63.63%).
MS *m*/*z* (ESI): 247.0 [M+1].

### Step 6

### 4-(5-Methylfuran-2-yl)-2-(methylsulfonyl)pyrazolo[1,5-a][1,3,5]triazine 1i

Compound **1h** (6.85 g, 27.81 mmol) was dissolved in 300 mL of dichloromethane, *m*-chloroperoxybenzoic acid (9.60 g, 55.63 mmol) was added, and the reaction solution was stirred for 2 hours. The reaction solution was washed successively with saturated aqueous sodium bicarbonate solution (50 mL × 1) and saturated sodium chloride solution (50 mL × 1). The organic phase was dried over anhydrous sodium sulfate, and filtered. The filtrate was concentrated under reduced pressure to obtain the crude title compound **1i** (8.92 g), which was used directly in the next reaction without purification.
MS *m*/*z* (ESI): 279.1 [M+1].

### Step 7

### N-(tert-Butyl)-4-(5-methylfuran-2-yl)pyrazolo[1,5-a][1,3,5]triazin-2-amine 1j

The crude compound **1i** (2.5 g, 6.99 mmol) was dissolved in 30 mL of 1,4-dioxane in a sealed tube, 6 mL of *tert*-butylamine was added. The tube is sealed and heated to 100 °C, and the reaction was allowed to run for 2 hours. The reaction solution was concentrated under reduced pressure, and the residue was purified by CombiFlash rapid preparation instrument with eluent system E to obtain the title compound **1j** (1.9 g, yield: 77.51%).
MS *m*/*z* (ESI): 272.3 [M+1].

### Step 8

### 8-Bromo-N-(tert-butyl)-4-(5-methylfuran-2-yl)pyrazolo[1,5-a][1,3,5]triazin-2-amine 1k

Compound **1j** (3 g, 11.06 mmol) was dissolved in 80 mL of dichloromethane, the resulting mixture was cooled to 0 °C, *N*-bromosuccinimide (2.07 g, 11.61 mmol) was slowly added. The reaction solution was warmed to room temperature and stirred for 1 hour. The reaction solution was concentrated under reduced pressure, and the residue was purified by CombiFlash rapid preparation instrument with eluent system E to obtain the title compound **1k** (3.14 g, yield: 80.96%).
MS *m*/*z* (ESI): 350.1 [M+1].

### Step 9

### (2-(tert-Butylamino)-4-(5-methylfuran-2-yl)pyrazolo[1,5-a][1,3,5]triazin-8-yl)(6-((((S)-tetrah ydrofuran-3-yl)oxy)methyl)pyridin-2-yl)methanol 1l

Compound **1k** (1.89 g, 5.40 mmol) was dissolved in 50 mL of tetrahydrofuran, the resulting mixture was cooled to -78 °C, 7 mL of 1.6 M *n*-butyl lithium was added dropwise. The reaction solution was stirred for 0.5 hour, and compound **1d** (1.45 g, 6.99 mmol) was added in one portion, and then the reaction solution was stirred for 0.5 hour. 30 mL of saturated ammonium chloride aqueous solution was added to the reaction solution and warmed to room temperature. The mixture was separated and the aqueous phase was extracted with ethyl acetate (100 mL × 3). The organic phases were combined, dried over anhydrous sodium sulfate and filtered. The filtrate was concentrated under reduced pressure. The residue was purified by CombiFlash rapid preparation instrument with eluent system C to obtain the title compound **11** (930 mg, yield: 36.01%).
MS *m*/*z* (ESI): 479.6 [M+1].

### Step 10

### (S)-N-(tert-Butyl)-4-(5-methylfuran-2-yl)-8-((6-(((tetrahydrofuran-3-yl)oxy)methyl)pyridin-2-yl)methyl)pyrazolo[1,5-a][1,3,5]triazin-2-amine 1m

Compound **1l** (350 mg, 731 µmol) was dissolved in 10 mL of dichloromethane, trifluoroacetic acid (2.25 g, 19.73 mmol, 1.5 mL) was added, and triethylsilane (2.07 g, 17.8 mmol, 3 mL) was added dropwise. The reaction solution was stirred overnight. Saturated sodium bicarbonate solution was added to the reaction solution until the pH > 7, and the mixture was separated. The aqueous phase was extracted with dichloromethane (25 mL × 3). The organic phases were combined, dried over anhydrous sodium sulfate and filtered. The filtrate was concentrated under reduced pressure. The residue was purified by thin-layer chromatography with developing system B to obtain the title compound **1m** (280 mg, yield: 82.76%).

### Step 11

### (S)-4-(5-Methylfuran-2-yl)-8-((6-(((tetrahydrofuran-3-yl)oxy)methyl)pyridin-2-yl)methyl)pyr azolo[1,5-a][1,3,5]triazin-2-amine 1

Compound **1m** (500 mg, 1.08 mmol) was dissolved in 10 mL of trifluoroacetic acid and heated to 70 °C overnight. After cooling to room temperature, the reaction solution was concentrated under reduced pressure, saturated sodium bicarbonate aqueous solution was added until pH > 7. The mixture was extracted with dichloromethane (30 mL × 3). The organic phases were combined, dried over anhydrous sodium sulfate and filtrated. The filtrate was concentrated under reduced pressure, and the residue was purified by CombiFlash rapid preparation instrument with eluent system A to obtain the title compound **1** (317 mg, yield: 72.15%).
MS *m*/*z* (ESI): 407.5 [M+1]
¹H NMR (400 MHz, CD₃OD): *δ* 8.27 (d, 1H), 7.91 (s, 1H), 7.72 (t, 1H), 7.25 (d, 1H), 7.21 (d, 1H), 6.45-6.46 (m, 1H), 4.61-4.66 (m, 2H), 4.33-4.35 (m, 1H), 4.12 (s, 2H), 3.89-3.93 (m, 2H), 3.80-3.84 (m, 2H), 2.50 (s, 3H), 2.05-2.11 (m, 2H).

### Example 2

### (6-((2-Amino-4-(5-methylfuran-2-yl)pyrazolo[1,5-a][1,3,5]triazin-8-yl)methyl)pyridin-2-yl)m ethanol 2

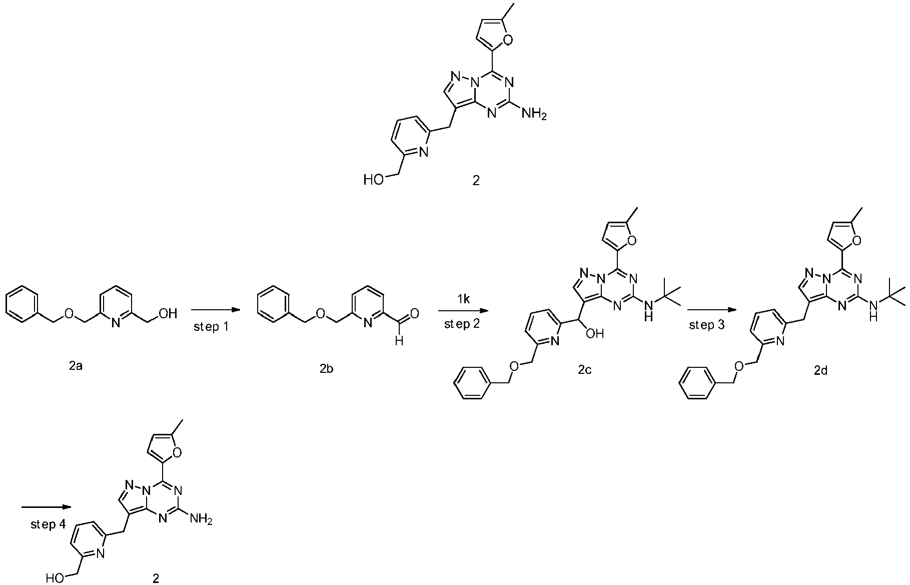

### Step 1

### 6-((Benzyloxy)methyl)picolinaldehyde 2b

(6-((Benzyloxy)methyl)pyridin-2-yl)methanol **2a** (23.400 g, 102.0614 mmol, prepared by a well-known method as *"*Journal of organic chemistry, 1998, Vol. 63 (12), 3884-3894") was dissolved in 600 mL of toluene, and manganese dioxide (44.364 g, 510.3017 mmol) was added. The resulting mixture was refluxed overnight. After cooling to room temperature, the mixture was filtered. The filter cake was washed with ethyl acetate three times. The filtrates were combined and concentrated under reduced pressure. The residue was purified by CombiFlash rapid preparation instrument with eluent system B to obtain the title compound **2b** (15 g, yield: 64.7%).

### Step 2

### (6-((Benzyloxy)methyl)pyridin-2-yl)(2-(tert-butylamino)-4-(5-methylfuran-2-yl)pyrazolo[1,5 -a][1,3,5]triazin-8-yl)methanol 2c

The compound **1k** (5.000 g, 14.2770 mmol) was dissolved in 200 mL of tetrahydrofuran. The mixture was degassed with argon three times and cooled to -78 °C, 18.3 mL of 1.6 M *n*-butyl lithium was added dropwise. The mixture was stirred for 30 minutes after the addition, and then the compound **2b** (3.244 g, 14.2745 mmol) was added and the resulting mixture was stirred for 30 minutes at -78 °C. Saturated ammonium chloride aqueous solution was added, and the resulting mixture was extracted three times with ethyl acetate (100 mL × 3). The organic phases were combined, dried over anhydrous sodium sulfate and concentrated under reduced pressure. The residue was purified by CombiFlash rapid preparation instrument with eluent system B to obtain the title compound **2c** (2.1 g, yield: 29.5%).

### Step 3

### 8-((6-((Benzyloxy)methyl)pyridin-2-yl)methyl)-N-(tert-butyl)-4-(5-methylfuran-2-yl)pyrazol o[1,5-a][1,3,5]triazin-2-amine 2d

Compound **2c** (2.100 g, 4.2120 mmol) was dissolved in 20 mL of dichloromethane, trifluoroacetic acid (4.802 g, 42.1143 mmol, 3.2013 mL) was added, and triethylsilane (4.897 g, 42.1149 mmol, 7.0971 mL) was added dropwise.The reaction solution was stirred for 24 hours. A saturated sodium bicarbonate aqueous solution was added, and the resulting mixture was separated. The aqueous phase was extracted three times with dichloromethane (20 mL × 3). The organic phases were combined, dried over anhydrous sodium sulfate, and concentrated under reduced pressure. The residue was purified by CombiFlash rapid preparation instrument with eluent system B to obtain the title compound **2c** (1.4 g, yield: 68.8%).
MS *m*/*z* (ESI): 483.5 [M+1].

### Step 4

### (6-((2-Amino-4-(5-methylfuran-2-yl)pyrazolo[1,5-a][1,3,5]triazin-8-yl)methyl)pyridin-2-yl)m ethanol 2

Compound **2d** (1.400 g, 2.9011 mmol) was added to 15 mL of trifluoroacetic acid and stirred for 16 hours at 70 °C. The reaction solution was concentrated under reduced pressure and saturated aqueous sodium bicarbonate was added. The aqueous phase was extracted with dichloromethane (30 mL × 2). The organic phases were combined, dried over anhydrous sodium sulfate, and concentrated under reduced pressure. The residue was purified by CombiFlash rapid preparation instrument with eluent system A to obtain the title compound **2** (709 mg, yield: 72.6%).
MS *m*/*z* (ESI): 337.1 [M+1].
¹H NMR (400 MHz, DMSO-*d*₆): *δ* 8.18 (d, 1H), 7.98 (s, 1H), 7.66 (t, 1H), 7.26 (d, 1H), 7.20 (s, 2H), 7.04 (d, 1H), 6.52-6.53 (m, 1H), 5.35 (t, 1H), 4.52 (d, 2H), 3.97 (s, 2H), 2.45 (s, 3H).

### Example 3

### 8-((6-((2-Methoxyethoxy)methyl)pyridin-2-yl)methyl)-4-(5-methylfuran-2-yl)pyrazolo[1,5-a] [1,3,5]triazin-2-amine 3

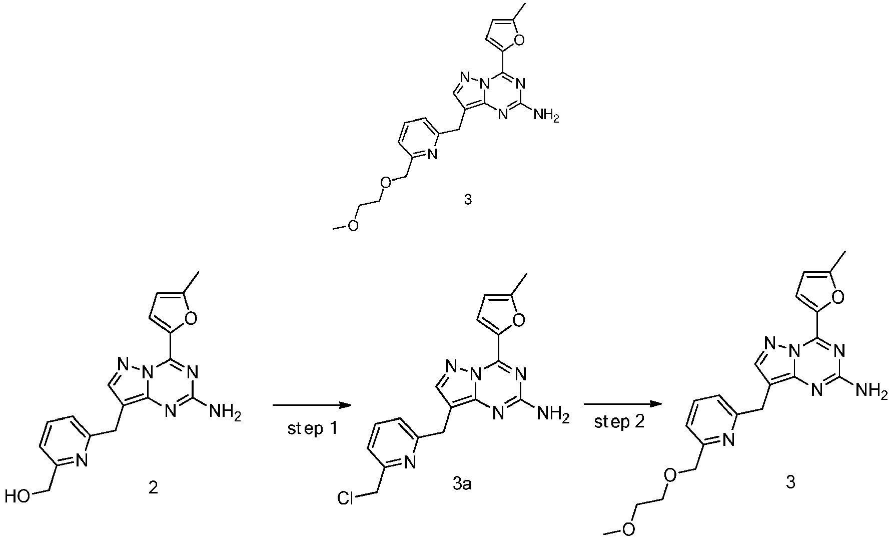

### Step 1

### 8-((6-(Chloromethyl)pyridin-2-yl)methyl)-4-(5-methylfuran-2-yl)pyrazolo[1,5-a][1,3,5]triazi n-2-amine 3a

Compound **2** (709 mg, 2.1079 mmol) was dissolved in 200 mL of dichloromethane, sulfoxide chloride (8.777 g, 73.77 mmol, 5.35 mL) was added, and the reaction solution was stirred for 1 hour. A saturated sodium bicarbonate solution was added to adjust the pH > 7. The resulting mixture was extracted with dichloromethane (100 mL × 3). The organic phases were combined, dried over anhydrous sodium sulfate, and concentrated under reduced pressure. The residue was purified by CombiFlash rapid preparation instrument with eluent system B to obtain the title compound **3a** (550 mg, yield: 73.5%).

### Step 2

### 8-((6-((2-Methoxyethoxy)methyl)pyridin-2-yl)methyl)-4-(5-methylfuran-2-yl)pyrazolo[1,5-a] [1,3,5]triazin-2-amine 3

Ethylene glycol monomethyl ether (19 mg, 250 µmol) was dissolved in 5 mL of tetrahydrofuran, sodium hydride (17 mg, 425 (µmol) was added, and the reaction solution was stirred for 30 minutes at room temperature. Then compound **3a** (50 mg, 141 µmol) was added and the resulting mixture was stirred for 16 hours. Water was added, the resulting mixture was extracted with ethyl acetate (10 mL × 3). The organic phases were combined, dried over anhydrous sodium sulfate, and concentrated under reduced pressure. The residue was purified by silica gel column chromatography with eluent system A to obtain the title compound **3** (10 mg, Yield: 30.0%).
MS *m*/*z* (ESI): 395.5 [M+1].
¹H NMR (400 MHz, CD₃OD) *δ* 8.26 (m, 1H), 7.91 (s, 1H), 7.71-7.73 (t, 1H), 7.37-7.39 (d, 1H), 7.20-7.22 (d, 1H), 6.46 (m, 1H), 4.64 (s, 2H), 4.11 (s, 2H), 3.72-3.73 (m, 2H), 3.62-3.63 (m, 2H), 3.39 (s, 3H), 2.50 (s, 3H).

### Example 4

### 8-(2-Fluorobenzyl)-4-(5-methylfuran-2-yl)pyrazolo[1,5-a][1,3,5]triazin-2-amine 4

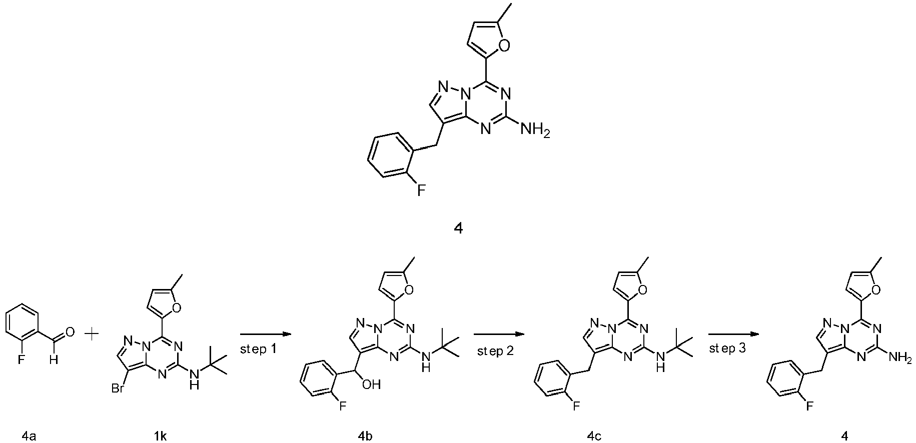

### Step 1

### (2-(tert-Butylamino)-4-(5-methylfuran-2-yl)pyrazolo[1,5-a][1,3,5]triazin-8-yl)(2-fluoropheny l)methanol 4b

Compound **1k** (600 mg, 1.71 mmol) was dissolved in 30 mL of tetrahydrofuran, the resulting mixture was cooled to -78 °C, *n*-butyllithium (1.6 M, 2.2 mL, 3.52 mmol) was added dropwise. The reaction solution was stirred for 0.5 hour. Then 2- fluorobenzaldehyde **4a** (319 mg, 2.57 mmol) was added in one portion and the reaction solution was stirred for 0.5 hour. 20 mL of saturated ammonium chloride aqueous solution was added to the reaction solution. The resulting mixture was warmed to room temperature, separated, and the aqueous phase was extracted with ethyl acetate (50 mL × 3). The organic phases were combined, dried over anhydrous sodium sulfate and filtered. The filtrate was concentrated under reduced pressure. The residue was purified by CombiFlash rapid preparation instrument with eluent system B to obtain the title compound **4b** (344 mg, yield: 50.78%).
MS *m*/*z* (ESI): 396.3 [M+1].

### Step 2

### N-(tert-Butyl)-8-(2-fluorobenzyl)-4-(5-methylfuran-2-yl)pyrazolo[1,5-a][1,3,5]triazin-2-amin e 4c

Compound **4b** (344 mg, 870 µmol) was dissolved in 10 mL of dichloromethane, trifluoroacetic acid (1.05 g, 9.21 mmol, 0.7 mL) was added, and triethylsilane (1.04 g, 8.90 mmol, 1.5 mL) was added dropwise .The reaction solution was stirred for 16 hours. Saturated sodium bicarbonate aqueous solution was added to the reaction solution until the pH > 7. The resulting solution was separated. The aqueous phase was extracted with dichloromethane (20 mL × 3). The organic phases were combined, dried over anhydrous sodium sulfate and filtered. The filtrate was concentrated under reduced pressure. The residue was purified by thin-layer chromatography with developing system B to obtain the title compound **4c** (168 mg, yield: 50.89%).
MS *m*/*z* (ESI): 380.1 [M+1].

### Step 3

### 8-(2-Fluorobenzyl)-4-(5-methylfuran-2-yl)pyrazolo[1,5-a][1,3,5]triazin-2-amine 4

Compound **4c** (168 mg, 447 µmol) was dissolved in 5 mL of trifluoroacetic acid and the resulting mixture was heated to 70 °C for 16 hours. After cooling to room temperature, the reaction solution was concentrated under reduced pressure, and a saturated sodium bicarbonate aqueous solution was added until pH > 7. The resulting mixture was extracted with dichloromethane (20 mL × 3). The organic phases were combined, dried over anhydrous sodium sulfate and filtered. The filtrate was concentrated under reduced pressure. The residue was purified by column chromatography with eluent system B to obtain the title compound **4** (96 mg, yield: 62.87%).
MS *m*/*z* (ESI): 323.9 [M+1].
¹H NMR (400 MHz, CDCl₃): *δ* 8.36 (d, 1H), 7.86 (s, 1H), 7.24-7.27 (m, 1H), 7.17-7.21 (m, 1H), 7.02-7.06 (m, 2H), 6.36-6.37 (d, 1H), 5.42 (s, 2H), 4.01 (s, 2H), 2.54 (s, 3H).

### Example 5

### 4-(5-Methylfuran-2-yl)-8-((6-(morpholinomethyl)pyridin-2-yl)methyl)pyrazolo[1,5-a][1,3,5]t riazin-2-amine 5

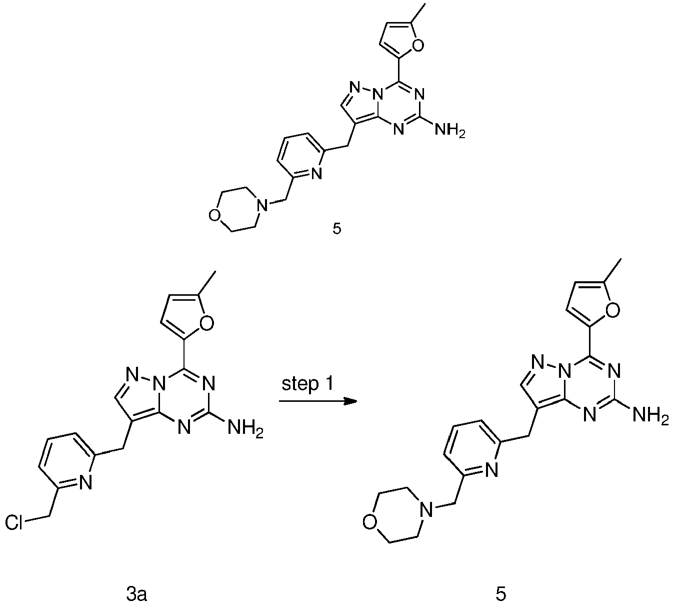

Compound **3a** (50 mg, 140.9 µmol) was dissolved in 30 mL of a mixed solvent of acetonitrile and tetrahydrofuran (V:V = 5:1), morpholine (70 mg, 803.4 µmol) was added. The resulting mixture was heated to 60 °C and reacted for 16 hours. After cooling to room temperature, the reaction solution was concentrated under reduced pressure, and the residue was slurried with methanol to obtain the title compound **5** (54 mg, yield: 94.50%).
MS *m*/*z* (ESI): 406.3 [M+1].
¹H NMR (400 MHz, DMSO-*d*₆): *δ* 8.20 (d, 1H), 7.98 (s, 1H), 7.64 (t, 1H), 7.25 (d, 1H), 7.21 (s, 2H), 7.06 (d, 1H), 6.53-6.55 (m, 1H), 3.98 (s, 2H), 3.57-3.60 (m, 4H), 3.56 (s, 2H), 2.43 (s, 3H), 2.40-2.42 (m, 4H).

### Example 6

### (R)-4-(5-Methylfuran-2-yl)-8-((6-(((tetrahydrofuran-3-yl)oxy)methyl)pyridin-2-yl)methyl)pyr azolo[1,5-a][1,3,5]triazin-2-amine 6

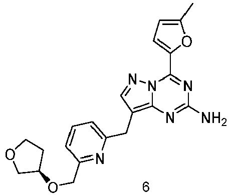

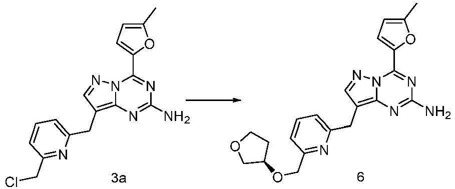

(3*R*)-tetrahydrofuran-3-ol (37 mg, 420 µmol) was dissolved in 5 mL of tetrahydrofuran, sodium hydride (16 mg, 696 µmol) was added. The resulting mixture was stirred for 30 minutes at room temperature, and then compound **3a** (50 mg, 141 µmol) was added, the resulting mixture was stirred for 16 hours. Water was added, the resulting mixture was extracted with ethyl acetate (10 mL × 3). The organic phases were combined, dried over anhydrous sodium sulfate, and concentrated under reduced pressure. The residue was purified by silica gel column chromatography with developing system A to obtain the title compound **6** (10 mg, Yield: 17.4%).
MS *m*/*z* (ESI): 407.5 [M+1].
¹H NMR (400 MHz, DMSO-*d*₆) *δ* 8.20 (m, 1H), 7.99 (s, 1H), 7.66-7.70 (t, 1H), 7.22-7.24 (brs, 3H), 7.10-7.12 (d, 1H), 6.54 (m, 1H), 4.52 (s, 2H), 4.26 (m, 1H), 3.99 (s, 2H), 3.75-3.77 (m, 2H), 3.66-3.70 (m, 2H), 2.46 (s, 3H), 1.95-1.99 (m, 2H).

### Example 7

### 8-((6-((2-Oxa-6-azaspiro[3.3]heptan-6-yl)methyl)pyridin-2-yl)methyl)-4-(5-methylfuran-2-yl) pyrazolo[1,5-a][1,3,5]triazin-2-amine 7

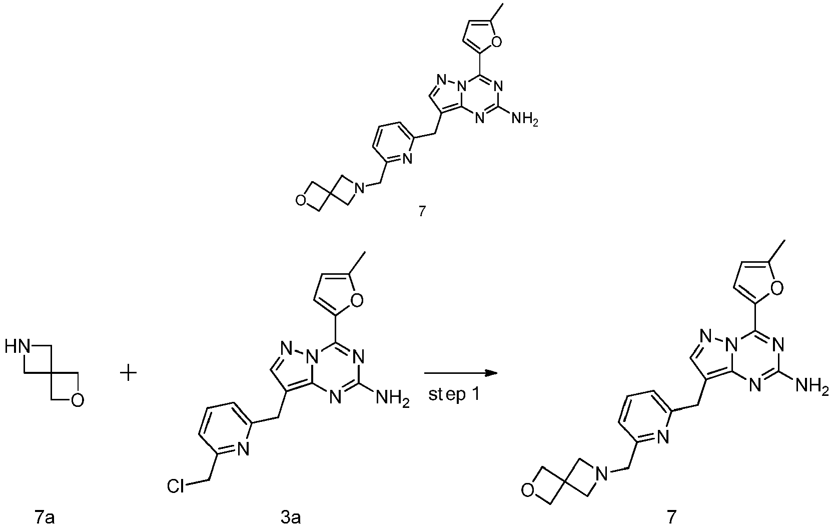

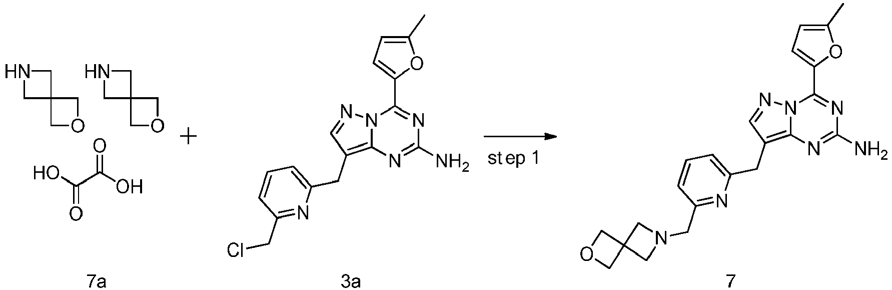

Sodium bicarbonate (360 mg, 4.28 mmol) and 2-oxa-6-azaspiro[3.3]heptane hemioxalate **7a** (190 mg, 1.32 mmol, prepared by the well-known method disclosed in *"*Angewandte Chemie-International Edition, 2008, 47 (24), 4512-4515") was added to 60 mL of acetonitrile, and the resulting mixture was stirred for 30 minutes. Compound **3a** (105 mg, 295.9 µmol) and 8 mL of tetrahydrofuran were added, and the reaction solution was heated to 60 °C and reacted for 16 hours. After cooling to room temperature, the reaction solution was concentrated under reduced pressure, and the residue was purified by thin-layer chromatography with developing system A to obtain the title compound **7** (75 mg, yield: 60.70%).
MS *m*/*z* (ESI): 418.5 [M+1].
¹H NMR (400 MHz, CD₃OD): *δ* 8.27 (d, 1H), 7.91 (s, 1H), 7.68 (t, 1H), 7.17-7.21 (m, 2H), 6.45-6.46 (m, 1H), 4.74 (s, 4H), 4.10 (s, 2H), 3.72 (s, 2H), 3.52 (s, 4H), 2.50 (s, 3H).

### Example 8

### 4-(5-Methylfuran-2-yl)-8-((6-((4-methylpiperazin-1-yl)methyl)pyridin-2-yl)methyl)pyrazolo[ 1,5-a][1,3,5]triazin-2-amine 8

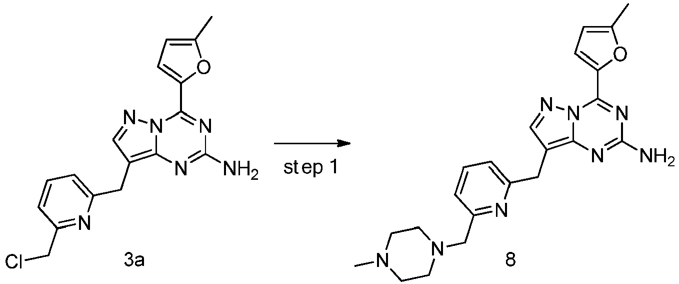

Compound **3a** (50 mg, 140.9 µmol) and *N*-methylpiperazine (70 mg, 698.9 µmol) were dissolved in 30 mL of a mixed solvent of acetonitrile and tetrahydrofuran (V:V = 5:1). The resulting mixture was heated to 60 °C and reacted for 16 hours. After cooling to room temperature, the reaction solution was concentrated under reduced pressure, and the residue was purified by thin-layer chromatography with developing system A to obtain the title compound **8** (47 mg, yield: 79.69%).
MS *m*/*z* (ESI): 419.5 [M+1].
¹H NMR (400 MHz, CD₃OD): *δ* 8.27 (d, 1H), 7.91 (s, 1H), 7.69 (t, 1H), 7.32 (d, 1H), 7.20 (s, 1H), 6.46-6.47 (m, 1H), 4.12 (s, 2H), 3.69 (s, 2H), 2.50-2.57 (m, 11H), 2.30 (s, 3H).

### Example 9

### 4-(5-Methylfuran-2-yl)-8-((6-((tetrahydro-1H-furo[3,4-c]pyrrol-5(3H)-yl)methyl)pyridin-2-yl )methyl)pyrazolo[1,5-a][1,3,5]triazin-2-amine 9

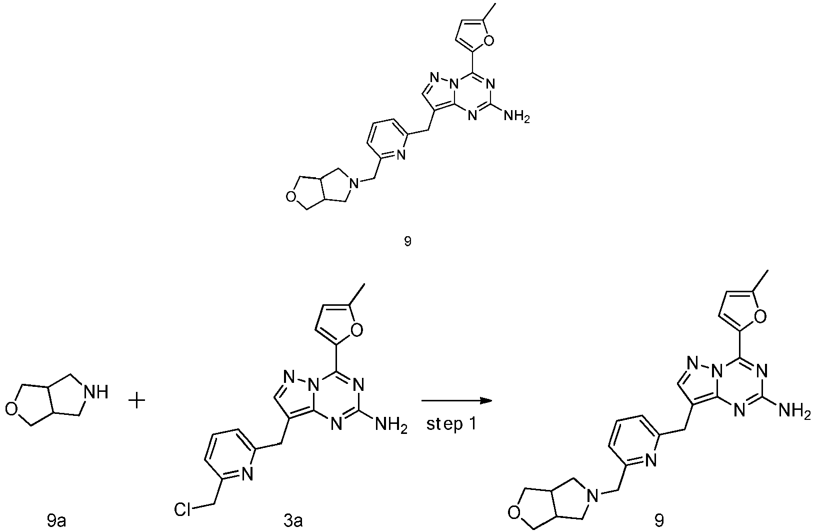

Compound **3a** (105 mg, 295.9 µmol) was dissolved in 55 mL of a mixed solvent of acetonitrile and tetrahydrofuran (V:V = 10:1), and hexahydro-1*H*-furo[3,4-c]pyrrole **9a** (90 mg, 795.3 µmol, prepared by the method disclosed in the patent application "WO2013071697") and *N*,*N*-diisopropylethylamine (365 mg, 2.82 mmol, 0.5 mL) were added. The resulting mixture was heated to 60 °C and reacted for 16 hours. After cooling to room temperature, the reaction solution was concentrated under reduced pressure, and the residue was purified by thin-layer chromatography with the developing system A to obtain the title compound **9** (50 mg, yield: 39.15%).
MS *m*/*z* (ESI): 432.5 [M+1]
¹H NMR (400 MHz, DMSO-*d*₆): *δ* 8.20 (d, 1H), 7.98 (s, 1H), 7.64 (t, 1H), 7.22-7.24 (m, 3H), 7.05 (d, 1H), 6.53-6.54 (m, 1H), 3.98 (s, 2H), 3.71-3.75 (m, 2H), 3.64 (s, 2H), 3.39-3.41 (m, 2H), 2.70-2.72 (m, 2H), 2.56-2.58 (m, 2H), 2.46 (s, 3H), 2.37-2.39 (m, 2H).

### Example 10

### 8-(2-Fluorobenzyl)-4-(furan-2-yl)pyrazolo[1,5-a][1,3,5]triazin-2-amine 10

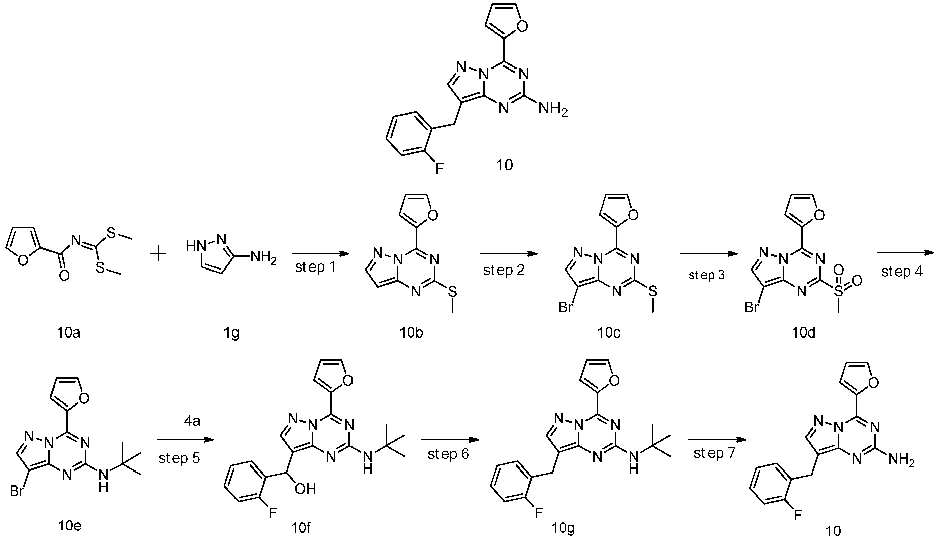

### Step 1

### 4-(Furan-2-yl)-2-(methylthio)pyrazolo[1,5-a][1,3,5]triazine 10b

Under argon atmosphere, dimethyl (furan-2-carbonyl)carbonimidodithioate **10a** (4.7 g, 21.83 mmol, prepared by the well-known method disclosed in *"*Synthesis, 1981, 7, 554-557") and 3-aminopyrazole (1.82 g, 21.90 mmol) were dissolved in 50 mL of *N*-methylpyrrolidone. The resulting mixture was heated to 100 °C for 30 minutes, and then heated to 185 °C for 3 hours. After cooling to room temperature, the reaction solution was poured into 500 mL of ethyl acetate, and the organic phase was washed successively with water (50 mL × 4) and saturated sodium chloride solution (50 mL × 1), dried over anhydrous sodium sulfate and filtered. The filtrate was concentrated under reduced pressure. The residue was purified by silica gel column chromatography with eluent system E to obtain the title compound **10b** (2.26 g, yield: 44.57%).

### Step 2

### 8-Bromo-4-(furan-2-yl)-2-(methylthio)pyrazolo[1,5-a][1,3,5]triazine 10c

Compound **10b** (1.78 g, 7.67 mmol) was dissolved in 100 mL of dichloromethane, *N*-bromosuccinimide (1.52 g, 8.54 mmol) was added, and the reaction solution was stirred for 2 hours. The reaction solution was concentrated under reduced pressure, and the residue was purified by silica gel column chromatography with eluent system E to obtain the title compound **10c** (2.68 g, yield: 112.2%).

### Step 3

### 8-Bromo-4-(furan-2-yl)-2-(methylsulfonyl)pyrazolo[1,5-a][1,3,5]triazine 10d

Compound **10c** (2.86 g, 9.19 mmol) was dissolved in 150 mL of dichloromethane, *m*-chloroperoxybenzoic acid (3.15 g, 18.25 mmol) was added, and the reaction solution was stirred for 2 hours. After adding 50 mL of saturated aqueous sodium bicarbonate solution, the layers were separated, and the organic phase was washed with water (30 mL), dried over anhydrous sodium sulfate, filtered, and the filtrate was concentrated under reduced pressure to obtain the crude title compound **10d** (4.04 g), which was used in the next step without purification.

### Step 4

### 8-Bromo-N-(tert-butyl)-4-(furan-2-yl)pyrazolo[1,5-a][1,3,5]triazin-2-amine 10e

In a sealed tube, the crude compound **10d** (4.04 g, 11.77 mmol) was added to 70 mL of dioxane, 10 mL of *tert*-butylamine was added. The reaction was performed at 100 °C for 3 hours after sealing. After cooling to room temperature, the reaction solution was concentrated under reduced pressure, and the residue was purified by a CombiFlash rapid preparation instrument with eluent system E to obtain the title compound **10e** (2.34 g, yield: 59.12%).

### Step5

### (2-(tert-Butylamino)-4-(furan-2-yl)pyrazolo[1,5-a][1,3,5]triazin-8-yl)(2-fluorophenyl)methan ol 10f

Compound **10e** (800 mg, 2.38 mmol) was dissolved in 40 mL of tetrahydrofuran, after the resulting mixture was cooled to -78 °C, n-butyllithium (1.6 M, 2 mL) was added dropwise, and the reaction solution was stirred for 30 minutes. Compound **4a** (443 mg, 3.57 mmol) was added and the reaction solution was continuously stirred for 30 minutes. 30 mL of saturated ammonium chloride aqueous solution was added and the reaction solution was warmed to room temperature. The resulting mixture was separated, and the aqueous phase was extracted with ethyl acetate (50 mL × 3). The organic phases were combined, dried over anhydrous sodium sulfate and filtered. The filtrate was concentrated under reduced pressure. The residue was purified by a CombiFlash rapid preparation instrument with eluent system B to obtain the title compound **10f** (168 mg, yield: 18.51%).

### Step 6

### N-(tert-Butyl)-8-(2-fluorobenzyl)-4-(furan-2-yl)pyrazolo[1,5-a][1,3,5]triazin-2-amine 10g

Compound **10f** (168 mg, 440 µmol) was dissolved in 10 mL of dichloromethane, trifluoroacetic acid (1.5 g, 13.15 mmol, 1 mL) was added, and triethylsilane (1.38 g, 11.86 mmol, 2 mL) was added dropwise. The reaction solution was stirred for 16 hours. 20 mL of saturated sodium bicarbonate aqueous solution was added, and the resulting solution was separated. The aqueous phase was extracted with dichloromethane (20 mL × 3). The organic phases were combined, dried over anhydrous sodium sulfate and filtered. The filtrate was concentrated under reduced pressure. The residue was purified by a CombiFlash rapid preparation instrument with eluent system B to obtain the title compound **10g** (168 mg, yield: 18.51%).

### Step 7

### 8-(2-Fluorobenzyl)-4-(furan-2-yl)pyrazolo[1,5-a][1,3,5]triazin-2-amine 10

Compound **10g** (90 mg, 246 µmol) was dissolved in 5 mL of trifluoroacetic acid. The resulting mixture was heated to 70 °C for 16 hours. The reaction solution was concentrated under reduced pressure and saturated sodium bicarbonate aqueous solution was added until pH > 7. The resulting mixture was extracted with dichloromethane (30 mL × 3). The organic phases were combined, dried over anhydrous sodium sulfate and filtered. The filtrate was concentrated under reduced pressure. The residue was purified by thin layer chromatography with eluent system B to obtain the title compound **10** (54 mg, yield: 70.88%).
MS *m*/*z* (ESI): 310.3 [M+1].
¹H NMR (400 MHz, CD₃OD): *δ* 8.34 (d, 1H), 7.97 (s, 1H), 7.84 (s, 1H), 7.21-7.26 (m, 2H), 7.05-7.07 (m, 2H), 6.79-6.81 (m, 1H), 3.96 (s, 2H).

### Example 11

### (6-((2-Amino-4-(5-methylfuran-2-yl)pyrazolo[1,5-a][1,3,5]triazin-8-yl)methyl)pyridin-2-yl)m ethyl sulfamate 11

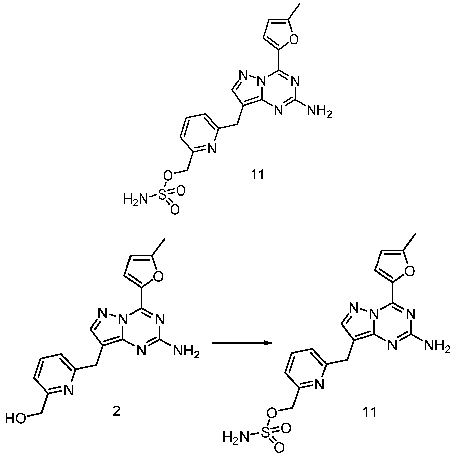

Compound **2** (60 mg, 178 µmol) was dissolved in 3 mL of *N*,*N*-dimethylacetamide, 1 mL of pyridine was added, and then 1 mL of a solution of sulfamoyl chloride (41 mg, 354 µmol) in dichloromethane was added. The resulting mixture was stirred for 2 hours at room temperature. Saturated ammonium chloride solution was added, and the resulting mixture was extracted with ethyl acetate (10 mL × 3). The organic phases were combined, dried over anhydrous sodium sulfate, and concentrated under reduced pressure. The residue was purified by silica gel column chromatography with developing system A to obtain the title compound **11** (10 mg, yield: 13.5%).
MS *m*/*z* (ESI): 415.9 [M+1].
1H NMR (400 MHz, DMSO-*d*₆) *δ* 8.19-8.20 (m, 1H), 8.01 (s, 1H), 7.73-7.75 (m, 1H), 7.70 (brs, 2H), 7.30-7.32 (m, 1H), 7.18-7.24 (m, 3H), 6.55 (m, 1H), 5.10 (s, 2H), 4.02 (s, 2H), 2.46 (s, 3H).

### Example 12

### (S)-4-(5-Methylfuran-2-yl)-8-(3-(((tetrahydrofuran-3-yl)oxy)methyl)phenoxy)pyrazolo[1,5-a] [1,3,5]triazin-2-amine 12

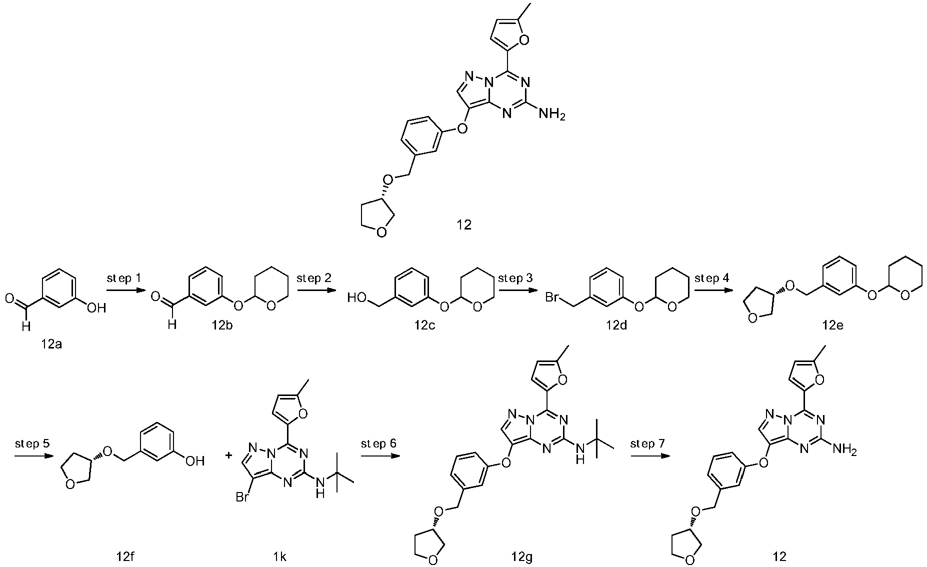

### Step 1

### 3-((Tetrahydro-2H-pyran-2-yl)oxy)benzaldehyde 12b

3-Hydroxybenzaldehyde **12a** (10.5 g, 85.98 mmol, prepared by the well-known method disclosed in *"*Synthetic Communications, 2008, 38 (15), 2638-2645"), and pyridinium 4-methylbenzenesulfonate (648 mg , 2.58 mmol) were dissolved in 100 mL of dichloromethane, 3,4-dihydro-2*H*-pyran (21.70 g, 257.94 mmol) was added, and the reaction solution was stirred for 48 hours. After the reaction was terminated, 200 mL of dichloromethane was added, and the resulting mixture was washed with water (100 mL × 2). The organic phase was concentrated under reduced pressure, and the residue was purified by silica gel chromatography with eluent system B to obtain the title compound **12b** (17.7 g, yield: 99.8%).

### Step 2

### (3-((Tetrahydro-2H-pyran-2-yl)oxy)phenyl)methanol 12c

Compound **12b** (8.0 g, 38.79 mmol) was dissolved in 100 mL of methanol, sodium borohydride (1.69 g, 44.61 mmol) was added at 0 °C, and the reaction solution was stirred for 20 minutes. After the reaction was terminated, 100 mL of water was added. The resulting mixture was concentrated under reduced pressure and extracted with ethyl acetate (100 mL × 2). The organic phases were combined and concentrated under reduced pressure. The residue was purified by silica gel chromatography with eluent system B to obtain the title compound **12c** (6.51 g, yield: 80.5%).
MS *m*/*z* (ESI): 209.4 [M+1].

### Step 3

### 2-(3-(Bromomethyl)phenoxy)tetrahydro-2H-pyran 12d

Compound **12c** (1.0 g, 4.80 mmol), carbon tetrabromide (1.75 g, 5.28 mmol), triphenylphosphine (1.40 g, 5.33 mmol) and *N*,*N*-diisopropylethylamine (670 mg, 5.19 mmol) were dissolved in 40 mL of dichloromethane. The resulting mixture was stirred for 1 hour. After the reaction was terminated, 30 mL of water was added. The resulting mixture was extracted with dichloromethane (30 mL × 3). The organic phases were combined and concentrated under reduced pressure. The residue was purified by silica gel chromatography with eluent system B to obtain the title compound **12d** (957 mg, Yield: 73.5%).

### Step 4

### 2-(3-((((S)-Tetrahydrofuran-3-yl)oxy)methyl)phenoxy)tetrahydro-2H-pyran 12e

*S*-3-Hydroxytetrahydrofuran (373 mg, 4.24 mmol, prepared by the method disclosed in the published patent of "WO2005/121111") was dissolved in 20 mL of tetrahydrofuran, sodium hydride (110 mg, 4.59 mmol) was added, and the resulting mixture was stirred for 40 minutes. A solution of compound **12d** (957 mg, 3.53 mmol) in 1 mL of tetrahydrofuran was added, and the resulting mixture was stirred at 0 °C for 17 hours. After the reaction was terminated, 50 mL of water was added, and the resulting mixture was extracted with ethyl acetate (50 mL × 3). The organic phases were combined and concentrated under reduced pressure. The residue was purified by silica gel chromatography with eluent system B to obtain the title compound **12e** (788 mg, Yield: 80.2%).
MS *m*/*z* (ESI): 279.5 [M+1].

### Step 5

### (S)-3-(((Tetrahydrofuran-3-yl)oxy)methyl)phenol 12f

Compound **12e** (788 mg, 2.83 mmol) was dissolved in 20 mL of methanol, pyridinium 4-methylbenzenesulfonate (22 mg, 0.084 mmol) was added, and the reaction solution was stirred for 65 hours. After the reaction was terminated, the reaction solution was concentrated under reduced pressure, and the residue was purified by silica gel chromatography with eluent system B to obtain the title compound **12f** (473 mg, yield: 86.0%).
MS *m*/*z* (ESI): 195.4 [M+1].

### Step 6

### (S)-N-(tert-Butyl)-4-(5-methylfuran-2-yl)-8-(3-(((tetrahydrofuran-3-yl)oxy)methyl)phenoxy)p yrazolo[1,5-a][1,3,5]triazin-2-amine 12g

Compound **12f** (266 mg, 1.37 mmol), compound **1k** (240 mg, 0.685 mmol) and cesium carbonate (447 mg, 1.37 mmol) were dissolved in 5 mL of *N*,*N*-dimethylformamide. The resulting mixture was stirred for 65 hours at 95 °C. After the reaction was terminated, 30 mL of water was added, the resulting mixture was extracted with ethyl acetate (30 mL × 3). The organic phases were combined and concentrated under reduced pressure. The residue was purified by silica gel chromatography with eluent system B to obtain the title compound **12g** (51 mg, Yield: 16.1%).
MS *m*/*z* (ESI): 464.8 [M+1].

### Step 7

### (S)-4-(5-Methylfuran-2-yl)-8-(3-(((tetrahydrofuran-3-yl)oxy)methyl)phenoxy)pyrazolo[1,5-a] [1,3,5]triazin-2-amine 12

Compound **12 g** (51 mg, 0.110 mmol) was dissolved in 2 mL of trifluoroacetic acid, the resulting mixture was refluxed and stirred for 2 hours. After the reaction was terminated, the reaction solution was concentrated under reduced pressure, then 50 mL of ethyl acetate was added, and the resulting mixture was washed with saturated sodium bicarbonate solution (30 mL× 3). The organic phases were combined and concentrated under reduced pressure. The residue was purified by thin-layer chromatography with developing system B to obtain the title compound **12** (21.2 mg, yield: 47.3%).
MS *m*/*z* (ESI): 408.5 [M+1].
¹H NMR (400 MHz, DMSO-*d*₆) *δ* 7.91-7.90 (m, 1H), 7.44-7.40 (m, 1H), 7.27-7.19 (m, 5H), 6.49-6.48 (m, 1H), 5.47 (s, 1H), 4.51-4.50 (m, 2H), 4.20 (s, 1H), 3.76-3.63 (m, 4H), 2.43 (s, 3H), 1.96-1.91 (m, 2H).

### Example 13

### 8-((6-(Methoxymethyl)pyridin-2-yl)methyl)-4-(5-methylfuran-2-yl)pyrazolo[1,5-a][1,3,5]tria zin-2-amine 13

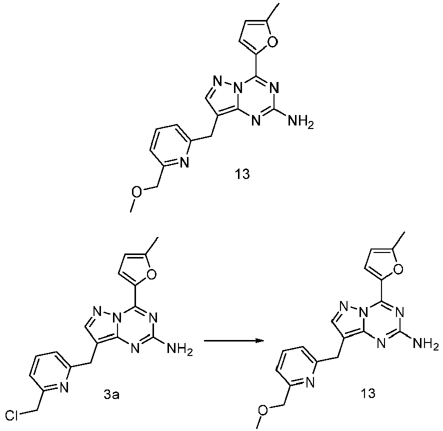

Compound **3a** (30 mg, 84 µmol) was dissolved in 5 mL of methanol, sodium methoxide (9 mg, 166 µmol) was added, and the reaction solution was stirred at 70 °C for 16 hours. Then water was added, and the resulting mixture was extracted with ethyl acetate (20 mL × 3). The organic phases were combined, dried over anhydrous sodium sulfate, and concentrated under reduced pressure. The residue was purified by silica gel column chromatography with developing system A to obtain the title compound **13** (5 mg, Yield: 16.9%).
MS *m*/*z* (ESI): 351.4 [M+1].
¹H NMR (400 MHz, CD₃OD) *δ* 8.28 (m, 1H), 7.91 (s, 1H), 7.71-7.73 (t, 1H), 7.32-7.34 (d, 1H), 7.20-7.22 (d, 1H), 6.46-6.47 (m, 1H), 4.56 (s, 2H), 4.12 (s, 2H), 3.47 (s, 3H), 2.50 (s, 3H).

### Example 14

### 8-((6-((2-Fluoroethoxy)methyl)pyridin-2-yl)methyl)-4-(5-methylfuran-2-yl)pyrazolo[1,5-a][1, 3,5]triazin-2-amine 14

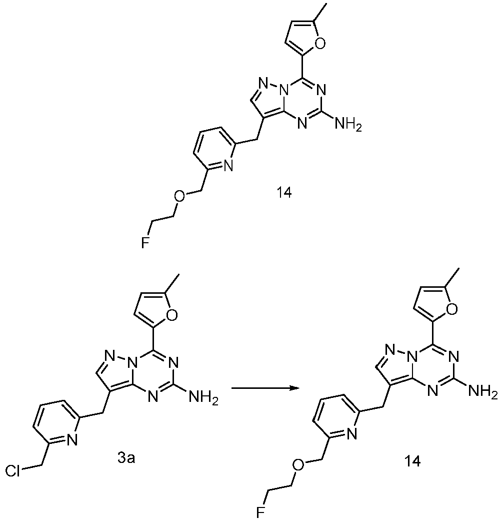

2-Fluoroethanol (27 mg, 421 µmol) was dissolved in 5 mL of tetrahydrofuran, sodium hydride (33 mg, 825 µmol) was added. The resulting mixture was stirred for 30 minutes at room temperature, then compound **3a** (30 mg, 84 µmol) was added and the reaction solution was stirred for 16 hours. Water was added, the resulting mixture was extracted with ethyl acetate (10 mL × 3). The organic phases were combined, dried over anhydrous sodium sulfate, and concentrated under reduced pressure. The residue was purified by silica gel column chromatography with developing system A to obtain the title compound **14** (19 mg, Yield: 49.7%).
MS *m*/*z* (ESI): 383.4 [M+1].
¹H NMR (400 MHz, CD₃OD) *δ* 8.28 (m, 1H), 7.91 (s, 1H), 7.71-7.73 (t, 1H), 7.38-7.39 (d, 1H), 7.21-7.23 (d, 1H), 6.5-6.47 (m, 1H), 4.68 (s, 2H), 4.65-4.67 (m, 1H), 4.53-4.55 (m, 1H), 4.12 (s, 2H), 3.85-3.87 (m, 1H), 3.785-3.80 (m, 1H), 2.50 (s, 3H).

### Example 15

### (S)-4-(5-Methylfuran-2-yl)-8-((6-(((1-methylpyrrolidin-3-yl)oxy)methyl)pyridin-2-yl)methyl) pyrazolo[1,5-a][1,3,5]triazin-2-amine 15

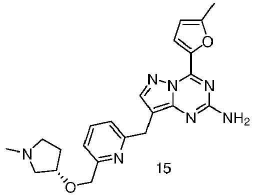

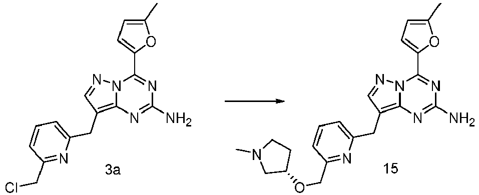

(3*S*)-3-hydroxy-1-methylpyrrolidine (42 mg, 415 µmol) was dissolved in 5 mL of tetrahydrofuran, sodium hydride (16 mg, 695 µmol) was added, the reaction solution was stirred for 30 minutes, and then compound **3a** (50 mg, 140 µmol) was added, and the reaction solution was stirred for 16 hours. Water was added, the resulting mixture was extracted with ethyl acetate (10 mL × 3). The organic phases were combined, dried over anhydrous sodium sulfate, and concentrated under reduced pressure. The residue was purified by silica gel column chromatography with developing system A to obtain the title compound **15** (5 mg, Yield: 8.4%).
MS *m*/*z* (ESI): 420.5 [M+1].
¹H NMR (400 MHz, CD₃OD) *δ* 8.26 (m, 1H), 7.90 (s, 1H), 7.70-7.73 (t, 1H), 7.35-7.37 (d, 1H), 7.20-7.22 (d, 1H), 6.45 (m, 1H), 4.58 (s, 2H), 4.25 (brs, 1H), 4.10 (s, 2H), 2.75-2.85 (m, 3H), 2.50-2.56 (m, 1H), 2.49 (s, 3H), 2.41 (s, 3H), 2.17-2.23(m, 1H), 1.96-1.99 (m, 1H).

### Example 16

### (S)-8-(imino(6-(((tetrahydrofuran-3-yl)oxy)methyl)pyridin-2-yl)methyl)-4-(5-methylfuran-2-y l)pyrazolo[1,5-a][1,3,5]triazin-2-amine 16

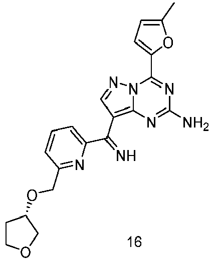

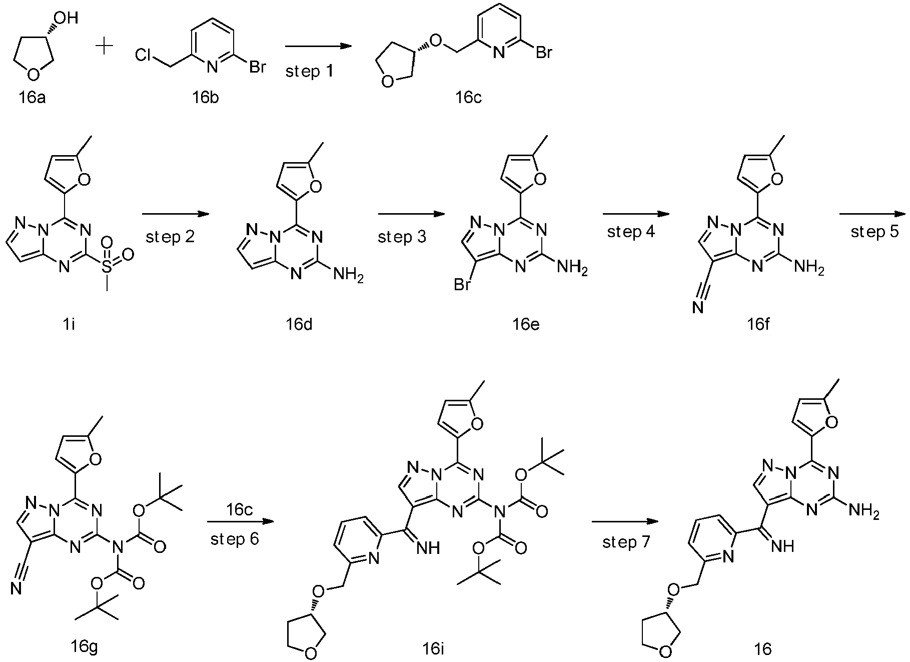

### Step 1

### (S)-2-Bromo-6-(((tetrahydrofuran-3-yl)oxy)methyl)pyridine 16c

(3*S*)-Tetrahydrofuran-3-ol **16a** (5.06 g, 57.43 mmol, 4.6 mL) was dissolved in 200 mL of tetrahydrofuran, sodium hydride (2.63 g, 65.88 mmol, 60% purity) was added, and the reaction solution was stirred for 0.5 hour. Then 2-bromo-6-(chloromethyl)pyridine **16b** (10.88 g, 52.70 mmol, prepared by the well-known method disclosed in *"*Journal of Medicinal Chemistry, 2010, 53 (23), 8421-8439") was added and the resulting mixture was heated to 70 °C for 16 hours. The reaction solution was cooled to room temperature, 100 mL of saturated sodium chloride solution was added, the resulting mixture was separated. The aqueous phase was extracted with ethyl acetate (100 mL × 3). The organic phases were combined, dried over anhydrous sodium sulfate and filtered. The filtrate was concentrated under reduced pressure. The residue was purified by CombiFlash rapid preparation instrument with eluent system B to obtain the title compound **16c** (12.2 g, yield: 89.69%).
MS *m*/*z* (ESI): 257.9 [M+1].

### Step 2

### 4-(5-Methylfuran-2-yl)pyrazolo[1,5-a][1,3,5]triazin-2-amine 16d

In a sealed tube, compound **1i** (3.77 g, 13.54 mmol) was added to 50 mL of dioxane, and then 10 mL of ammonia water was added, the resulting mixture was heated to 100 °C and reacted for 2 hours after sealing. The reaction solution was cooled to room temperature, and concentrated under reduced pressure to obtain the crude title compound **16d** (3.28 g), which was used in the next step without purification.
MS *m*/*z* (ESI): 216.2 [M+1].

### Step 3

### 8-Bromo-4-(5-methylfuran-2-yl)pyrazolo[1,5-a][1,3,5]triazin-2-amine 16e

The crude compound **16d** (1.8 g, 8.36 mmol) was dissolved in 250 mL of dichloromethane, *N*-bromosuccinimide (1.65 g, 9.27 mmol) was added, and the reaction solution was stirred for 1 hour. The reaction solution was concentrated under reduced pressure, and the residue was purified by silica gel column chromatography with eluent system D to obtain the title compound **16e** (2.25 g, yield: 91.46%).
MS *m*/*z* (ESI): 294.0 [M+1].

### Step 4

### 2-Amino-4-(5-methylfuran-2-yl)pyrazolo[1,5-a][1,3,5]triazine-8-carbonitrile 16f

Under an argon atmosphere, compound **16e** (1 g, 3.4 mmol), cuprous cyanide (1.52 g, 16.97 mmol), and cuprous iodide (120 mg, 630 µmol) were dissolved in 30 mL of dimethyl sulfoxide. The resulting mixture was heated to 165 °C and reacted for 5 hours. The reaction solution was cooled to room temperature. 250 mL of ethyl acetate was added and the resulting mixture was filtered. The filter cake was washed with 50 mL of ethyl acetate. The filtrates were combined, washed successively with water (30 mL × 4) and saturated sodium chloride solution (30 mL × 1), dried over anhydrous sodium sulfate, and filtered. The filtrate was concentrated under reduced pressure to obtain the crude title compound **16f** (600 mg), which was used in the next step without purification.
MS *m*/*z* (ESI): 241.1 [M+1].

### Step 5

### tert-Butyl N-tert-butoxycarbonyl-N-[8-cyano-4-(5-methyl-2-furyl)pyrazolo[1,5-a][1,3,5]triazin-2-yl]car bamate 16g

The crude compound **16f** (600 mg, 2.50 mmol) was dissolved in 50 mL of tetrahydrofuran, di-*tert*-butyl dicarbonate (2.72 g, 12.46 mmol) and 4-dimethylaminopyridine (3 mg, 24.3 µmol) were added, and the reaction solution was stirred overnight. The reaction solution was concentrated under reduced pressure, and the residue was purified by CombiFlash rapid preparation instrument using eluent system B to obtain the title compound **16g** (220 mg, yield: 20.0%).

### Step 6

### tert-Butyl N-tert-butoxycarbonyl-N-[4-(5-methyl-2-furyl)-8-[6-[[(3S)-tetrahydrofuran-3-yl]oxymethyl]p yridine-2-carboximidoyl]pyrazolo[1,5-a][1,3,5]triazin-2-yl]carbamate 16i

Compound **16c** (176 mg, 681.9 µmol) was dissolved in 30 mL of tetrahydrofuran, after the resulting mixture was cooled to -78 °C, *n*-butyllithium (1.6 M, 0.42 mL) was added dropwise, and the resulting mixture was stirred for 0.5 hour. A solution of compound **16g** (200 mg, 454.1 µmol) in 10 mL of tetrahydrofuran was added and the resulting mixture was stirred for 1 hour. 10 mL of saturated ammonium chloride aqueous solution was added, the resulting mixture was warmed to room temperature and separated. The aqueous phase was extracted with ethyl acetate (30 mL × 3). The organic phases were combined, dried over anhydrous sodium sulfate and filtered. The filtrate was concentrated under reduced pressure. The residue was purified by CombiFlash rapid preparation instrument with eluent system B to obtain the title compound **16i** (53 mg, yield: 18.83%).

### Step 7

### (S)-8-(Imino(6-(((tetrahydrofuran-3-yl)oxy)methyl)pyridin-2-yl)methyl)-4-(5-methylfuran-2-yl)pyrazolo[1,5-a][1,3,5]triazin-2-amine 16

Compound **16i** (53 mg, 85.5 µmol) was dissolved in 2 mL of dichloromethane, 1 mL of trifluoroacetic acid was added, and the reaction solution was stirred for 1 hour. The reaction solution was concentrated under reduced pressure. Saturated sodium bicarbonate aqueous solution was added until the pH > 7. The resulting mixture was extracted with dichloromethane (20 mL × 3). The organic phases were combined, dried over anhydrous sodium sulfate and filtered. The filtrate was concentrated under reduced pressure. The residue was purified by thin-layer chromatography with developing system A to obtain the title compound **16** (20 mg, yield: 55.75%).
MS *m*/*z* (ESI): 420.2 [M+1].
¹H NMR (400 MHz, DMSO-*d*₆): *δ* 8.55 (d, 1H), 7.87 (t, 1H), 7.76 (s, 1H), 7.49 (d, 1H), 6.99 (d, 1H), 6.71 (brs, 2H), 6.08-6.09 (m, 1H), 6.01-6.02 (m, 1H), 4.57-4.61 (m, 2H), 4.27-4.29 (m, 1H), 3.74-3.80 (m, 2H), 3.67-3.70 (m, 2H), 2.39 (s, 3H), 1.94-1.99 (m, 2H).

### Example 17

### 8-((6-((Cyclopropylmethoxy)methyl)pyridin-2-yl)methyl)-4-(5-methylfuran-2-yl)pyrazolo[1, 5-a][1,3,5]triazin-2-amine 17

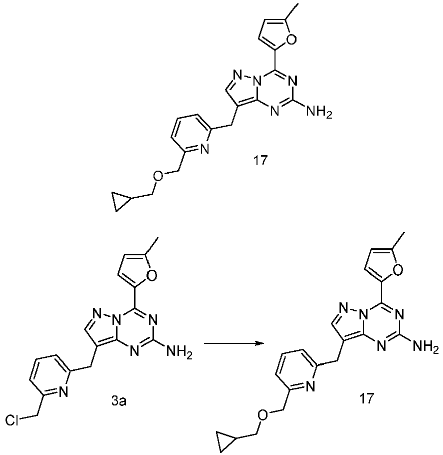

Cyclopropylmethanol (30 mg, 422 µmol) was dissolved in 5 mL of tetrahydrofuran, sodium hydride (33 mg, 1.41 mmol) was added, and the reaction solution was stirred for 30 minutes at room temperature. Then compound **3a** (50 mg, 140 µmol) was added, and the reaction solution was stirred for 16 hours at room temperature. Water was added, and the resulting mixture was extracted with ethyl acetate (10 mL × 3). The organic phases were combined, dried over anhydrous sodium sulfate, and concentrated under reduced pressure. The residue was purified by silica gel column chromatography with developing system A to obtain the title compound **17** (8 mg, Yield: 14.5%).
MS *m*/*z* (ESI): 391.1 [M+1].
¹H NMR (400 MHz, CD₃OD) *δ* 8.23-8.24 (m, 1H), 7.88 (s, 1H), 7.68-7.72 (t, 1H), 7.33-7.35 (d, 1H), 7.18-7.20 (d, 1H), 6.44 (m, 1H), 4.61 (s, 2H), 4.09 (s, 2H), 3.40-3.42 (d, 2H), 2.48 (s, 3H), 1.10-1.12 (m, 1H), 0.52-0.55 (m, 2H), 0.22-0.24 (m, 2H).

### Example 18

### (S)-4-(5-Methylfuran-2-yl)-N⁸-(6-(((tetrahydrofuran-3-yl)oxy)methyl)pyridin-2-yl)pyrazolo[1 ,5-a][1,3,5]triazine-2,8-diamine 18

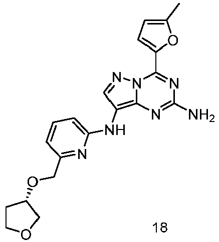

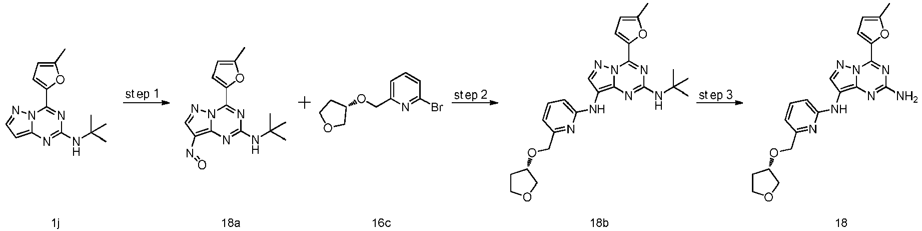

### Step 1

### N-(tert-Butyl)-4-(5-methylfuran-2-yl)-8-nitrosopyrazolo[1,5-a][1,3,5]triazin-2-amine 18a

Compound **1j** (11.03 g, 40.65 mmol) was dissolved in 250 mL of ethanol and cooled to 0 °C, a solution of hydrogen chloride in dioxane (4 M, 30 mL) was added, and isoamyl nitrite (4.78 g, 40.80 mmol, 5.5 mL) was added dropwise. After completion of the addition, the resulting mixture was warmed to room temperature and stired for 2 hours. The reaction solution was poured into 2 L of saturated sodium bicarbonate aqueous solution. The resulting mixture was stirred for 5 minutes and filtered. The filter cake was washed with water, and dissolved in 500 mL of dichloromethane. The resulting solution was dried over anhydrous sodium sulfate and filtered. The filtrate was concentrated under reduced pressure, the crude product was slurried in 100 mL of a mixed solvent of dichloromethane and *n*-hexane (V:V = 1:10) and filtered. The filter cake was dried to obtain the title compound **18a** (10.7 g, yield: 87.64%).
MS *m*/*z* (ESI): 300.9 [M+1].

### Step 2

### (S)-N²-(tert-Butyl)-4-(5-methylfuran-2-yl)-N⁸-(6-(((tetrahydrofuran-3-yl)oxy)methyl)pyridin-2-yl)pyrazolo[1,5-a][1,3,5]triazine-2,8-diamine 18b

Under an argon atmosphere, compound **16c** (13 g, 50.36 mmol) was dissolved in 150 mL of tetrahydrofuran. After cooling to -78 °C, *n*-butyllithium (1.6 M, 30 mL) was added dropwise, and the resulting mixture was stirred for 30 minutes. The above reaction solution was added in one portion to a solution of compound **18a** (6.75 g, 22.47 mmol) in 100 mL of tetrahydrofuran which had been pre-cooled to -78 °C, and the reaction solution was stirred for 1 hour. 50 mL of saturated ammonium chloride aqueous solution was added. The resulting mixture was warmed to room temperature and separated. The aqueous phase was extracted with ethyl acetate (150 mL × 3). The organic phases were combined, dried over anhydrous sodium sulfate and filtered. The filtrate was concentrated under reduced pressure. The residue was purified by CombiFlash rapid preparation instrument with eluent system B to obtain the title compound **18b** (1.6 g, yield: 15.35%).
MS *m*/*z* (ESI): 463.9 [M+1].

### Step 3

### (S)-4-(5-Methylfuran-2-yl)-N⁸-(6-(((tetrahydrofuran-3-yl)oxy)methyl)pyridin-2-yl)pyrazolo[1 ,5-a][1,3,5]triazine-2,8-diamine 18

Compound **18b** (2 g, 4.31 mmol) was dissolved in 30 mL of trifluoroacetic acid and heated to 70 °C for 16 hours. After cooling to room temperature, the reaction solution was concentrated under reduced pressure. A saturated sodium bicarbonate aqueous solution was added to the residue until pH > 7, the resulting mixture was extracted with dichloromethane (100 mL × 3). The organic phases were combined, dried over anhydrous sodium sulfate and filtrated. The filtrate was concentrated under reduced pressure, and the residue was purified by CombiFlash rapid preparator with eluent system A to obtain the title compound **18** (1.2 g, yield: 68.26%).
MS *m*/*z* (ESI): 408.4 [M+1].
¹H NMR (400 MHz, DMSO-*d*₆): *δ* 8.29 (s, 1H), 8.23 (d, 1H), 8.05 (s, 1H), 7.41 (t, 1H), 7.18 (s, 2H), 6.63 (d, 1H), 6.56 (d, 1H), 6.44 (d, 1H), 4.36-4.39 (m, 2H), 4.23-4.26 (m, 1H), 3.72-3.79 (m, 2H), 3.66-3.69 (m, 2H), 2.47 (s, 3H), 1.76-1.99 (m, 2H).

### Example 19

### (S)-4-(5-Methylfuran-2-yl)-8-((6-(((tetrahydrofuran-3-yl)oxy)methyl)pyridin-2-yl)oxy)pyrazo lo[1,5-a][1,3,5]triazin-2-amine 19

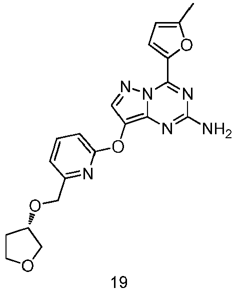

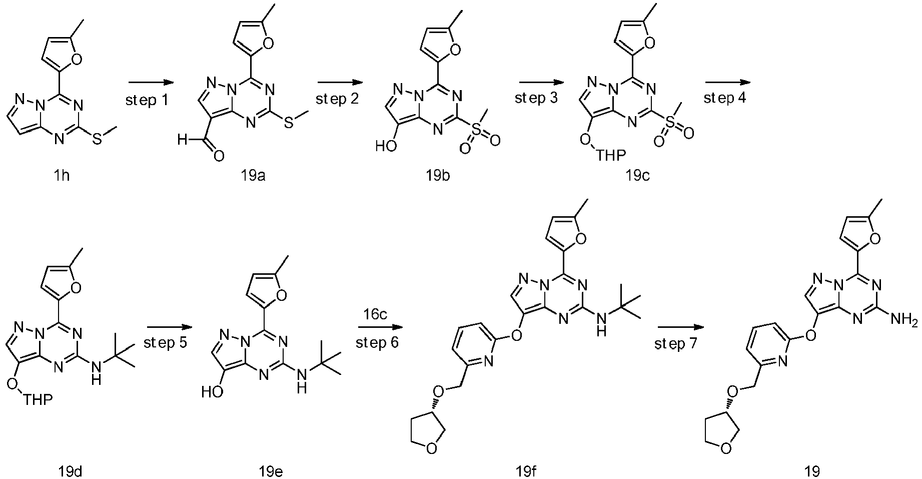

### Step 1

### 4-(5-Methylfuran-2-yl)-2-(methylthio)pyrazolo[1,5-a][1,3,5]triazine-8-carbaldehyde 19a

Compound **1h** (4 g, 16.24 mmol) was dissolved in 50 mL of *N*,*N*-dimethylformamide, phosphorus oxychloride (3.79 g, 24.75 mmol) was added, and the reaction solution was heated to 80 °C and reacted for 1 hour. After cooling to room temperature, the reaction solution was poured into 500 mL of saturated sodium bicarbonate aqueous solution, stirred for 15 minutes and filtered. The filter cake was washed successively with water, a small amount methanol, and ether, and dried in vacuo to obtain the title compound **19a** (4.3 g, yield: 96.52%).

### Step 2

### 4-(5-Methylfuran-2-yl)-2-(methylsulfonyl)pyrazolo[1,5-a][1,3,5]triazin-8-ol 19b

Compound **19a** (4.3 g, 15.67 mmol) was dissolved in 100 mL of chloroform, *m*-chloroperoxybenzoic acid (10.8 g, 62.58 mmol) was added, and the reaction solution was heated to 65 °C and reacted for 1 hour. The reaction solution was cooled to room temperature, washed with saturated sodium bicarbonate aqueous solution (100 mL × 2), water (50 mL × 1), saturated sodium chloride solution (50 mL × 1), dried over anhydrous sodium sulfate, and filtered. The filtrate was concentrated under reduced pressure, and the residue was purified by a CombiFlash rapid preparation instrument with eluent system C to obtain the title compound **19b** (650 mg, yield: 14.08%).

### Step 3

### 4-(5-Methylfuran-2-yl)-2-(methylsulfonyl)-8-((tetrahydro-2H-pyran-2-yl)oxy)pyrazolo[1,5-a] [1,3,5]triazine 19c

Compound **19b** (650 mg, 2.21 mmol) was dissolved in 50 mL of dichloromethane, 3,4-dihydro-2*H*-pyran (6.45 g, 76.72 mmol) and pyridine tosylate (55 mg, 218.8 µmol) were added. The reaction solution was stirred for 1 hour. 2 mL of tert-butylamine was added, and the reaction solution was concentrated under reduced pressure to obtain the crude title compound **19c** (1 g), which was directly used in the next step without purification.

### Step 4

### N-(tert-Butyl)-4-(5-methylfuran-2-yl)-8-((tetrahydro-2H-pyran-2-yl)oxy)pyrazolo[1,5-a][1,3, 5]triazin-2-amine 19d

The crude compound **19c** (1.0 g, 2.64 mmol) was dissolved in 30 mL of 1,4-dioxane, and 6 mL of *tert*-butylamine was added. The reaction was allowed to run for 1.5 hours in a sealed tube at 100 °C. The reaction solution was cooled to room temperature, and concentrated under reduced pressure. The residue was purified by CombiFlash rapid preparation instrument with eluent system B to obtain the title compound **19d** (420 mg, yield: 42.78%).

### Step 5

### 2-(tert-butylamino)-4-(5-methylfuran-2-yl)pyrazolo[1,5-a][1,3,5]triazin-8-ol 19e

Compound **19d** (420 mg, 1.13 mmol) was dissolved in 30 mL of methanol, pyridinium *p*-toluenesulfonate (28 mg, 111.4 µmol) was added, and the reaction solution was stirred for 16 hours. The reaction solution was concentrated under reduced pressure, and the residue was purified by a CombiFlash rapid preparation instrument with eluent system B to obtain the title compound **19e** (140 mg, 43.09%).

### Step 6

### (S)-N-(tert-Butyl)-4-(5-methylfuran-2-yl)-8-((6-(((tetrahydrofuran-3-yl)oxy)methyl)pyridin-2-yl)oxy)pyrazolo[1,5-a][1,3,5]triazin-2-amine 19f

Under an argon atmosphere, compound **19e** (50 mg, 0.174 mmol), compound **16c** (54 mg, 0.209 mmol), tridibenzylideneacetone dipalladium (8 mg, 0.009 mmol), 2-(dicyclohexylphosphine)-3,6-dimethoxy-2'-4'-6'-tri-*i*-propyl-11'-biphenyl (10 mg, 0.019 mmol) and cesium carbonate (114 mg, 0.350 mmol) were dissolved in 4 mL of toluene. After the temperature warmed to 95 °C, the reaction solution was stirred for 3 hours. The reaction solution was concentrated under reduced pressure, and the residue was purified by silica gel column chromatography with eluent system B to obtain the title compound **19f** (54 mg, yield: 66.80%).
MS *m*/*z* (ESI): 465.2 [M+1].

### Step 7

### (S)-4-(5-Methylfuran-2-yl)-8-((6-(((tetrahydrofuran-3-yl)oxy)methyl)pyridin-2-yl)oxy)pyrazo lo[1,5-a][1,3,5]triazin-2-amine 19

Compound **19f** (77 mg, 0.166 mmol) was dissolved in 4 mL of trifluoroacetic acid. The resulting mixture was refluxed, and stirred for 2 hours. After cooling to room temperature, the reaction solution was concentrated under reduced pressure. A saturated sodium bicarbonate aqueous solution was added until pH > 7. The resulting mixture was extracted with ethyl acetate (30 mL × 3). The organic phases were combined, dried over anhydrous sodium sulfate and filtrated. The filtrate was concentrated under reduced pressure, and the residue was purified by silica gel column chromatography with eluent system B to obtain the title compound **19** (53.4 mg, yield: 78.88%).
MS *m*/*z* (ESI): 409.2 [M+1].
¹H NMR (400 MHz, DMSO-*d*₆): *δ* 8.23-8.22 (m, 1H), 8.20 (s, 1H), 7.82-7.78 (m, 1H), 7.31 (*brs,* 2H), 7.12-7.10 (m, 1H), 6.91-6.89 (m, 1H), 6.58-6.57 (m, 1H), 4.37 (s, 2H), 4.21-4.20 (m, 1H), 3.74-3.60 (m, 4H), 2.47 (s, 3H), 1.92-1.88 (m, 2H).

### Example 20

### 4-(5-Methylfuran-2-yl)-8-((6-propylpyridin-2-yl)methyl)pyrazolo[1,5-a][1,3,5]triazin-2-amin e 20

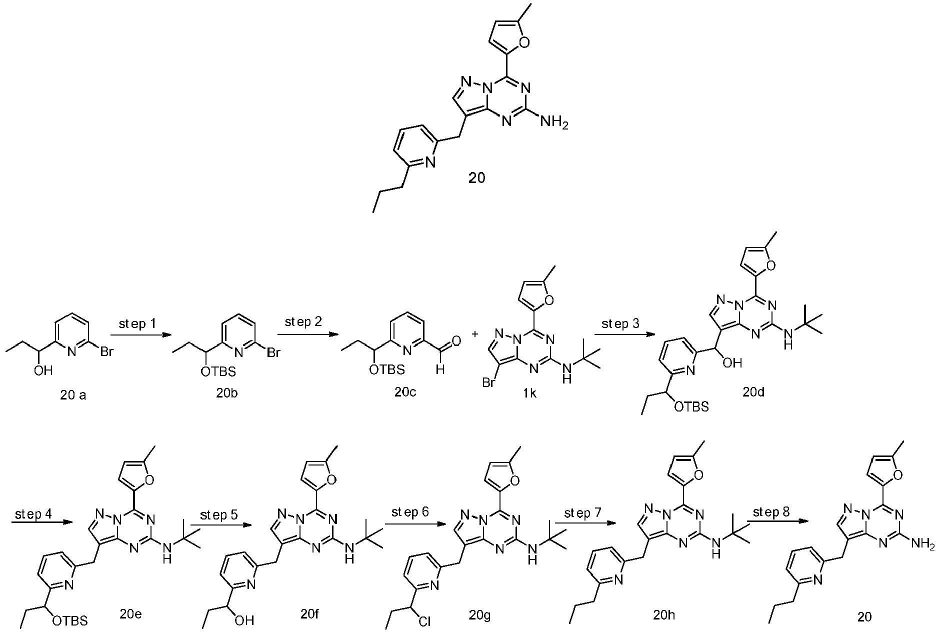

### Step 1

### 2-Bromo-6-(1-((tert-butyldimethylsilyl)oxy)propyl)pyridine20b

1-(6-Bromopyridin-2-yl)propan--ol **20a** (760 mg, 3.52 mmol, prepared by the well-known method disclosed in *"*Tetrahedron Letters, 2014, 55 (41), 5591-5594") was dissolved in 20 mL of dichloromethane, *tert*-butyldimethylchlorosilane (795 mg, 5.27 mmol) and imidazole (359 mg, 5.27 mmol) were added, and the reaction solution was stired for 16 hours. The reaction solution was concentrated under reduced pressure, and the residue was purified by CombiFlash rapid preparator with eluent system B to obtain the title compound **20b** (830 mg, yield: 71.44%).

### Step 2

### 6-(1-((tert-Butyldimethylsilyl)oxy)propyl)picolinaldehyde 20c

Compound **20b** (830 mg, 2.51 mmol) was dissolved in 10 mL of tetrahydrofuran. After cooling to -78 °C, *n*-butyl lithium 1.6 M (1.6 M, 1.73 mL) was added dropwise, the reaction solution was stirred for 1 hour, and then *N*,*N*-dimethylformamide (367 mg, 5.02 mmol, 387.13µL), the reaction solution was continued to stir for 1 hour. Saturated ammonium chloride aqueous solution was added, the resulting mixture was extracted with ethyl acetate (20 mL × 3), the organic phases were combined, dried over anhydrous sodium sulfate and filtered. The filtrate was concentrated under reduced pressure, and the residue was purified by CombiFlash rapid preparation instrument with eluent system B to obtain the title compound **20c** (480 mg, yield: 68.36%).
MS *m*/*z* (ESI): 280.2 [M+1].

### Step 3

### (2-(tert-Butylamino)-4-(5-methylfuran-2-yl)pyrazolo[1,5-a][1,3,5]triazin-8-yl)(6-(1-((tert-but yldimethylsilyl)oxy)propyl)pyridin-2-yl)methanol 20d

Compound **1k** (600 mg, 1.71 mmol) was dissolved in 10 mL of tetrahydrofuran, after cooling to -78 °C, *n*-butyllithium (1.6 M, 2.20 mL) was added dropwise. The resulting mixture was continued to stir for 30 minutes after the addition. Compound **20c** (480 mg, 1.72 mmol) was added, and the reaction solution was stirred for 30 minutes at -78 °C. Saturated ammonium chloride aqueous solution was added and the resulting mixture was extracted with ethyl acetate (20 mL × 3). The organic phases were combined, dried over anhydrous sodium sulfate and filtered. The filtrate was concentrated under reduced pressure. The residue was purified by CombiFlash rapid preparation instrument with eluent system B to obtain the title compound **20d** (300 mg, yield: 31.79%).
MS *m*/*z* (ESI): 551.3 [M+1].

### Step 4

### N-(tert-Butyl)-8-((6-(1-((tert-butyldimethylsilyl)oxy)propyl)pyridin-2-yl)methyl)-4-(5-methyl furan-2-yl)pyrazolo[1,5-a][1,3,5]triazin-2-amine 20e

Compound **20d** (300 mg, 544.70 µmol) was dissolved in dichloromethane, trifluoroacetic acid (621 mg, 5.45 mmol, 414.00 µL) was added, and triethylsilane (633 mg, 5.44 mmol, 917.39 µL) was added dropwise. The reaction solution was stirred for 24 hours. A saturated sodium bicarbonate aqueous solution was added, and the resulting mixture was separated. The aqueous phase was extracted with dichloromethane (20 mL × 3). The organic phases were combined, dried over anhydrous sodium sulfate and filtered. The filtrate was concentrated under reduced pressure. The residue was purified by CombiFlash rapid preparation instrument with eluent system B to obtain the title compound **20e** (110 mg, yield: 37.76%).
MS *m*/*z* (ESI): 535.3 [M+1].

### Step 5

### 1-(6-((2-(tert-Butylamino)-4-(5-methylfuran-2-yl)pyrazolo[1,5-a][1,3,5]triazin-8-yl)methyl)p yridin-2-yl)propan-1-ol 20f

Compound **20e** (110 mg, 205.70 µmol) was added to 10 mL of tetrahydrofuran, followed by *n*-tetrabutylammonium fluoride (69 mg, 306.43 µmol) was added, and the reaction solution was stirred for 2 hours. Water was added and the resulting mixture was extracted with ethyl acetate (20 mL × 3). The organic phases were combined, dried over anhydrous sodium sulfate and filtered. The filtrate was concentrated under reduced pressure to obtain the crude title compound **20f** (72 mg), which was used directly in next step without purification.
MS *m*/*z* (ESI): 421.3 [M+1].

### Step 6

### N-(tert-Butyl)-8-((6-(l-chloropropyl)pyridin-2-yl)methyl)-4-(5-methylfuran-2-yl)pyrazolo[1, 5-a][1,3,5]triazin-2-amine 20g

The crude compound **20f** (170 mg, 404.27 µmol) was dissolved in 20 mL of dichloromethane, and then thionyl chloride (96 mg, 806.93 µmol) was added, and the reaction solution was stirred for 2 hours. The reaction solution was poured into ice water, and extracted with dichloromethane (20 mL × 3). The organic phases were combined, dried over anhydrous sodium sulfate and filtered. The filtrate was concentrated under reduced pressure to obtain the crude title compound **20g** (170 mg), which was used directly in next step without purification.
MS *m*/*z* (ESI): 439.2 [M+1].

### Step 7

### N-(tert-Butyl)-4-(5-methylfuran-2-yl)-8-((6-propylpyridin-2-yl)methyl)pyrazolo[1,5-a][1,3,5] triazin-2-amine 20h

Compound **20g** (170 mg, 387.29 µmol) was dissolved in 20 mL of methanol, and then a palladium-carbon hydrogenation catalyst (wet) (82 mg, 38.53 µmol, 5% purity) was added. The reaction system was purged with hydrogen three times, stirred for 1 hour and filtered. The filtrate was concentrated under reduced pressure, and the residue was purified by silica gel column chromatography with the developing system A to obtain the title compound **20h** (72 mg, yield: 45.96%).
MS *m*/*z* (ESI): 405.2 [M+1].

### Step 8

### 4-(5-Methylfuran-2-yl)-8-((6-propylpyridin-2-yl)methyl)pyrazolo[1,5-a][1,3,5]triazin-2-amin e 20

Compound **20h** (72 mg, 177.99 µmol) was dissolved in 5 mL of trifluoroacetic acid and stirred for 16 hours at 70 °C. After cooling, the resulting mixture was concentrated under reduced pressure. Water was added to the residue, and then saturated sodium bicarbonate solution was added to adjust pH > 7. The resulting mixture was extracted with ethyl acetate (20 mL × 3). The organic phases were combined, dried over anhydrous sodium sulfate and filtered. The filtrate was concentrated under reduced pressure. The residue was purified by silica gel column chromatography with the developing system A to obtain the title compound **20** (41 mg, yield: 66.11%).
MS *m*/*z* (ESI): 349.2 [M+1].
¹H NMR (400 MHz, DMSO-*d*₆): *δ* 8.19-8.20 (m, 1H), 7.98 (s, 1H), 7.54-7.56 (m, 1H), 7.22 (br, 2H), 7.04-7.05 (d, 1H), 6.98-7.00 (d, 1H), 6.53-6.54 (m, 1H), 3.97 (s, 2H), 2.65-2.69 (t, 2H), 2.46 (s, 3H), 1.65-1.71 (m, 2H), 0.89-0.93 (t, 3H).

### Example 21

### (R)-4-(5-Methylfuran-2-yl)-N⁸-(6-(((tetrahydrofuran-3-yl)oxy)methyl)pyridin-2-yl)pyrazolo[1 ,5-a][1,3,5]triazine-2,8-diamine 21

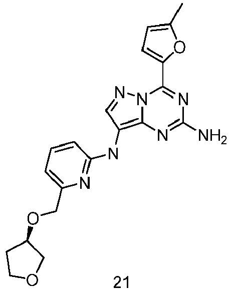

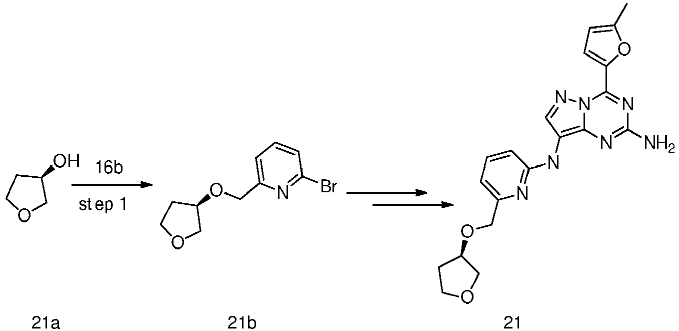

### Step 1

### (S)-2-Bromo-6-(((tetrahydrofuran-3-yl)oxy)methyl)pyridine 21b

Using the synthetic route of compound **16c** in Example 16, the starting material compound **16a** in the step 1 was replaced with the compound (3*R*)-tetrahydrofuran-3-ol **21a** to obtain the title compound **21b** (3.87 g).

Using the synthetic route of Example 18, the starting material compound **16c** in the step 2 was replaced with the compound **21b** to obtain the title compound **21** (125 mg).
MS *m*/*z* (ESI): 408.3 [M+1].
¹H NMR (400 MHz, DMSO-*d*₆) *δ* 8.29 (s, 1H), 8.23 (d, 1H), 8.05 (s, 1H), 7.41 (t, 1H), 7.18 (s, 2H), 6.63 (d, 1H), 6.57 (d, 1H), 6.44 (d, 1H), 4.36 (s, 2H), 4.24-4.25 (m, 1H), 3.73-3.79 (m, 2H), 3.66-3.71 (m, 2H), 2.47 (s, 3H), 1.93-1.99 (m, 2H).

### Example 22

### 8-((6-((Cyclopentyloxy)methyl)pyridin-2-yl)methyl)-4-(5-methylfuran-2-yl)pyrazolo[1,5-a][1 ,3,5]triazin-2-amine 22

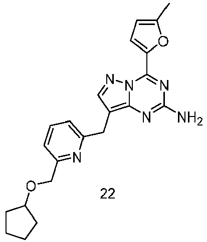

Using the synthetic route of Example **17,** the raw material cyclopropyl methanol was replaced with cyclopentanol to obtain the target product **22** (5 mg).
MS *m*/*z* (ESI): 405.0 [M+1].
¹H NMR (400 MHz, CD₃OD): *δ* 8.25-8.26 (m, 1H), 7.90 (s, 1H), 7.71-7.75 (m, 1H), 7.35 (d, 1H), 7.21 (d, 1H), 6.44-6.45 (m, 1H), 4.57 (s, 2H), 4.09-4.11 (m, 3H), 2.49 (s, 3H), 1.71-1.78 (m, 6H), 1.56-1.58 (m, 2H).

### Example 23

### 4-(5-Methylfuran-2-yl)-8-(pyridin-2-yloxy)pyrazolo[1,5-a][1,3,5]triazin-2-amine 23

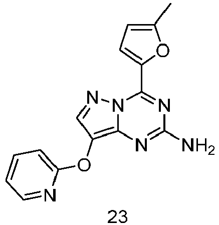

Using the synthetic route of Example **19,** the raw material **16c** in the step 6 was replaced with 2-bromopyridine to obtain the target product **23** (5 mg).
MS *m*/*z* (ESI): 309.1 [M+1].
¹H NMR (400 MHz, CD₃OD): *δ* 8.32-8.33 (m, 1H), 8.05-8.06 (m, 1H), 8.05 (s, 1H), 7.79-7.83 (m, 1H), 7.03-7.10 (m, 2H), 6.47-6.48 (m, 1H), 2.50 (s, 3H).

### Example 24

### (S)-4-(5-Methylfuran-2-yl)-8-(3-(((tetrahydrofuran-3-yl)oxy)methyl)benzyl)pyrazolo[1,5 -a][1,3,5]triazin-2-amine 24

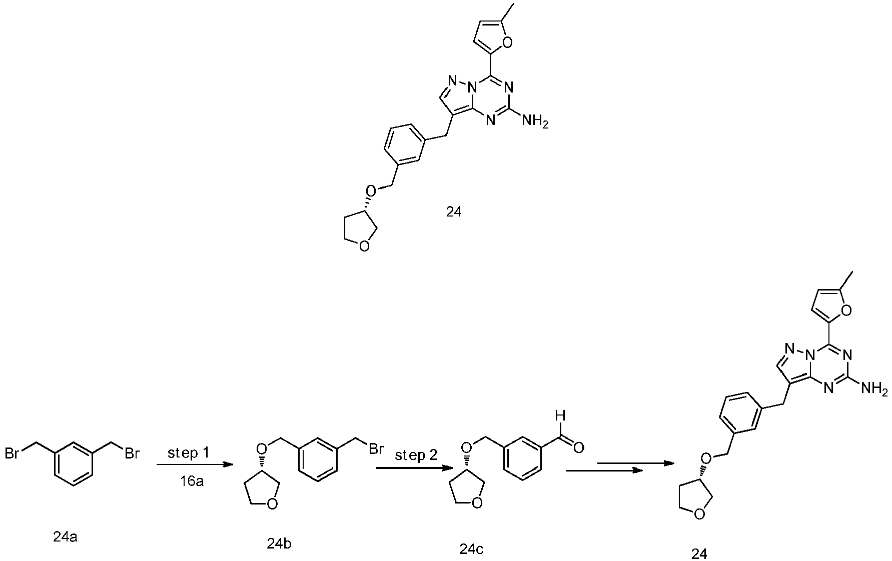

### Step 1

### (S)-3-((3-(Bromomethyl)benzyl)oxy)tetrahydrofuran 24b

Compound **16a** (8.41 g, 95.47 mmol, 7.65 mL) was dissolved in 300 mL of tetrahydrofuran, sodium hydride (4.00 g, 100.02 mmol, 60% purity) was added, and the resulting mixture was stirred for 1 hour at room temperature. Further, 1,3-bis(bromomethyl)benzene **24a** (24 g, 90.92 mmol, Adamas Reagent Co., Ltd.) was added and the reaction solution was refluxed for 16 hours. 100 mL of saturated sodium chloride solution and 200 mL of ethyl acetate were added. The resulting mixture was separated, and the aqueous phase was extracted with ethyl acetate (100 mL × 3). The organic phases were combined, dried over anhydrous sodium sulfate and filtered. The filtrate was concentrated under reduced pressure. The residue was purified by CombiFlash rapid preparation instrument with eluent system B to obtain the title compound **24b** (13.75 g, yield: 55.77%).

### Step 2

### (S)-3-(((Tetrahydrofuran-3-yl)oxy)methyl)benzaldehyde 24c

Compound **24b** (4.42 g, 16.30 mmol) and molecular sieve (2 g, 16.30 mmol) were added to 200 mL of acetonitrile, *N*-methyl-*N*-oxymorpholine (3.82 g, 32.60 mmol) was added. The resulting mixture was stirred for 1 hour and filtered. The filter cake was washed once with ethyl acetate. The filtrate was concentrated under reduced pressure, and the residue was purified by CombiFlash rapid preparation instrument with eluent system B to obtain the title compound **24c** (2.14 g, yield: 63.57%).

Using the synthetic route of Example **1,** the starting material compound **1d** in the step 9 was replaced with compound **24c** to obtain the title product **24** (15 mg).
MS *m*/*z* (ESI): 406.2 [M+1].
¹H NMR (400 MHz, CDCl₃): *δ* 8.37-8.39 (m, 1H), 7.85 (s, 1H), 7.22-7.24 (m, 2H), 6.39-6.40 (m, 1H), 5.35-5.36 (m, 2H), 4.51-4.53 (m, 2H), 4.23-4.25 (m, 1H), 3.84-4.02 (m, 6H), 2.57 (s, 3H), 2.05-2.07 (m, 2H).

### Example 25

### (S)-4-(5-Methylfuran-2-yl)-N⁸-(3-(((tetrahydrofuran-3-yl)oxy)methyl)phenyl)pyrazolo[1,5-a][ 1,3,5]triazine-2,8-diamine 25

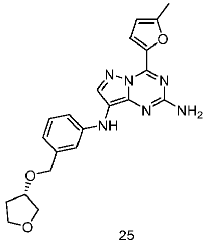

Using the synthetic route of Example **21,** the raw material compound **16b** and compound **21a** in the step 1 were respectively replaced with compound 1-bromo-3-bromomethylbenzene (Shaoyuan Technology (Shanghai) Co., Ltd.) and compound **16a** to obtain the title compound **25** (10 mg, yield: 5.2%).
MS *m*/*z* (ESI): 407.2 [M+1].
¹H NMR (400 MHz, DMSO-*d*₆): *δ* 8.23 (d, 1H), 8.05 (s, 1H), 7.24 (brs, 2H), 7.16 (s, 1H), 7.01 (t, 1H), 6.56 (brs, 2H), 6.47-6.53 (m, 2H), 4.30 (s, 2H), 4.13 (s, 1H), 3.60-3.71 (m, 4H), 2.47 (s, 3H), 1.89 (brs, 2H).

### Example 26

### (S)-N⁸-(2-fluoro-3-(((tetrahydrofuran-3-yl)oxy)methyl)phenyl)-4-(5-methylfuran-2-yl)py razolo[1,5-a][1,3,5]triazine-2,8-diamine 26

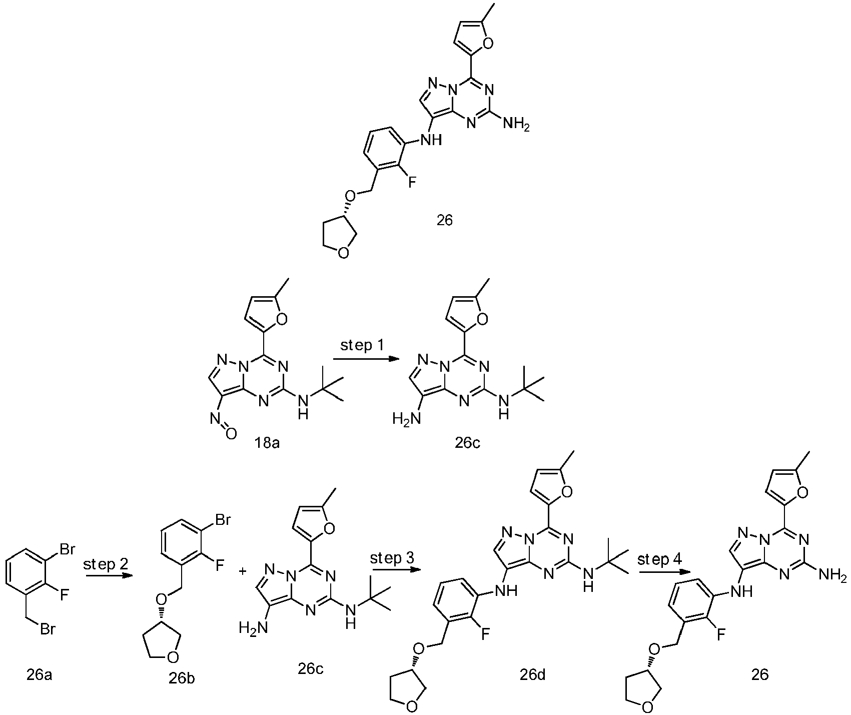

### Step 1

*N*²-(tert-Butyl)-4-(5-methylfuran-2-yl)pyrazolo[1,5-a][1,3,5]triazine-2,8-diamine **26c** Compound **18a** (950 mg, 3.16 mmol) was dissolved in 20 mL of ethanol and 10 mL of water and then heated to 70 °C, ammonium chloride (1.38 g, 25.32 mmol) and iron powder (884 mg, 15.83 mmol) were added respectively. The resulting mixture was stirred for 1 hour at 70 °C, filtered with diatomite and washed with ethyl acetate (50 mL × 3). The filtrate was concentrated under reduced pressure, and then saturated sodium bicarbonate solution was added to neutralize. The resulting mixture was extracted with ethyl acetate (50 mL × 3). The organic phases were combined, washed with saturated sodium chloride solution (20 mL), dried over sodium sulfate and filtered. The filtrate was concentrated under reduced pressure, and the residue was purified by CombiFlash rapid preparation instrument with eluent system B to obtain the title compound **26c** (780 mg, yield: 86.11%).

### Step 2

### (S)-3-((3-Bromo-2-fluorobenzyl)oxy)tetrahydrofuran 26b

Compound **16a** (658 mg, 7.47 mmol) was dissolved in 20 mL of *N*,*N*-dimethylformamide, sodium hydride (299 mg, 7.48 mmol, 60% purity) was added. The resulting mixture was stirred for 1 hour. 1-Bromo-3-(bromomethyl)-2-fluorobenzene **26a** (1.00 g, 3.73 mmol, prepared by the method disclosed in the patent application "US2012172448A1") was added, and the reaction solution was continued to stir for 17 hours. Water was added, and the resulting mixture was extracted with ethyl acetate (20 mL × 3). The organic phases were combined, washed with water (20 mL × 2) and saturated sodium chloride solution (20 mL), dried over anhydrous sodium sulfate and filtered. The filtrate was concentrated under reduced pressure. The residue was purified by CombiFlash rapid preparation instrument with eluent system B to obtain the title compound **26b** (950 mg, yield: 92.51%).

### Step 3

### (S)-N²-(tert-butyl)-N⁸-(2-fluoro-3-(((tetrahydrofuran-3-yl)oxy)methyl)phenyl)-4-(5-methylfur an-2-yl)pyrazolo[1,5-a][1,3,5]triazine-2,8-diamine 26d

Under an argon atmosphere, compound **26b** (500 mg, 1.82 mmol), compound **26c** (496 mg, 1.73 mmol), tris(dibenzylideneacetone)dipalladium (159 mg, 173.63 µmol), 4,5-bis(diphenylphosphino)-9,9-dimethylxanthene (200 mg, 345.65 µmol) and sodium *tert*-butoxide (167 mg, 1.74 mmol) were added to 50 mL of toluene and the resulting mixture was stirred for 17 hours at 100 °C . After cooling, the resulting mixture was filtered with diatomite. The filtrate was concentrated under reduced pressure, and the residue was purified by CombiFlash rapid preparation instrument with eluent system B to obtain the title compound **26d** (200 mg, yield: 24.04%).
MS *m*/*z* (ESI): 481.3 [M+1].

### Step 4

### (S)-N⁸-(2-fluoro-3-(((tetrahydrofuran-3-yl)oxy)methyl)phenyl)-4-(5-methylfuran-2-yl)py razolo[1,5-a][1,3,5]triazine-2,8-diamine 26

Compound **26d** (200 mg, 416.20 µmol) was dissolved in 5 mL of trifluoroacetic acid and the resulting mixture was heated to 70 °C for 7 hours. After cooling to room temperature, the reaction solution was concentrated under reduced pressure. A saturated sodium bicarbonate aqueous solution was added to the residue until pH > 7. The resulting mixture was extracted with dichloromethane (100 mL × 3). The organic phases were combined, dried over anhydrous sodium sulfate and filtrated. The filtrate was concentrated under reduced pressure, and the residue was purified by silica gel column chromatography with the developing system A to obtain the title compound **26** (45 mg, yield: 25.47%).
MS *m*/*z* (ESI): 425.1 [M+1].
¹H NMR (400 MHz, CDCl₃): *δ* 8.33-8.34 (m, 1H), 8.03 (s, 1H), 6.71-6.91 (m, 1H), 6.69-6.72 (m, 1H), 6.56-6.60 (m, 1H), 6.49-6.50 (m, 1H), 4.59-4.61 (m, 2H), 4.31-4.32 (m, 1H), 3.81-3.90 (m, 4H), 2.52 (s, 3H), 2.00-2.10 (m, 2H).

### Example 27

### 4-(5-Methylfuran-2-yl)-N⁸-(6-(morpholinomethyl)pyridin-2-yl)pyrazolo[1,5-a] [1,3,5]tria zine-2,8-diamine 27

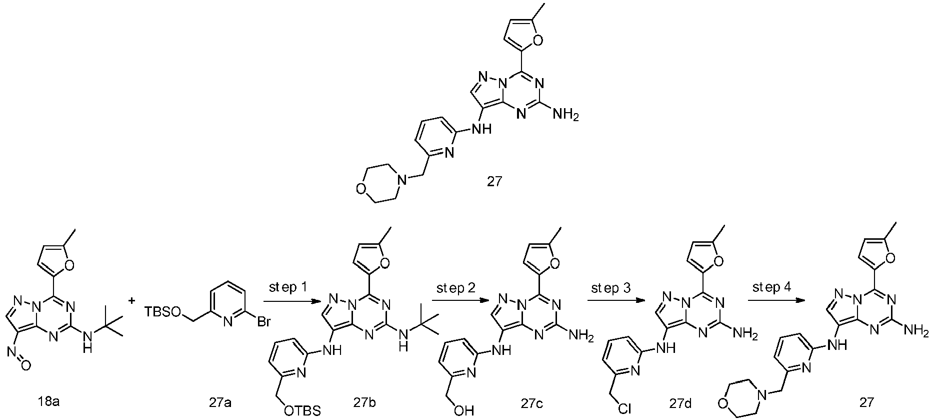

### Step 1

### N²-(tert-butyl)-N⁸-(6-(((tert-butyldimethylsilyl)oxy)methyl)pyridin-2-yl)-4-(5-methylfuran-2-yl)pyrazolo[1,5-a][1,3,5]triazine-2,8-diamine 27b

2-Bromo-6-(((*tert*-butyldimethylsilyl)oxy)methyl)pyridine **27a** (2.013 g, 6.66 mmol) was dissolved in 10 mL of tetrahydrofuran. After cooling to -78 °C, *n*-butyl lithium (1.6 M, 4.17 mL) was added. The reaction solution was stirred for 30 minutes at -78 °C. The above reaction solution was added to 30 mL of a solution of compound **18a** (1.00 g, 3.33 mmol) in tetrahydrofuran that had been precooled to -78 °C for 1 hour. Saturated ammonium chloride aqueous solution was added. The resulting mixture was separated. The aqueous phase was extracted with ethyl acetate (50 mL × 3). The organic phases were combined, dried over anhydrous sodium sulfate and filtered. The filtrate was concentrated under reduced pressure. The residue was purified by CombiFlash rapid preparation instrument with eluent system B to obtain the title compound **27b** (300 mg, yield: 8.87%).

### Step 2

### (6-((2-Amino-4-(5-methylfuran-2-yl)pyrazolo[1,5-a][1,3,5]triazin-8-yl)amino)pyridin-2-yl)m ethanol 27c

Compound **27b** (300 mg, 590.90 µmol) was dissolved in 10 mL of trifluoroacetic acid and stirred at 70 °C for 16 hours. The solvent was concentrated under reduced pressure and water was added. Saturated sodium bicarbonate solution was added to adjust the pH until pH > 7. The resulting mixture was extracted with dichloromethane (20 mL × 3). The organic phases were combined and dried over anhydrous sodium sulfate. The filtrate was concentrated under reduced pressure to obtain crude title compound **27c** (199 mg), which was used in the next reaction without purification.

### Step 3

### N⁸-(6-(Chloromethyl)pyridin-2-yl)-4-(5-methylfuran-2-yl)pyrazolo[1,5-a][1,3,5]triazine-2,8-d iamine 27d

Compound **27c** (199 mg, 589.92 µmol) was dissolved in 10 mL of dichloromethane, sulfoxide chloride (140 mg, 1.18 mmol) was added, and the reaction solution was stirred for 1 hour. Sodium bicarbonate aqueous solution was added and the pH was adjusted to > 7. The resulting mixture was separated. The aqueous phase was extracted with dichloromethane (20 mL × 3). The organic phases were combined, dried over anhydrous sodium sulfate and filtered. The filtrate was concentrated under reduced pressure. The residue was purified by CombiFlash rapid preparation instrument with eluent system B to obtain the title compound **27d** (120 mg, 57.17%).
MS *m*/*z* (ESI): 356.1 [M+1].

### Step 4

### 4-(5-Methylfuran-2-yl)-N⁸-(6-(morpholinomethyl)pyridin-2-yl)pyrazolo[1,5-a][1,3,5]tria zine-2,8-diamine 27

Compound **27d** (30 mg, 84.3215 µmol) and morpholine (73 mg, 837.92 µmol) were added to 10 mL of tetrahydrofuran and the resulting mixture was stirred at 70 °C for 16 hours. After cooling, the resulting mixture was concentrated under reduced pressure. The residue was purified by silica gel column chromatography with developing system A to obtain a crude product. The crude product was washed twice with eluent system B to obtain the title compound **27** (21 mg, yield: 61.27%).
MS *m*/*z* (ESI): 407.2 [M+1].
¹H NMR (400 MHz, DMSO-*d*₆): *δ* 8.30-8.31 (m, 1H), 8.23-8.24 (m, 1H), 8.01 (s, 1H), 7.36-7.40 (m, 1H), 7.17 (brs, 2H), 6.64 (d, 1H), 6.56-6.57 (m, 1H), 6.39 (d, 1H), 3.57-3.60 (m, 4H), 3.40 (s, 2H), 2.47 (s, 3H), 2.42-2.43 (m, 4H).

### Example 28

### (S)-4-(5-Methylfuran-2-yl)-8-((6-(((tetrahydrofuran-3-yl)oxy)methyl)pyridin-2-yl)thio)pyrazo lo[1,5-a][1,3,5]triazin-2-amine 28

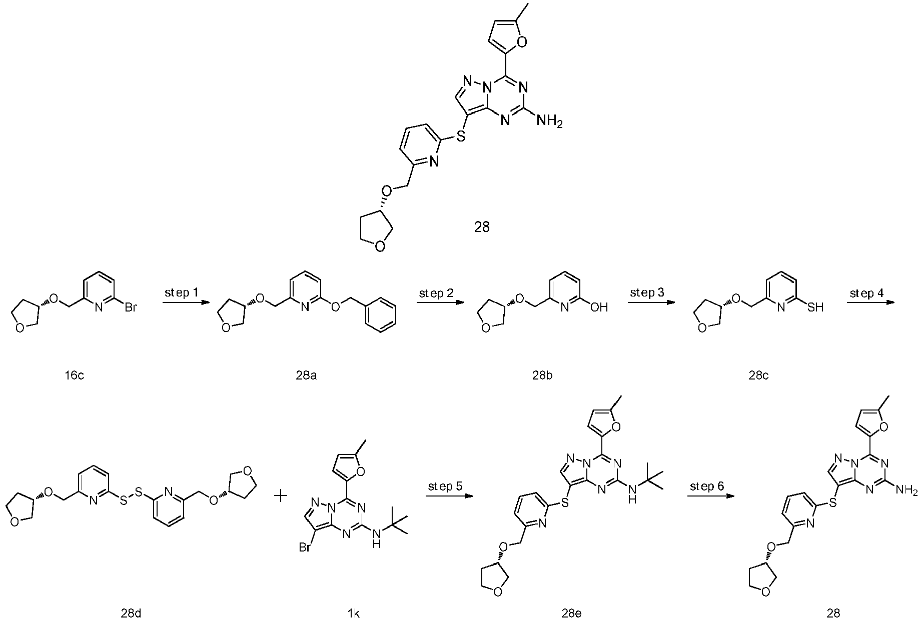

### Step 1

### (S)-2-(Benzyloxy)-6-(((tetrahydrofuran-3-yl)oxy)methyl)pyridine 28a

Compound **16c** (7.00 g, 27.12 mmol) and benzyl alcohol (3.52 g, 32.55 mmol) were dissolved in 200 mL of *N*,*N*-dimethylformamide, and sodium hydride (1.62 g, 40.67 mmol, 60% purity) was added. The resulting mixture was heated to 90 °C and stirred for 17 hours. After the reaction solution was cooled to room temperature, 500 mL of water was added. The resulting mixture was extracted with ethyl acetate (250 mL × 3). The organic phases were combined, washed with water (250 mL) and saturated sodium chloride solution (250 mL), dried over anhydrous sodium sulfate and filtered. The filtrate was concentrated under reduced pressure. The residue was purified by CombiFlash rapid preparation instrument with eluent system B to obtain the title compound **28a** (7.32 g, yield: 94.59%).

### Step 2

### (S)-6-(((tetrahydrofuran-3-yl)oxy)methyl)pyridin-2-ol 28b

Compound **28a** (7.32 g, 25.65 mmol) was dissolved in 500 mL of methanol, and 10% palladium on carbon (1.36 g, 12.82 mmol, 50% water) was added, and the reaction mixture was stirred for 4 hours under a hydrogen atmosphere. The reaction solution was filtered, and the filtrate was concentrated to obtain the title compound **28b** (4.8 g, yield: 95.84%).

### Step 3

### (S)-6-(((Tetrahydrofuran-3-yl)oxy)methyl)pyridine-2-thiol 28c

Under an argon atmosphere, compound **28b** (1.37 g, 7.01 mmol) and 1,3,2,4-disulfide, 2,4-bis(4-methoxyphenyl)-2,4-disulfide (1.72 g, 4.25 mmol) were added to 50 mL of toluene and heated to 115 °C for 16 hours. After cooling to room temperature, the reaction solution was concentrated with a rotary evaporator, and purified by CombiFlash rapid preparation instrument with eluent system A, then 20 mL of 3 M sodium hydroxide aqueous solution was added. The resulting mixture was extracted with methyl *tert*-butyl ether (50 mL × 3). The aqueous phase was adjusted to pH < 7 with citric acid, and extracted with dichloromethane (100 mL × 4). The organic phases were combined, dried over anhydrous sodium sulfate and filtered. The filtrate was concentrated under reduced pressure to obtain the title compound **28c** (580 mg, yield: 39.11%).

### Step 4

### 1,2-Bis(6-((((S)-tetrahydrofuran-3-yl)oxy)methyl)pyridin-2-yl)disulfane 28d

Sodium hydroxide (126 mg, 3.15 mmol) was dissolved in 5 mL of water, and the resulting solution was added to compound **28c** (580 mg, 2.74 mmol), and stirred for 0.5 hour. 10 mL of an aqueous solution of potassium ferricyanide (1.04 g, 3.15 mmol) was added and stirred for 16 hours. The reaction solution was filtered, the filter cake was washed thoroughly with water, and dried in vacuo to obtain the title compound **28d** (385 mg, yield; 33.35%).

### Step 5

### (S)-N-(tert-Butyl)-4-(5-methylfuran-2-yl)-8-((6-(((tetrahydrofuran-3-yl)oxy)methyl)pyridin-2-yl)thio)pyrazolo[1,5-a][1,3,5]triazin-2-amine 28e

Compound **1k** (217 mg, 619.2 µmol) was dissolved in 20 mL of tetrahydrofuran. After cooling to -78 °C, 0.78 mL of 1.6 M *n*-butyllithium was added dropwise, and stirred for 0.5 hour. A solution of compound **28d** in 10 mL of tetrahydrofuran was added and stirred for 1 hour. After adding 30 mL of saturated ammonium chloride aqueous solution to the reaction solution, the temperature was raised to room temperature, and the resulting mixture was separated. The aqueous phase was extracted with ethyl acetate (50 mL × 3). The organic phases were combined, dried over anhydrous sodium sulfate and filtered. The filtrate was concentrated under reduced pressure. The residue was purified by CombiFlash rapid preparator with eluent system B to obtain the title compound **28e** (120 mg, yield: 40.29%).

### Step 6

### (S)-4-(5-Methylfuran-2-yl)-8-((6-(((tetrahydrofuran-3-yl)oxy)methyl)pyridin-2-yl)thio)pyrazo 10[1,5-a][1,3,5]triazin-2-amine 28

Compound **28e** (120 mg, 249.6 µmol) was dissolved in 5 mL of trifluoroacetic acid and the resulting mixture was heated to 70 °C and stirred overnight. After cooling to room temperature, the reaction solution was concentrated under reduced pressure. Saturated aqueous sodium bicarbonate solution was added to the residue until the pH > 7. The resulting mixture was extracted with dichloromethane (30 mL×3). The organic phases were combined, dried over anhydrous sodium sulfate and filtered. The filtrate was concentrated under reduced pressure, and the residue was purified by CombiFlash rapid preparation instrument with eluent system A to obtain the title compound **28** (43 mg, yield: 40.57%).
MS *m*/*z* (ESI): 425.2 [M+1].
¹H NMR (400 MHz, DMSO-*d*₆) *δ* 8.24 (s, 1H), 8.23 (d, 1H), 7.61 (s, 2H), 7.56 (t, 1H), 7.10 (d, 1H), 6.72 (d, 1H), 6.58-6.59 (m, 1H), 4.47 (s, 2H), 4.24-4.25 (m, 1H), 3.73-3.79 (m, 2H), 3.66-3.69 (m, 2H), 2.48 (s, 3H), 1.93-1.99 (m, 2H).

### Example 29

### 4-(5-Methylfuran-2-yl)-N⁸-(6-((4-methylpiperazin-1-yl)methyl)pyridin-2-yl)pyrazolo[1,5 -a][1,3,5]triazine-2,8-diamine 29

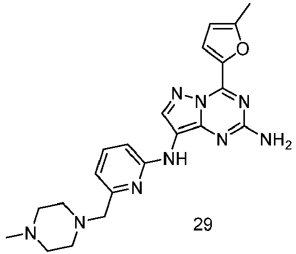

Using the synthetic route of Example **27,** the material compound morpholine in the step 4 was replaced with the compound *N*-methylpiperazine to obtain the title product **29** (5 mg, yield: 14.13%).
MS *m*/*z* (ESI): 420.2 [M+1].
¹H NMR (400 MHz, CD₃OD) *δ* 8.27-8.31 (m, 2H), 7.43-7.45 (m, 1H), 6.72-6.77 (m, 1H), 6.45-6.47 (m, 2H), 3.52 (s, 2H), 2.50 (s, 3H), 2.47-2.70 (m, 8H), 2.29 (s, 3H).

### Example 30

### 4-(5-Methylfuran-2-yl)-N⁸-(6-((tetrahydro-1H-furo[3,4-c]pyrrol-5(3H)-yl)methyl)pyridin -2-yl)pyrazolo[1,5-a][1,3,5]triazine-2,8-diamine 30

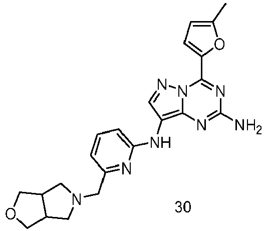

Using the synthetic route of Example **27**, the raw material morpholine in the step 4 was replaced with the compound hexahydro-1*H*-furo[3,4-*c*]pyrrole to obtain the title product **30** (18 mg, yield: 29.61%).
MS *m*/*z* (ESI): 433.2 [M+1].
¹H NMR (400 MHz, DMSO-*d*₆) *δ* 8.30 (s, 1H), 8.23-8.24 (m, 1H), 8.00-8.01 (m, 1H), 7.36-7.41 (m, 1H), 7.18 (*brs,* 2H), 6.63 (d, 1H), 6.56-6.57 (m, 1H), 6.41-6.43 (m, 1H), 3.70-3.72 (m, 2H), 3.44-3.53 (m, 3H), 3.24-3.25 (m, 1H), 2.70-2.71 (m, 2H), 2.47 (s, 3H), 2.39-2.41 (m, 2H), 1.23-1.24 (m, 1H), 0.81-0.82 (m, 1H).

### Example 31

### N⁸-(2-Fluorophenyl)-4-(5-methylfuran-2-yl)pyrazolo[1,5-a][1,3,5]triazine-2,8-diamine 31

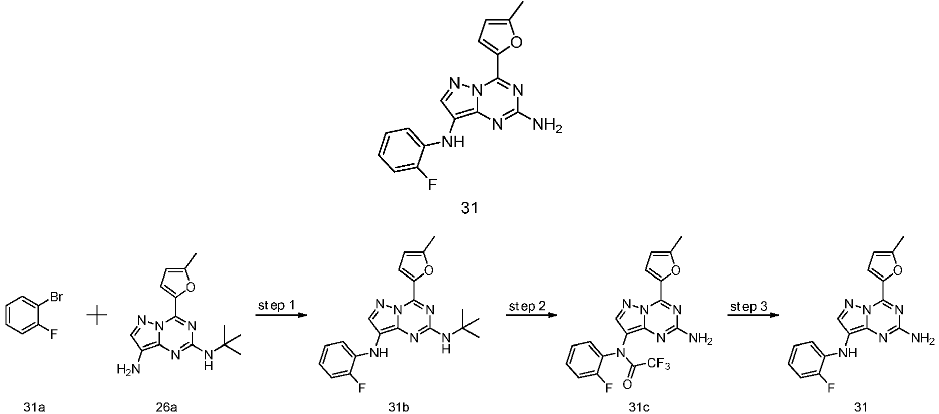

### Step 1

### N²-(tert-Butyl)-N⁸-(2-fluorophenyl)-4-(5-methylfuran-2-yl)pyrazolo[1,5-a][1,3,5]triazine-2,8-diamine 31b

Under an argon atmosphere, 2-fluorobromobenzene (160 mg, 914.2 µmol), compound **26a** (250 mg, 873.1 µmol), sodium *tert*-butoxide (84 mg, 874.0 µmol), 4,5-bis(diphenylphosphino)-9,9-dimethylxanthene (101 mg, 174.5 µmol) and tris(dibenzylideneacetone)dipalladium (80 mg, 87.36 µmol) were added to 15 mL of toluene and heated to 95 °C. The resulting mixture was stirred for 16 hours. After cooling to room temperature, the reaction solution was concentrated under reduced pressure, and the residue was purified by CombiFlash rapid preparation instrument with eluent system B to obtain the title compound **31b** (226 mg, yield: 68.04%).

### Step 2

### N-(2-Amino-4-(5-methylfuran-2-yl)pyrazolo[1,5-a][1,3,5]triazin-8-yl)-2,2,2-trifluoro-N-(2-fl uorophenyl)acetamide 31c

Compound **31b** (220 mg, 578.31 µmol) was dissolved in 10 mL of trifluoroacetic acid and heated to 70 °C for 16 hours. The reaction solution was cooled to room temperature, concentrated under reduced pressure. Saturated sodium bicarbonate aqueous solution was added to the residue adjusting pH > 7. The resulting mixture was extracted with dichloromethane (30 mL × 3). The organic phases were combined, dried over anhydrous sodium sulfate and filtered. The filtrate was concentrated under reduced pressure, and the residue was purified by CombiFlash rapid preparation instrument with eluent system B to obtain the title compound **31c** (75 mg, yield: 30.85%).

### Step 3

### N⁸-(2-Fluorophenyl)-4-(5-methylfuran-2-yl)pyrazolo[1,5-a][1,3,5]triazine-2,8-diamine 31

Compound **31c** (75 mg, 178.4 µmol) was added to 10 mL of methanol, potassium carbonate (200 mg, 1.45 mmol) was added, and the reaction solution was heated to 50 °C and reacted for 2 hours. The reaction solution was cooled to room temperature, and concentrated under reduced pressure. 20 mL of water and 20 mL of dichloromethane were added and the resulting mixture was separated. The aqueous phase was extracted with dichloromethane (30 mL × 3). The organic phases were combined, dried over anhydrous sodium sulfate and filtered. The filtrate was concentrated under reduced pressure, and the residue was purified by CombiFlash rapid preparation instrument with eluent system B to obtain the title compound **31** (30 mg, yield: 51.84%).
MS *m*/*z* (ESI): 325.1 [M+1].
¹H NMR (400 MHz, DMSO-*d*₆) *δ* 8.24 (d, 1H), 8.05 (s, 1H), 7.28 (s, 2H), 7.03-7.06 (m, 1H), 7.00 (s, 1H), 6.85 (d, 1H), 6.56-6.57 (m, 2H), 6.45-6.47 (m, 1H), 2.48 (s, 3H).

### Example 32

### N⁸-(6-((2-Oxa-6-azaspiro[3.3]heptan-6-yl)methyl)pyridin-2-yl)-4-(5-methylfuran-2-yl)pyrazo lo[1,5-a][1,3,5]triazine-2,8-diamine 32

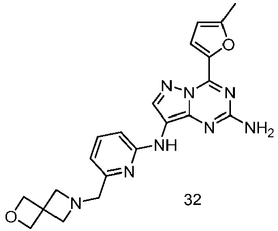

Using the synthetic route of Example **27,** the raw material morpholine in the step 4 was replaced with the compound 2-oxa-6-azaspiro[3.3]heptane hemioxalate (PharmaBlock Sciences (Nanjing), Inc.) to obtain the title product 32 (43 mg).
MS *m*/*z* (ESI): 419.2 [M+1].
¹H NMR (400 MHz, CD₃OD) *δ* 8.30-8.31 (m, 1H), 8.27 (s, 1H), 7.41-7.45 (t, 1H), 6.59 (d, 1H), 6.46-6.50 (m, 2H), 4.72 (s, 4H), 3.61 (s, 2H), 3.58 (s, 4H), 2.49 (s, 3H).

### Example 33

### 8-Benzyl-4-(furan-2-yl)pyrazolo[1,5-a][1,3,5]triazin-2-amine 33

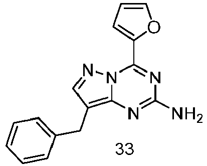

Using the synthetic route of Example **10,** the raw material compound **4a** in the step 5 was replaced with the compound benzaldehyde to obtain the title product **33** (82 mg, yield: 69.85%).
MS *m*/*z* (ESI): 292.2 [M+1].
¹H NMR (400 MHz, DMSO-*d*₆): *δ* 8.24-8.25 (m, 1H), 8.17-8.18 (m, 1H), 7.95-7.96 (m, 1H), 7.23-7.24 (m, 4H), 7.15-7.17 (m, 1H), 6.87-6.89 (m, 1H), 3.87 (s, 2H).

### Example 34

### 8-(3-Fluorobenzyl)-4-(furan-2-yl)pyrazolo[1,5-a][1,3,5]triazin-2-amine 34

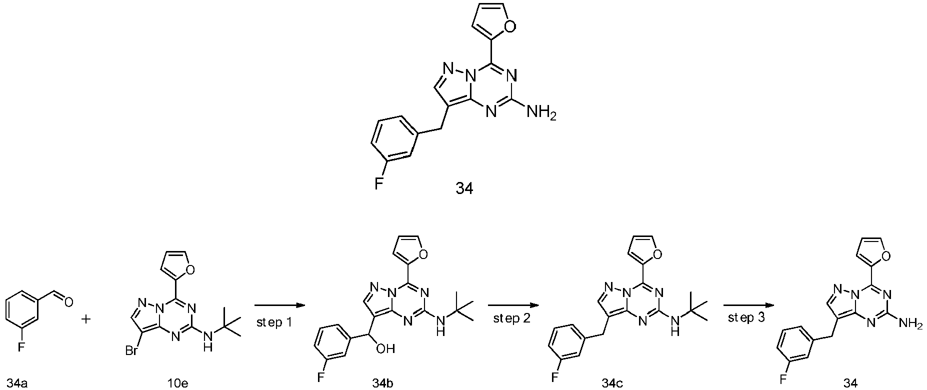

### Step 1

### (2-(tert-Butylamino)-4-(furan-2-yl)pyrazolo[1,5-a][1,3,5]triazin-8-yl)(3-fluorophenyl)methan ol 34b

Compound **10e** (600 mg, 1.78 mmol) was dissolved in 50 mL of tetrahydrofuran. After cooling to -78 °C, *n*-butyllithium (1.6 M, 2.2 mL) was added dropwise, and reacted for 30 minutes. 3-Fluorobenzaldehyde **34a** (332 mg, 2.67 mmol) was added and the resulting mixture was stirred for 30 minutes. 30 mL of saturated ammonium chloride aqueous solution was added. The resulting mixture was warmed to room temperature, and separated. The aqueous phase was extracted with ethyl acetate (50 mL × 3). The organic phases were combined, dried over anhydrous sodium sulfate and filtered. The filtrate was concentrated under reduced pressure. The residue was purified by CombiFlash rapid preparation instrument with eluent system B to obtain the title compound **34b** (210 mg, yield: 30.85%).

### Step 2

### N-(tert-Butyl)-8-(3-fluorobenzyl)-4-(furan-2-yl)pyrazolo[1,5-a][1,3,5]triazin-2-amine 34c

Compound **34b** (210 mg, 550 µmol) was dissolved in 10 mL of dichloromethane, trifluoroacetic acid (1.25 g, 10.96 mmol) was added, triethylsilane (1.28 g, 11.01 mmol) was added dropwise, and the reaction solution was stirred for 16 hours. 20 mL of saturated sodium bicarbonate aqueous solution was added and the resulting mixture was separated. The aqueous phase was extracted with dichloromethane (20 mL × 3). The organic phases were combined, dried over anhydrous sodium sulfate and filtered. The filtrate was concentrated under reduced pressure. The residue was purified by a CombiFlash rapid preparation instrument with eluent system B to obtain the title compound **34c** (160 mg, yield: 79.53%).

### Step 3

### 8-(3-Fluorobenzyl)-4-(furan-2-yl)pyrazolo[1,5-a][1,3,5]triazin-2-amine 34

Compound **34c** (160 mg, 437 µmol) was dissolved in 5 mL of trifluoroacetic acid, heated to 70 °C and reacted for 16 hours. The reaction solution was concentrated under reduced pressure, saturated sodium bicarbonate aqueous solution was added to the residue to obtain pH > 7. The resulting mixture was extracted with dichloromethane (30 mL × 3). The organic phases were combined, dried over anhydrous sodium sulfate and filtered. The filtrate was concentrated under reduced pressure, and the residue was purified by thin layer chromatography with eluent system B to obtain the title compound **34** (10 mg, yield: 7.38%).
MS *m*/*z* (ESI): 309.9 [M+1].
¹H NMR (400 MHz, CD₃OD) *δ* 8.35 (d, 1H), 7.99 (s, 1H), 7.89 (s, 1H), 7.30-7.25 (m, 1H), 7.11-7.09 (m, 1H), 7.03-7.00 (m, 1H), 6.92-6.83 (m, 2H), 3.97 (s, 2H).

### Example 35

### 2-(6-((2-Amino-4-(5-methylfuran-2-yl)pyrazolo[1,5-a][1,3,5]triazin-8-yl)oxy)pyridin-2-yl)pr opan-2-ol 35

### 1-(6-((2-Amino-4-(5-methylfuran-2-yl)pyrazolo[1,5-a][1,3,5]triazin-8-yl)oxy)pyridin-2-yl)eth an-1-one 36

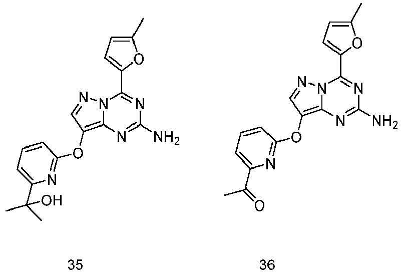

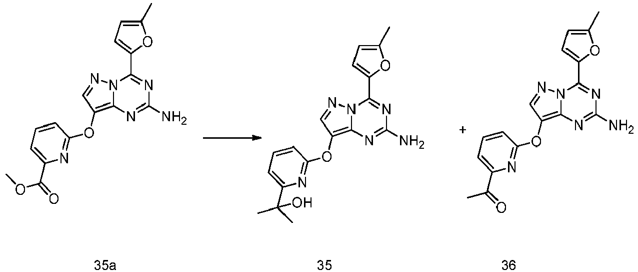

Using the synthesis method of Example **19,** the raw material **16c** in the step 6 was replaced with methyl 6-bromopicolinate to obtain the compound methyl 6-((2-amino-4-(5-methylfuran-2-yl)pyrazolo[1,5-*a*][1,3,5]triazin-8-yl)oxy)picolinate **35a** (60 mg, yield: 93.49%).

Compound **35a** (60 mg, 163.7 µmol) was dissolved in 10 mL of tetrahydrofuran, methylmagnesium bromide (3 M, 0.28 mL) was added, the reaction solution was stirred for 30 minutes. Then supplementary methyl magnesium bromide (3 M, 0.28 mL) was added, and the reaction solution was stirred for 30 minutes. 10 mL of saturated ammonium chloride aqueous solution was added. The resulting mixture was separated, and the aqueous phase was extracted with ethyl acetate (30 mL × 3). The organic phases were combined, dried over anhydrous sodium sulfate and filtered. The filtrate was concentrated under reduced pressure. The residue was purified by a preparative plate with developing system A to obtain the title compound **35** (16 mg) and compound **36** (20 mg).

Compound **35**
MS *m*/*z* (ESI): 367.2 [M+1].
¹H NMR (400 MHz, DMSO-*d*₆) *δ* 8.20-8.21 (m, 2H), 7.72 (t, 1H), 7.29 (d, 1H), 7.25 (s, 2H), 6.74 (d, 1H), 6.53-6.54 (m, 1H), 5.09 (s, 1H), 2.44 (s, 3H), 1.28 (s, 6H).

Compound **36**
MS *m*/*z* (ESI): 351.1 [M+1].
¹H NMR (400 MHz, DMSO-*d*₆) *δ* 8.29 (s, 1H), 8.20 (d, 1H), 7.96 (t, 1H), 7.63 (d, 1H), 7.26-7.28 (m, 3H), 6.52 (d, 1H), 2.43 (s, 3H), 2.38 (s, 3H).

### Example 37

### 1-(6-((2-Amino-4-(5-methylfuran-2-yl)pyrazolo[1,5-a][1,3,5]triazin-8-yl)oxy)pyridin-2-yl)eth an-1-ol 37

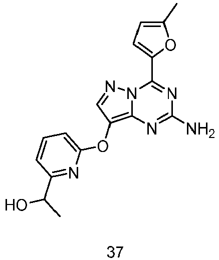

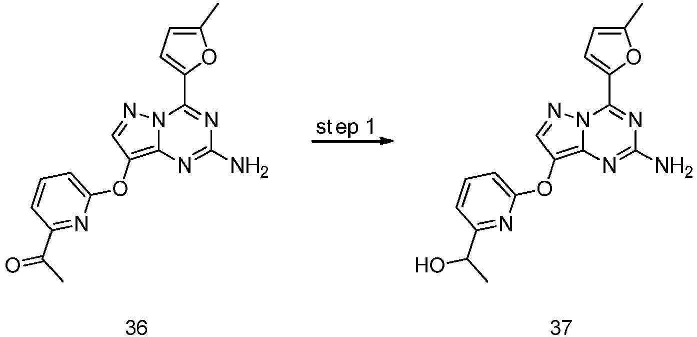

Compound **36** (6 mg, 17.12 µmol) was dissolved in 6 mL of methanol, lithium borohydride (2 M, 0.1 mL) was added, and the reaction solution was stirred for 1 hour. 15 mL of ammonium chloride aqueous solution was added, and the resulting mixture was stirred for 5 minutes and extracted with dichloromethane (30 mL × 3). The organic phases were combined, dried over anhydrous sodium sulfate and filtered. The filtrate was concentrated under reduced pressure, and the residue was purified by a preparative plate with developing system A to obtain the title compound **37** (2 mg, yield: 33.14%).
MS *m*/*z* (ESI): 353.1 [M+1].
¹H NMR (400 MHz, CD₃OD) *δ* 8.34 (d, 1H), 8.12 (d, 1H), 7.78 (t, 1H), 7.22 (d, 1H), 6.85 (d, 1H), 6.48 (d, 1H), 4.70 (q, 1H), 2.51 (d, 3H), 1.40 (d, 3H).

### Example 38

### 2-(6-((2-Amino-4-(5-methylfuran-2-yl)pyrazolo[1,5-a][1,3,5]triazin-8-yl)methyl)pyridin-2-yl )propan-2-ol 38

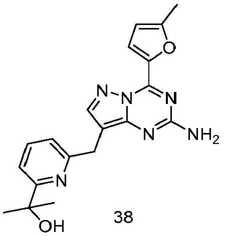

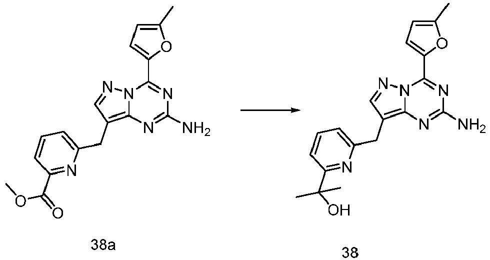

Using the synthetic route of Example **1**, the raw material compound **1d** in the step 9 was replaced with methyl 6-formyl-2-pyridinecarboxylate (Shanghai Bide Technology Pharmaceutical Co.,Ltd.) to prepare compound methyl 6-((2-amino-4-(5-methylfuran-2-yl)pyrazolo[1,5-*a*][1,3,5]triazin-8-yl)methyl)picolinate **38a** (70 mg).

Compound **38a** (70 mg, 192 µmol) was dissolved in 20 mL of tetrahydrofuran and cooled to 0 °C, methylmagnesium bromide (91 mg, 768 µmol) was added, the resulting mixture was slowly warmed to room temperature and reacted for 2 hours. 30 mL of saturated ammonium chloride aqueous solution was added, and then the resulting mixture was warmed to room temperature and separated. The aqueous phase was extracted with ethyl acetate (50 mL × 3). The organic phases were combined, dried over anhydrous sodium sulfate and filtered. The filtrate was concentrated under reduced pressure, and the residue was purified by a CombiFlash rapid preparation instrument with eluent system B to obtain the title compound **38** (20 mg, yield: 28.56%).
MS *m*/*z* (ESI): 365.3 [M+1].
¹H NMR (400 MHz, CDCl₃): *δ* 8.35 (d, 1H), 7.96 (s, 1H), 7.64 (s, 1H), 7.20-7.14 (m, 2H), 5.41 (s, 2H), 4.20 (s, 2H), 2.54 (s, 3H), 1.55 (s, 6H).

### Test Examples:

### Biological Assay

Test Example 1. Determination of the inhibitory activities of the compounds of the present disclosure on the adenosine A₂ₐ receptor (A₂ₐR) cAMP signaling pathway, the adenosine A₁ receptor (A₁R) cAMP signaling pathway and the adenosine A₃ receptor (A₃R) cAMP signaling pathway.

The inhibitory activities of the compounds of the present disclosure on the adenosine A₂ₐ receptor (A₂ₐR) cAMP signaling pathway, the adenosine A₁ receptor cAMP signaling pathway and the adenosine A₃ receptor cAMP signaling pathway were determined by the following methods. The experimental method is briefly described as follows:

### I. Experimental materials and instruments

1. CHO-K1/A₂ₐR cells (NM_000675.5) or CHO-K1/A₁R cells (NM_000674.2) or CHO-K1/A₃R cells (NM_000677.3)
2. Fetal bovine serum (Gibco, 10099-141)
3. Zeocin™ (Thermo, R25001) or G418 (ENZO, ALX-380-013-G005) or puromycin (Thermo, 10687-010)
4. DMEM/F12 medium (GE, SH30023.01)
5. Cell dissociation buffer (Thermo Fisher, 13151014)
6. HEPES (Gibco, 42360-099)
7. Bovine serum albumin (MP Biomedicals, 219989725)
8. Rolipram (sigma, R6520-10MG)
9. Adenosine deaminase (sigma, 10102105001)
10. Forskolin (sigma, F6886)
11. 2Cl-IB-MECA (Tocrics, 1104/10)
12. N6-cyclopentyladenosine (Tocris, 1702/50)
13. HBSS buffer (Thermo, 14025-092)
14. cAMP dynamic 2 kit (Cisbio, 62AM4PEB)
15. 384-well plate (Corning, 4514) or (Nunc, 267462#)
16. Ethylcarbazole (Torcis, 1691/10)
17. PHERAstar multi-function microplate reader (Cisbio, 62AM4PEB)

### II. Experimental procedures

### 2.1 Adenosine A₂ₐ receptor

CHO-K1/A₂ₐR cells were cultured in DMEM/F12 medium containing 10% fetal bovine serum and 800 µg/mL Zeocin™. The cells were digested with the cell dissociation buffer during the experiment. The cells were resuspended in the HBSS buffer containing 20 mM HEPES and 0.1% bovine serum albumin and counted, and the cell density was adjusted to 10⁶ cells/mL. In 384-well plates, each well was added with 5 µL of the cell suspension, and 2.5 µL of test compounds (4× concentration) formulated with the HBSS buffer containing 20 mM HEPES, 0.1% bovine serum albumin, 54 µM rolipram and 2.7 U/mL adenosine deaminase, and the plates were incubated at room temperature for 30 minutes. Each well was then added with 2.5 µL of ethylcarbazole (4× concentration) formulated with the balanced salt buffer containing 20 mM HEPES, 0.1% bovine serum albumin, 54 µM rolipram and 2.7 U/mL adenosine deaminase, and the plates were incubated at room temperature for 30 minutes. The final concentrations of the compounds were: 10000, 2000, 400, 80, 16, 3.2, 0.64, 0.128, 0.0256, 0.00512, and 0.001024 nM. The final concentration of ethylcarbazole was 20 nM. Intracellular cAMP concentration was determined with cAMP dynamic 2 kits. cAMP-d2 and Anti-cAMP-Eu-Cryptate were diluted respectively with the cAMP lysis buffer at a ratio of 1:4. Each well was added with 5 µL of the diluted cAMP-d2, followed by addition of 5 µL of the diluted Anti-cAMP-Eu-Cryptate, and the plates were incubated at room temperature in the dark for 1 hour. The HTRF signal values were read by the PHERAstar multi-function microplate reader. IC₅₀ values of inhibitory activities of the compounds were calculated by Graphpad Prism software, and are shown in Table 1.

### 2.2 Adenosine A₁ receptor

CHO-K1/A₁R cells were cultured in DMEM/F12 medium containing 10% fetal bovine serum and 1 mg/mL G418. The cells were digested with the cell dissociation buffer during the experiment. The cells were then resuspended in the HBSS buffer containing 20 mM HEPES and 0.1% bovine serum albumin and counted, and the cell density was adjusted to 5 × 10⁵ cells/mL. In 384-well plates, each well was added with 12.5 µL of the cell suspension, and 6.25 µL of the test compounds (4× concentration) formulated with the HBSS buffer containing 20 mM HEPES, 0.1% bovine serum albumin, 54 µM rolipram and 2.7 U/mL adenosine deaminase, and the plates were incubated at room temperature for 30 minutes. Each well was then added with 6.25 µL of forskolin and N6-cyclopentyladenosine (4× concentration) formulated with the HBSS buffer containing 20 mM HEPES, 0.1% bovine serum albumin, 54 µM rolipram and 2.7 U/mL adenosine deaminase, and the plates were incubated at room temperature for 30 minutes. The final concentrations of the compounds were: 100000, 10000, 1000, 100, 10, 1, 0.1 and 0 nM. The final concentration of forskolin was 10 µM. The final concentration of CPA was 10 nM. Intracellular cAMP concentration was determined with cAMP dynamic 2 kits. cAMP-d2 and Anti-cAMP-Eu-Cryptate were diluted respectively with the cAMP lysis buffer at a ratio of 1:4. Each well was added with 12.5 µL of the diluted cAMP-d2, followed by addition of 12.5 µL of the diluted Anti-cAMP-Eu-Cryptate, and the plates were incubated at room temperature in the dark for 1 hour. The HTRF signal values were read by the PHERAstar multi-function microplate reader. IC₅₀ values of the inhibitory activities of the compounds were calculated by Graphpad Prism software, and are shown in Table 2.

### 2.3 Adenosine A₃ receptor

CHO-K1/A₃R cells were cultured in DMEM/F12 medium containing 10% fetal bovine serum and 10 µg/mL puromycin. The cells were digested with the cell dissociation buffer during the experiment. The cells were resuspended in the HBSS buffer containing 20 mM HEPES and 0.1% bovine serum albumin and counted, and the cell density was adjusted to 5 × 10⁵ cells/mL. In 384-well plates, each well was added with 12.5 µL of the cell suspension, and 6.25 µL of the test compounds (4× concentration) formulated with the HBSS buffer containing 20 mM HEPES, 0.1% bovine serum albumin, 54 µM rolipram and 2.7 U/mL adenosine deaminase, and the plates were incubated at room temperature for 30 minutes. Each well was then added with 6.25 µL of forskolin and 2Cl-IB-MECA (4× concentration) formulated with the HBSS buffer containing 20 mM HEPES, 0.1% bovine serum albumin, 54 µM rolipram and 2.7 U/mL adenosine deaminase, and the plates were incubated at room temperature for 30 minutes. The final concentrations of the compounds were: 100000, 10000, 1000, 100, 10, 1, 0.1 and 0 nM. The final concentration of forskolin was 10 µM. The final concentration of 2Cl-IB-MECA was 5 nM. Intracellular cAMP concentration was determined with cAMP dynamic 2 kits. cAMP-d2 and Anti-cAMP-Eu-Cryptate were diluted respectively with the cAMP lysis buffer at a ratio of 1:4. Each well was added with 12.5 µL of the diluted cAMP-d2, followed by addition of 12.5 µL of the diluted Anti-cAMP-Eu-Cryptate, and the plates were incubated at room temperature in the dark for 1 hour. The HTRF signal values were read by the PHERAstar multi-function microplate reader. IC₅₀ values for the inhibitory activities of the compounds were calculated by Graphpad Prism software, and are shown in Table 3.

**Table 1: IC₅₀ values of the inhibitory activities of the compounds of the present disclosure on the adenosine A₂ₐ receptor (A₂ₐR) cAMP signaling pathway.**

| Example No. | IC₅₀/nM (A₂ₐR) |
|---|---|
| 1 | 0.33 |
| 2 | 0.87 |
| 3 | 0.45 |
| 4 | 0.14 |
| 5 | 0.55 |
| 6 | 0.4 |
| 7 | 1.18 |
| 8 | 1.32 |
| 9 | 1.64 |
| 10 | 1.71 |
| 11 | 1.86 |
| 13 | 1.7 |
| 14 | 0.68 |
| 15 | 1.83 |
| 17 | 0.72 |
| 18 | 0.87 |
| 19 | 0.36 |
| 20 | 0.09 |
| 21 | 0.4 |
| 22 | 0.57 |
| 23 | 0.58 |
| 24 | 1.2 |
| 25 | 1.64 |
| 26 | 1.66 |
| 27 | 1.9 |
| 28 | 2.13 |
| 29 | 2.8 |
| 30 | 2.87 |
| 31 | 3.53 |
| 32 | 3.74 |
| 33 | 4.14 |
| 34 | 4.64 |

Conclusion: The compounds of the present disclosure have significant inhibitory activities on the adenosine A₂ₐ receptor cAMP signaling pathway.

**Table 2: IC₅₀ values of the inhibitory activities of the compounds of the present disclosure on the adenosine A₁ receptor (A₁R) cAMP signaling pathway.**

| Example No. | IC₅₀/nM (A₂ₐR) | IC₅₀/nM (A₁R) | IC₅₀ ratio (A₁R/A₂ₐR) |
|---|---|---|---|
| 1 | 0.33 | 1123 | >10³ |
| 2 | 0.87 | 1101 | >10³ |
| 3 | 0.45 | 434 | 964 |
| 5 | 0.55 | 835 | >10³ |
| 6 | 0.4 | 316 | 790 |
| 7 | 1.18 | 340 | 288 |
| 8 | 1.32 | 943 | 714 |
| 9 | 1.64 | 483 | 294 |
| 10 | 1.71 | 147 | 86 |
| 11 | 1.86 | 410 | 220 |
| 13 | 1.7 | 1233 | 725 |
| 14 | 0.68 | 293 | 431 |
| 15 | 1.83 | 165 | 90 |
| 18 | 0.87 | 2536 | >10³ |
| 19 | 0.36 | 2862 | >10³ |
| 21 | 0.4 | 609 | >10³ |
| 22 | 0.57 | 303 | 532 |
| 24 | 1.2 | 2017 | >10³ |
| 25 | 1.64 | 2045 | >10³ |
| 26 | 1.66 | 1213 | 731 |
| 27 | 1.9 | 286 | 150 |
| 28 | 2.13 | 595 | 279 |
| 29 | 2.8 | 3692 | >10³ |
| 30 | 2.87 | 684 | 238 |
| 32 | 3.74 | 287 | 78 |
| 33 | 4.14 | 565 | 136 |

Conclusion: The compounds of the present disclosure have weak inhibitory activities on the adenosine A₁ receptor, indicating that the compounds of the present disclosure are highly selective for the adenosine A₂ₐ receptor.

**Table 3: IC₅₀ values of the inhibitory activities of the compounds of the present disclosure on the adenosine A₃ receptor cAMP signaling pathway.**

| Example No. | IC₅₀/nM (A₂ₐR) | IC₅₀/nM (A₃R) | IC₅₀ ratio (A₃ R/A₂ₐ R) |
|---|---|---|---|
| 1 | 0.33 | 2057 | > 10³ |
| 2 | 0.87 | 2421 | > 10³ |
| 3 | 0.45 | 4121 | > 10³ |
| 4 | 0.14 | > 10⁴ | > 10⁴ |
| 5 | 0.55 | 2282 | > 10³ |
| 6 | 0.4 | 4810 | > 10³ |
| 7 | 1.18 | 7367 | > 10³ |
| 8 | 1.32 | 6492 | > 10³ |
| 9 | 1.64 | 3372 | > 10³ |
| 10 | 1.71 | 358 | 209 |
| 13 | 1.7 | 5502 | > 10³ |
| 14 | 0.68 | 1393 | > 10³ |
| 15 | 1.83 | 8966 | > 10³ |
| 17 | 0.72 | 1393 | > 10³ |
| 18 | 0.87 | 2204 | > 10³ |
| 19 | 0.78 | 4790 | > 10³ |
| 20 | 0.09 | 280 | > 10³ |
| 21 | 0.4 | 1696 | > 10³ |
| 22 | 0.57 | 1045 | > 10³ |
| 24 | 1.2 | 2040 | > 10³ |
| 25 | 1.64 | 8153 | > 10³ |
| 26 | 1.66 | 5303 | > 10³ |
| 27 | 1.9 | 977 | 514 |
| 28 | 2.13 | 1151 | 540 |
| 29 | 2.8 | 2181 | 779 |
| 30 | 2.87 | 7595 | > 10³ |
| 32 | 3.74 | 5611 | > 10³ |

Conclusion: The compounds of the present disclosure have weak inhibitory activities on the adenosine A₃ receptor, indicating that the compounds of the present disclosure are highly selective for the adenosine A₂ₐ receptor.

### Pharmacokinetics Evaluation

### Test Example 2. Pharmacokinetics assay of the compounds of the present disclosure in mice

### 1. Abstract

Mice were used as test animals. The drug concentration in plasma at different time points was determined by LC/MS/MS method after intragastrical administration of the compounds of Example 1, Example 18 and Example 19 to mice. The pharmacokinetic behavior of the compounds of the present disclosure was studied and evaluated in mice.

### 2. Test protocol

### 2.1 Test compounds

Compounds of Example 1, Example 18 and Example 19.

### 2.2 Test animals

Twenty-seven C57 mice (female) were purchased from Shanghai Jiesijie Laboratory Animal Co., LTD, with Certificate No.: SCXK (Shanghai) 2013-0006, and equally divided into 3 groups (9 mice per group).

### 2.3 Preparation of the test compounds

A certain amount of the test compound was weighed, and added with 2.5% by volume of DMSO, 2.5% by volume of tween 80 and 95% by volume of normal saline to prepare a 0.1 mg/mL colorless, clear and transparent solution.

### 2.4 Administration

After an overnight fast, C57 mice were administered intragastrically with the test compounds at an administration dose of 2.0 mg/kg and an administration volume of 0.2 mL/10 g.

### 3. Process

The mice were intragastrically administered with the compounds of Example 1, Example 18 and Example 19. 0.1 mL of blood (3 animals at each time point) was taken before administration and at 0.5, 1.0, 2.0, 4.0, 6.0, 8.0, 11.0 and 24.0 hours after administration. The samples were stored in heparinized tubes, and centrifuged for 10 minutes at 3,500 rpm to separate the plasma. The plasma samples were stored at -20 °C, the mice was feed 2 hours after administration.

The content of the test compounds in the plasma of the mice after intragastrical administration of the test compounds at different concentrations was determined: 25 µL of the plasma obtained at each time point after administration was taken, added with 30 µL of the internal standard solution of camptothecin (100 ng/mL) and 200 µL of acetonitrile, vortex-mixed for 5 minutes, and centrifuged for 10 minutes (4000 rpm). 3 µL of the supernatant was taken for LC/MS/MS analysis.

### 4. Results of pharmacokinetic parameters

Pharmacokinetic parameters of the compounds of the present disclosure are shown in Table 4:

**Table 4: Pharmacokinetic parameters of the compounds of the present disclosure**

| No. | Pharmacokinetics assay in mice (2 mg/kg) | | | | | |
|---|---|---|---|---|---|---|
| | Drug concentration in blood | Area under curve | Half-life | Residence time | Clearance | Apparent distribution volume |
| | Cmax (ng/mL) | AUC (ng/mL^{∗}h) | T1/2 (h) | MRT (h) | CLz/F (mL/min/kg ) | Vz/F (mL/kg) |
| Example 1 | 800 | 724 | 0.48 | 0.85 | 45.9 | 1897 |
| Example 18 | 3177 | 4859 | 1.42 | 1.63 | 6.83 | 841 |
| Example 19 | 2358 | 2493 | 1.85 | 1.24 | 13.3 | 2122 |

Conclusion: The compounds of the present disclosure are well absorbed, and have a pharmacokinetic advantage.

## Claims

1. A compound of formula (I) or a tautomer, mesomer, racemate, enantiomer, diastereomer thereof, or a mixture thereof, or a pharmaceutically acceptable salt thereof: 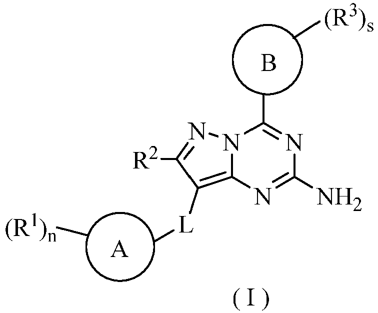 wherein:
L is selected from the group consisting of CR⁴R⁵, O, NH and S;
ring A and ring B are identical or different and are each independently selected from the group consisting of cycloalkyl, heterocyclyl, aryl and heteroaryl;
R¹ is identical or different and is each independently selected from the group consisting of hydrogen, halogen, alkyl, alkoxy, haloalkyl, haloalkoxy, hydroxyl, hydroxyalkyl, cyano, amino, nitro, cycloalkyl, heterocyclyl, heterocyclylalkyl, heterocyclyloxy, aryl, heteroaryl and -Y-R^{a};
Y is a covalent bond or alkylene;
R^{a} is selected from the group consisting of hydrogen, halogen, alkyl, alkoxy, haloalkyl, haloalkoxy, hydroxyl, hydroxyalkyl, cyano, amino, nitro, cycloalkyl, heterocyclyl, heterocyclylalkyl, heterocyclyloxy, -OR^{c}, -C(O)R⁹, -C(O)OR⁹, -OS(O)ₘR⁶, aryl and heteroaryl, wherein the alkyl, alkoxy, cycloalkyl, heterocyclyl, heterocyclylalkyl, heterocyclyloxy, aryl and heteroaryl are each optionally substituted by one or more substituents independently selected from the group consisting of halogen, alkyl, alkoxy, haloalkyl, hydroxyl, hydroxyalkyl, cyano, amino, nitro, cycloalkyl, heterocyclyl, heterocyclyloxy, aryl, heteroaryl and -OS(O)ₘR⁶;
R^{c} is selected from the group consisting of hydrogen, alkyl, haloalkyl, hydroxyalkyl, cycloalkyl and heterocyclyl, wherein the alkyl, cycloalkyl and heterocyclyl are each optionally substituted by one or more substituents independently selected from the group consisting of halogen, alkyl, alkoxy, haloalkyl, hydroxyl, hydroxyalkyl, cyano, amino, nitro, cycloalkyl and heterocyclyl;
R² is selected from the group consisting of hydrogen, halogen, alkyl, alkoxy, haloalkyl, hydroxyl, hydroxyalkyl, cyano, amino, nitro, cycloalkyl, heterocyclyl, aryl and heteroaryl;
R³ is selected from the group consisting of hydrogen, halogen, alkyl, alkoxy, haloalkyl, hydroxyl, hydroxyalkyl, cyano, amino, nitro, cycloalkyl, heterocyclyl, aryl and heteroaryl;
R⁴ and R⁵ are identical or different and are each independently selected from the group consisting of hydrogen, halogen, alkyl, alkoxy, haloalkyl, hydroxyl and hydroxyalkyl;
or, R⁴ and R⁵ together with each other form =NH or =O;
R⁶ is selected from the group consisting of hydrogen, halogen, alkoxy, haloalkoxy, amino, cycloalkyl, heterocyclyl, aryl, heteroaryl and -NR⁷R⁸;
R⁷ and R⁸ are identical or different and are each independently selected from the group consisting of hydrogen, alkyl, alkoxy, haloalkyl, haloalkoxy, hydroxyl, hydroxyalkyl, cyano, amino, nitro, cycloalkyl, heterocyclyl, aryl and heteroaryl;
or, R⁷ and R⁸, together with the nitrogen atom to which they are attached, form a heterocyclyl, wherein the heterocyclyl optionally contains one to two identical or different heteroatoms selected from the group consisting of N, O and S besides the nitrogen atom to which R⁷ and R⁸ are attached, and the heterocyclyl is optionally substituted by one or more substituents selected from the group consisting of alkyl, alkoxy, oxo, halogen, amino, cyano, nitro, hydroxyl, hydroxyalkyl, cycloalkyl, heterocyclyl, aryl and heteroaryl;
R⁹ is selected from the group consisting of hydrogen, alkyl, haloalkyl, alkoxy, haloalkoxy, amino, cycloalkyl, heterocyclyl, aryl and heteroaryl;
n is 0, 1, 2, 3 or 4;
s is 0, 1, 2 or 3; and
m is 1 or 2.

2. The compound of formula (I) according to claim 1, or a tautomer, mesomer, racemate, enantiomer or diastereomer thereof, or a mixture thereof, or a pharmaceutically acceptable salt thereof, wherein R^{a} is selected from the group consisting of hydrogen, halogen, alkyl, alkoxy, haloalkyl, haloalkoxy, hydroxyl, hydroxyalkyl, cyano, amino, nitro, cycloalkyl, heterocyclyl, heterocyclylalkyl, heterocyclyloxy, -OS(O)ₘR⁶, aryl and heteroaryl, wherein the alkyl, alkoxy, cycloalkyl, heterocyclyl, heterocyclylalkyl, heterocyclyloxy, aryl and heteroaryl are each optionally substituted by one or more substituents independently selected from the group consisting of halogen, alkyl, alkoxy, haloalkyl, hydroxyl, hydroxyalkyl, cyano, amino, nitro, cycloalkyl, heterocyclyl, heterocyclyloxy, aryl, heteroaryl and -OS(O)ₘR⁶; R⁶ is as defined in claim 1.

3. The compound of formula (I) according to claim 1 or 2, or a tautomer, mesomer, racemate, enantiomer or diastereomer thereof, or a mixture thereof, or a pharmaceutically acceptable salt thereof, being a compound of formula (II): 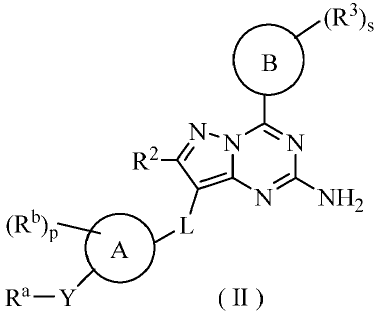 wherein:
R^{b} is identical or different and is each independently selected from the group consisting of hydrogen, halogen, alkyl, alkoxy, haloalkyl, haloalkoxy, hydroxyl, hydroxyalkyl, cyano, amino, nitro, cycloalkyl, heterocyclyl, heterocyclylalkyl, heterocyclyloxy, aryl and heteroaryl;
p is 0, 1, 2 or 3;
ring A, ring B, L, Y, R^{a}, R², R³ and s are as defined in claim 1.

4. The compound of formula (I) according to any one of claims 1 to 3, or a tautomer, mesomer, racemate, enantiomer or a diastereomer thereof, or a mixture thereof, or a pharmaceutically acceptable salt thereof, wherein ring A and ring B are identical or different and are each independently selected from the group consisting of aryl and heteroaryl, preferably selected from the group consisting of phenyl, pyridyl, furyl and thienyl.

5. The compound of formula (I) according to any one of claims 1 to 4, or a tautomer, mesomer, racemate, enantiomer or a diastereomer thereof, or a mixture thereof, or a pharmaceutically acceptable salt thereof, being a compound of formula (III): 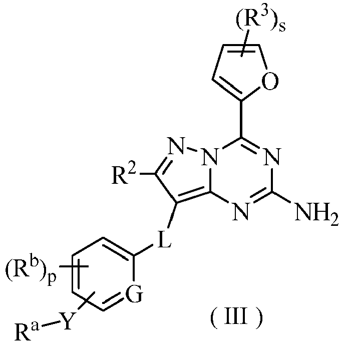 wherein:
G is selected from the group consisting of C, CH and N;
L, Y, R^{a}, R^{b}, R², R³, p and s are as defined in claim 1.

6. The compound of formula (I) according to any one of claims 1 to 5, or a tautomer, mesomer, racemate, enantiomer or diastereomer thereof, or a mixture thereof, or a pharmaceutically acceptable salt thereof, wherein -Y- is a covalent bond or -CH₂-.

7. The compound of formula (I) according to any one of claims 1 to 6, or a tautomer, mesomer, racemate, enantiomer or diastereomer thereof, or a mixture thereof, or a pharmaceutically acceptable salt thereof, being a compound of formula (IV): 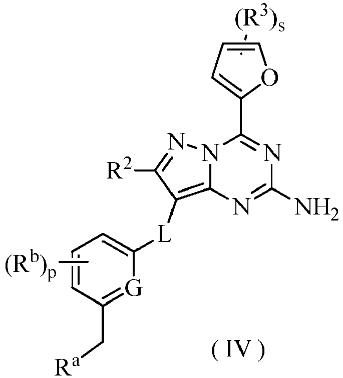 wherein:
G is selected from the group consisting of C, CH and N;
L, R^{a}, R^{b}, R², R³, p and s are as defined in claim 1.

8. The compound of formula (I) according to any one of claims 1 to 7, or a tautomer, mesomer, racemate, enantiomer or diastereomer thereof, or a mixture thereof, or a pharmaceutically acceptable salt thereof, wherein L is selected from the group consisting of CR⁴R⁵, O, NH and S; R⁴ and R⁵ are hydrogen; or R⁴ and R⁵ together with each other form =NH.

9. The compound of formula (I) according to any one of claims 1 to 8, or a tautomer, mesomer, racemate, enantiomer or diastereomer thereof, or a mixture thereof, or a pharmaceutically acceptable salt thereof, wherein R² is selected from the group consisting of hydrogen, halogen and alkyl.

10. The compound of formula (I) according to any one of claims 1 to 9, or a tautomer, mesomer, racemate, enantiomer or diastereomer thereof, or a mixture thereof, or a pharmaceutically acceptable salt thereof, wherein R³ is selected from the group consisting of hydrogen, halogen and alkyl.

11. The compound of formula (I) according to any one of claims 1 and 3 to 10, or a tautomer, mesomer, racemate, enantiomer or diastereomer thereof, or a mixture thereof, or a pharmaceutically acceptable salt thereof, wherein R^{a} is selected from the group consisting of hydrogen, halogen, alkyl, haloalkyl, alkoxy, haloalkoxy, hydroxyl, hydroxyalkyl, heterocyclyl, heterocyclylalkyl, heterocyclyloxy, -OR^{c}, -COR⁹, -COOR⁹ and -OS(O)ₘR⁶, wherein the alkyl, alkoxy, heterocyclyl, heterocyclylalkyl and heterocyclyloxy are each optionally substituted by one or more substituents independently selected from the group consisting of halogen, alkyl, alkoxy and cycloalkyl; R⁶ is alkyl or amino; R^{c} is selected from the group consisting of hydrogen, alkyl, haloalkyl, hydroxyalkyl, cycloalkyl and heterocyclyl, wherein the alkyl, cycloalkyl and heterocyclyl are each optionally substituted by one or more substituents independently selected from the group consisting of alkyl, alkoxy, hydroxyl, hydroxyalkyl, cycloalkyl and heterocyclyl; R⁹ is alkyl.

12. The compound of formula (I) according to any one of claims 3 to 11, or a tautomer, mesomer, racemate, enantiomer or diastereomer thereof, or a mixture thereof, or a pharmaceutically acceptable salt thereof, wherein R^{b} is selected from the group consisting of hydrogen, halogen and alkyl; p is 0, 1 or 2.

13. The compound of formula (I) according to any one of claims 1 to 6, or a tautomer, mesomer, racemate, enantiomer or diastereomer thereof, or a mixture thereof, or a pharmaceutically acceptable salt thereof, selected from the group consisting of:

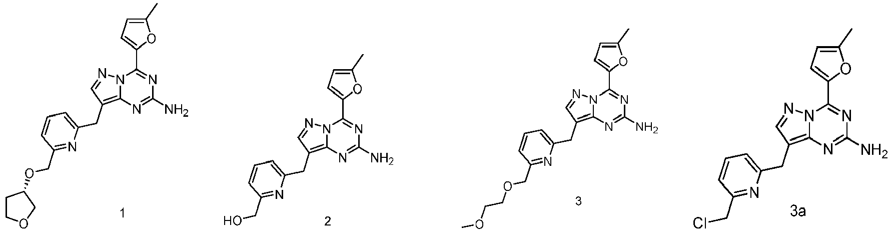

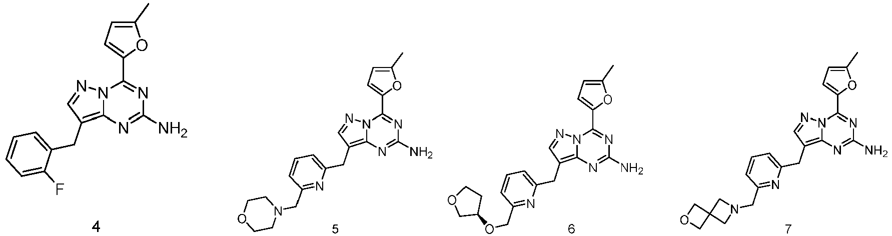

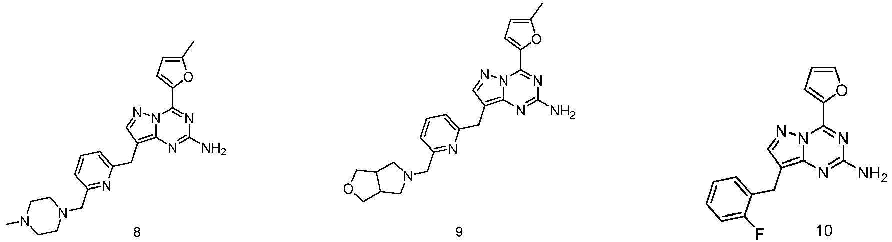

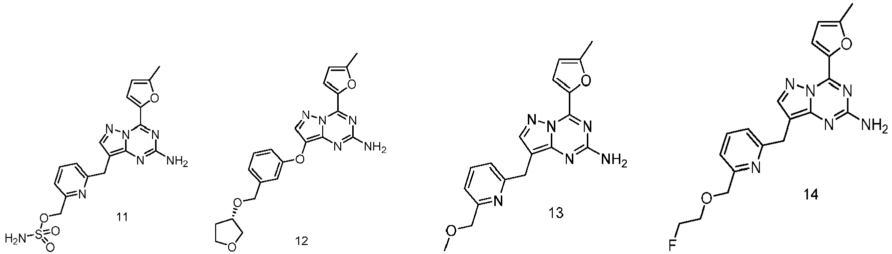

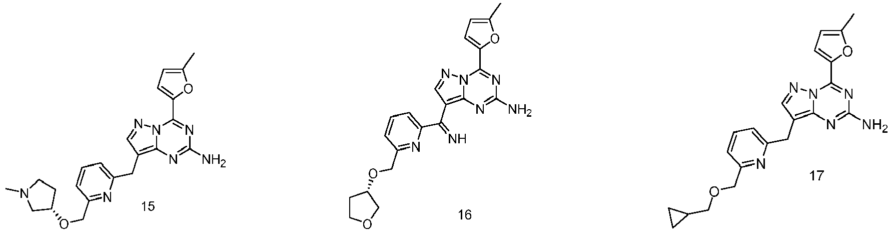

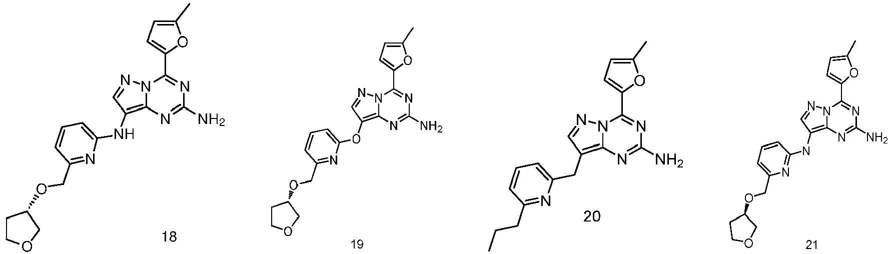

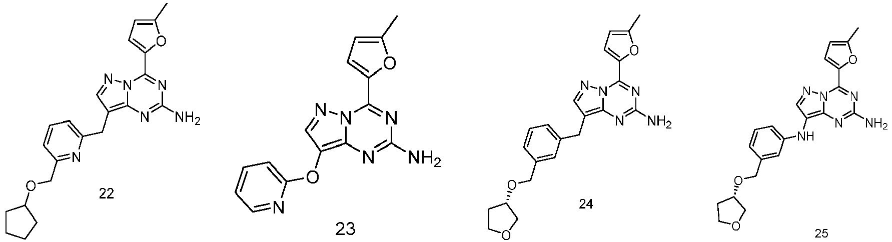

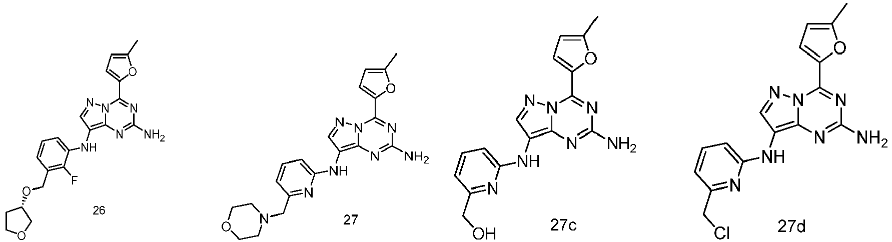

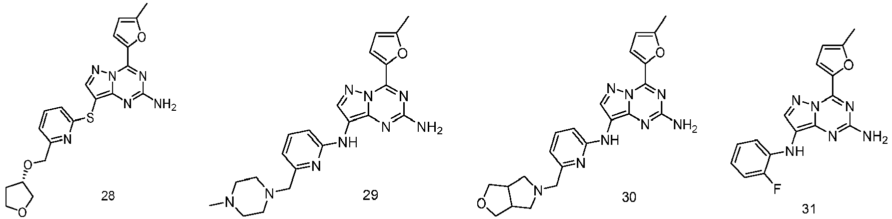

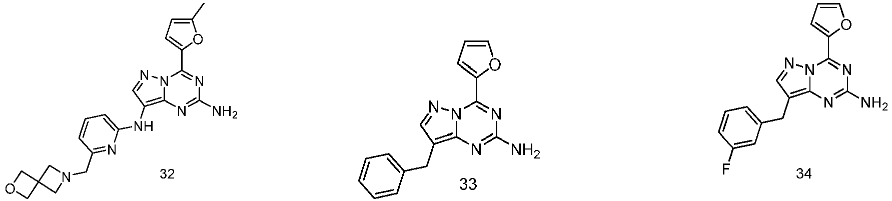

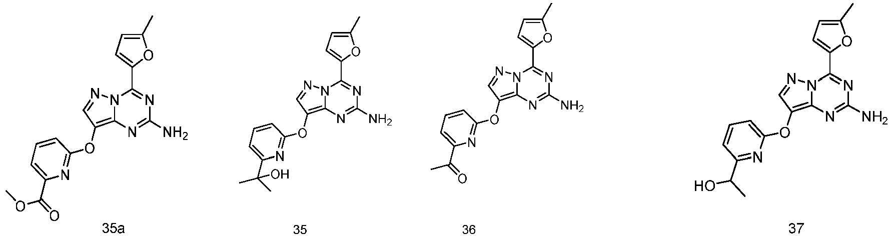

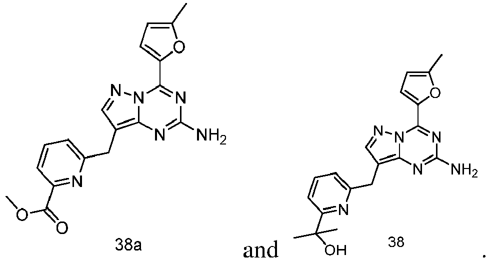

14. A compound of formula (IA): 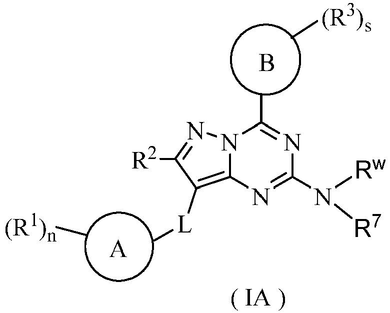 or a tautomer, mesomer, racemate, enantiomer or diastereomer thereof, or a mixture thereof, or a pharmaceutically acceptable salt thereof,
wherein:
R^{w} is an amino protecting group, preferably *tert*-butyl or *tert*-butoxycarbonyl;
R⁷ is hydrogen or R^{w};
L is selected from the group consisting of CR⁴R⁵, O, NH and S;
ring A and ring B are identical or different and are each independently selected from the group consisting of cycloalkyl, heterocyclyl, aryl and heteroaryl;
R¹ is identical or different and is each independently selected from the group consisting of hydrogen, halogen, alkyl, alkoxy, haloalkyl, haloalkoxy, hydroxyl, hydroxyalkyl, cyano, amino, nitro, cycloalkyl, heterocyclyl, heterocyclylalkyl, heterocyclyloxy, aryl, heteroaryl and -Y-R^{a};
Y is a covalent bond or alkylene;
R^{a} is selected from the group consisting of hydrogen, halogen, alkyl, alkoxy, haloalkyl, haloalkoxy, hydroxyl, hydroxyalkyl, cyano, amino, nitro, cycloalkyl, heterocyclyl, heterocyclylalkyl, heterocyclyloxy, -OR^{c}, -C(O)R⁹, -C(O)OR⁹, -OS(O)ₘR⁶, aryl and heteroaryl, wherein the alkyl, alkoxy, cycloalkyl, heterocyclyl, heterocyclylalkyl, heterocyclyloxy, aryl and heteroaryl are each optionally substituted by one or more substituents independently selected from the group consisting of halogen, alkyl, alkoxy, haloalkyl, hydroxyl, hydroxyalkyl, cyano, amino, nitro, cycloalkyl, heterocyclyl, heterocyclyloxy, aryl, heteroaryl and -OS(O)ₘR⁶;
R^{c} is selected from the group consisting of hydrogen, alkyl, haloalkyl, hydroxyalkyl, cycloalkyl and heterocyclyl, wherein the alkyl, cycloalkyl and heterocyclyl are each optionally substituted by one or more substituents independently selected from the group consisting of halogen, alkyl, alkoxy, haloalkyl, hydroxyl, hydroxyalkyl, cyano, amino, nitro, cycloalkyl and heterocyclyl;
R² is selected from the group consisting of hydrogen, halogen, alkyl, alkoxy, haloalkyl, hydroxyl, hydroxyalkyl, cyano, amino, nitro, cycloalkyl, heterocyclyl, aryl and heteroaryl;
R³ is selected from the group consisting of hydrogen, halogen, alkyl, alkoxy, haloalkyl, hydroxyl, hydroxyalkyl, cyano, amino, nitro, cycloalkyl, heterocyclyl, aryl and heteroaryl;
R⁴ and R⁵ are identical or different and are each independently selected from the group consisting of hydrogen, halogen, alkyl, alkoxy, haloalkyl, hydroxyl and hydroxyalkyl;
or, R⁴ and R⁵ together with each other form =NH or =O;
R⁶ is selected from the group consisting of hydrogen, halogen, alkoxy, haloalkoxy, amino, cycloalkyl, heterocyclyl, aryl and heteroaryl;
R⁹ is selected from the group consisting of hydrogen, alkyl, haloalkyl, alkoxy, haloalkoxy, amino, cycloalkyl, heterocyclyl, aryl and heteroaryl;
n is 0, 1, 2, 3 or 4;
s is 0, 1, 2 or 3; and
m is 1 or 2.

15. The compound of formula (IA) according to claim 14, selected from the group consisting of:

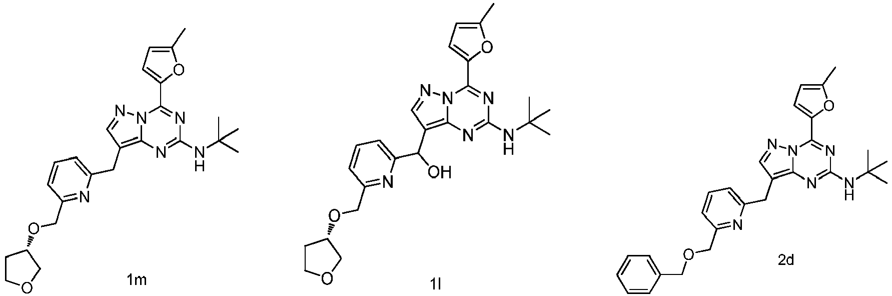

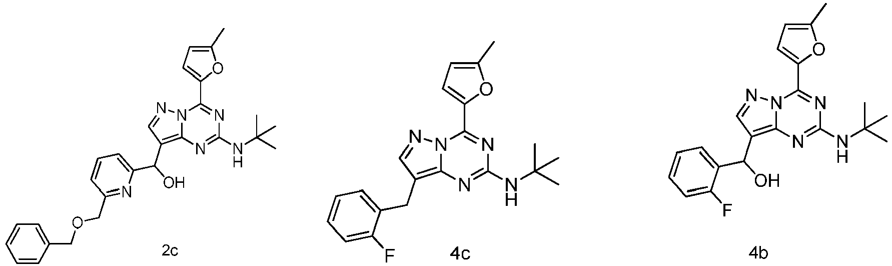

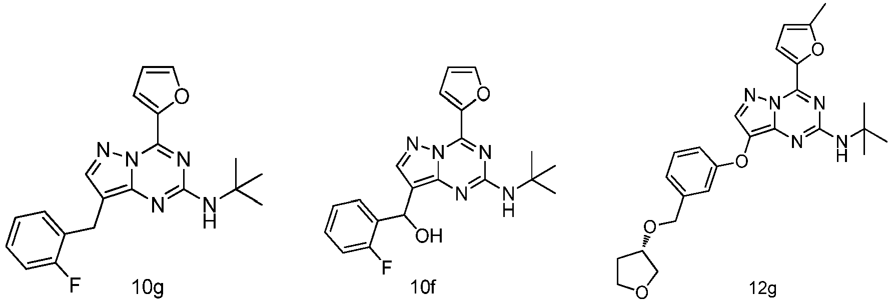

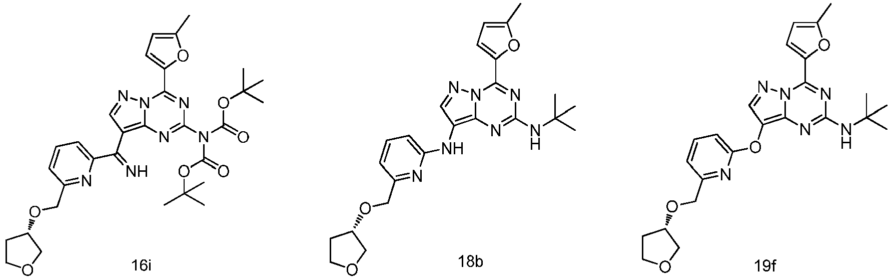

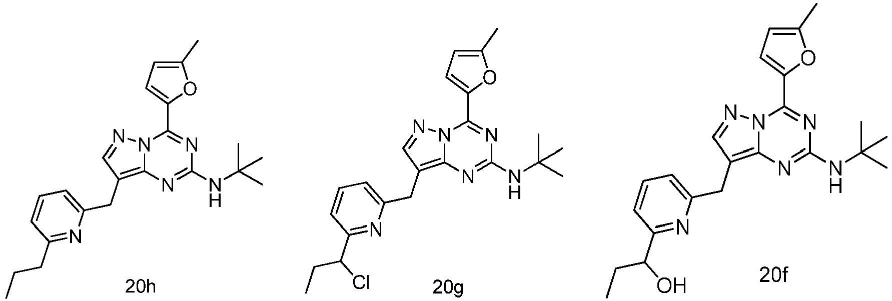

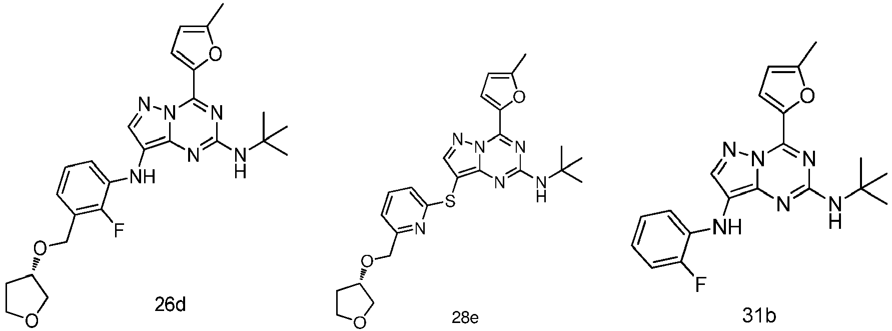

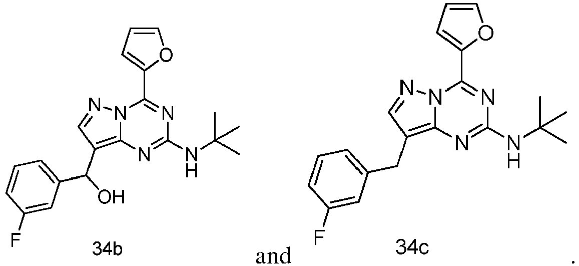

16. A method for preparing the compound of formula (I) as defined in claim 1, comprising a step of: 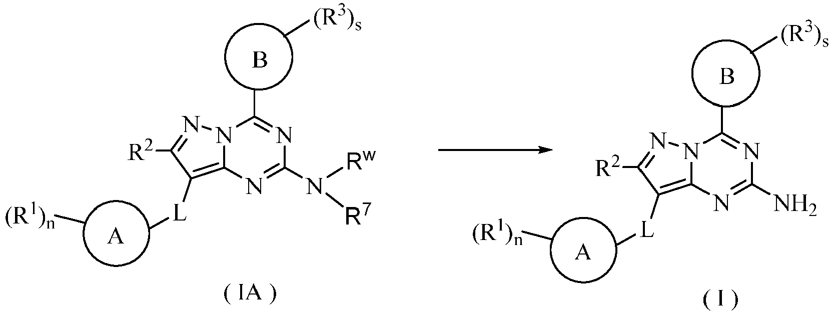 removing the amino protecting group from a compound of formula (IA) to obtain the compound of formula (I);
wherein:
R^{w} is an amino protecting group, preferably *tert*-butyl or *tert*-butoxycarbonyl;
R⁷ is hydrogen or R^{w};
ring A, ring B, L, R¹-R³, n and s are as defined in claim 1.

17. A pharmaceutical composition, comprising a therapeutically effective amount of the compound of formula (I), or the tautomer, mesomer, racemate, enantiomer or diastereomer thereof, or the mixture thereof, or the pharmaceutically acceptable salt thereof as defined in any one of claims 1 to 13, and one or more pharmaceutically acceptable carriers, diluents or excipients.

18. Use of the compound of formula (I), or the tautomer, mesomer, racemate, enantiomer or diastereomer thereof, or the mixture thereof, or the pharmaceutically acceptable salt thereof as defined in any one of claims 1 to 13 or the pharmaceutical composition as defined in claim 17 in the preparation of a medicament for inhibiting an A2a receptor.

19. Use of the compound of formula (I), or the tautomer, mesomer, racemate, enantiomer or diastereomer thereof, or the mixture thereof, or the pharmaceutically acceptable salt thereof as defined in any one of claims 1 to 13 or the pharmaceutical composition as defined in claim 17 in the preparation of a medicament for treating a disease or condition ameliorated by the inhibition of an A₂ₐ receptor.

20. The use according to claim 19, wherein the disease or condition ameliorated by the inhibition of an A₂ₐ receptor is selected from the group consisting of tumor, depression, cognitive dysfunction, neurodegenerative disorder, attention-related disorder, extrapyramidal syndrome, abnormal movement disorder, cirrhosis, liver fibrosis, fatty liver, dermal fibrosis, sleep disorder, stroke, brain injury, neuroinflammation and addictive behavior, and preferably tumor.

21. The use according to claim 20, wherein the tumor is selected from the group consisting of melanoma, brain tumor, esophageal cancer, stomach cancer, liver cancer, pancreatic cancer, colorectal cancer, lung cancer, kidney cancer, breast cancer, ovarian cancer, prostate cancer, skin cancer, neuroblastoma, sarcoma, osteochondroma, osteoma, osteosarcoma, seminoma, testicular tumor, uterine cancer, head and neck tumor, multiple myeloma, malignant lymphoma, polycythemia vera, leukemia, thyroid tumor, ureteral tumor, bladder tumor, gallbladder cancer, cholangiocarcinoma, chorionic epithelioma and pediatric tumor.
